# EUROPEAN PATENT APPLICATION

(11) **EP 4 785 960 A1**
(43) Date of publication of application: **05.08.2026**
(21) Application number: 25155422.6
(22) Date of filing: 31.01.2025
(51) Int. Cl.: A61K 47/62, A61K 47/68, A61P 3/04, A61P 5/48, A61K 47/64

(54) **GLP-1 ANTIBODY CONJUGATES**

(71) Applicant: SixPeaks Bio AG, 4051 Basel (CH)
(72) Inventor: Bleicher, Konrad, 79102 Freiburg i.Br. (DE); Schäublin, Adrian, 4436 Oberdorf (CH); Kunz, Christian, 4052 Basel (CH)
(74) Representative: AstraZeneca Intellectual Property

(57) **Abstract**

The present invention relates to immunoconjugates comprising an ActRII receptor antibody and an agonistic GLP-1 peptide. The molecules described herein were engineered to achieve the best possible therapeutic effect. Such constructs are useful in the treatment of numerous diseases and disorders, including metabolic disorders, such as obesity, diabetes and others.

## Description

### Field of the invention

The present invention relates to immunoconjugates comprising an ActRII receptor antibody and an agonistic GLP-1 peptide. The molecules described herein were engineered to achieve the best possible therapeutic effect. Such constructs are useful in the treatment of numerous diseases and disorders, including metabolic disorders, such as obesity, diabetes and others.

### Background of the invention

Activin type 2 receptors belong to a larger TGF-beta receptor family and modulate signals for transforming growth factor beta ligands. These receptors are involved in a host of physiological processes including, growth, cell differentiation, homeostasis, osteogenesis, apoptosis and many other functions. There are two activin type two receptors: ActRIIA (ACVR2A) and ActRllB (ACVR2B).

Despite the large number of processes that these ligands regulate, they all operate through essentially the same pathway: A ligand binds to a type 2 receptor, which recruits and trans-phosphorylates a type I receptor. The type I receptor recruits a receptor regulated SMAD (R-SMAD) which it phosphorylates. R-SMAD then translocates to the nucleus where it functions as a transcription factor.

It was reported that several ligands that signal through the activin type 2 receptors regulate muscle growth (Proc. Natl. Acad. Sci. U.S.A. 102 (50): 18117-22).

Glucagon-like peptide-1 (GLP-1) is a peptide hormone deriving from the tissue-specific posttranslational processing of the proglucagon peptide. It is produced and secreted by intestinal enteroendocrine L-cells and certain neurons within the nucleus of the solitary tract in the brainstem upon food consumption. The initial product GLP-1 (1-37) is susceptible to amidation and proteolytic cleavage, which gives rise to the two truncated and equipotent biologically active forms, GLP-1 (7-36) amide and GLP-1 (7-37). Active GLP-1 protein secondary structure includes two α-helices from amino acid position 13-20 and 24-35 separated by a linker region.

Alongside glucose-dependent insulinotropic peptide (GIP), GLP-1 is an incretin; thus, it has the ability to decrease blood sugar levels in a glucose-dependent manner by enhancing the secretion of insulin. Besides the insulinotropic effects, GLP-1 has been associated with numerous regulatory and protective effects. Unlike GIP, the action of GLP-1 is preserved in patients with type 2 diabetes. Glucagon-like peptide-1 receptor agonists gained approval as drugs to treat diabetes and obesity starting in the 2000s.

Combination treatments comprising ActRII receptor antibodies and a glucagon-like peptide- 1 receptor (GLP-1) agonist were reported (WO2023/028606), but the individual moieties (i.e. the ActRII receptor antibody and the glucagon-like peptide-1 receptor (GLP-1) agonist) are separate, unconjugated molecules. PCT/EP2024/071630 describes the beneficial effects of conjugating GLP-1 agonists to ActRII receptor antibodies, but the molecules disclosed therein are not yet ideal for therapeutic development.

The present invention describes the development of novel and improved immunoconjugates with higher in vivo efficacy, particular an increased fat mass reduction associated with full preservation of the lean mass. The invention also enables dosing at a reduced frequency.

### Figure legends

Figure 1 shows the molecular structure of Compounds 1 ( top), 2 (middle) and 3 (bottom).
Figure 2 shows the molecular structure of Compounds 4 ( top), 5 (middle) and 6 (bottom).
Figure 3 shows the molecular structure of Compounds 7 ( top), 8 (middle) and 9 (bottom).
Figure 4 shows the molecular structure of Compounds 10 ( top), 11 (middle) and 12 (bottom).
Figure 5 shows the molecular structure of Compounds 13 ( top), 14 (middle) and 15 (bottom).
Figure 6 shows results of the affinity measurements of the conjugates of the present inventions to ActRIIA. Reference = unconjugated antibody with variable heavy chain of SEQ ID No. 18 and variable light chain of SEQ ID No. 19.

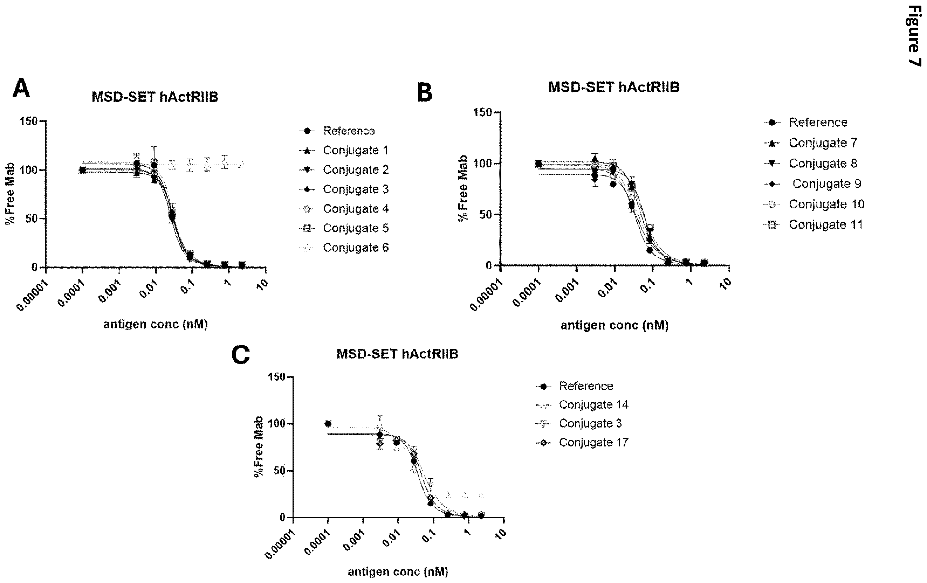

Figure 7 shows results of the affinity measurements of the conjugates of the present inventions to ActRIIB. Reference = unconjugated antibody with variable heavy chain of SEQ ID No. 18 and variable light chain of SEQ ID No. 19.
Figure 8 shows results of the measurements of antagonistic activity in an ActRIIA/B reporter cell assays (Activin A). Reference A = unconjugated antibody with variable heavy chain of SEQ ID No. 28 and variable light chain of SEQ ID No. 29. Reference B & Reference (Panel C-F): unconjugated antibody with variable heavy chain of SEQ ID No. 18 and variable light chain of SEQ ID No. 19.

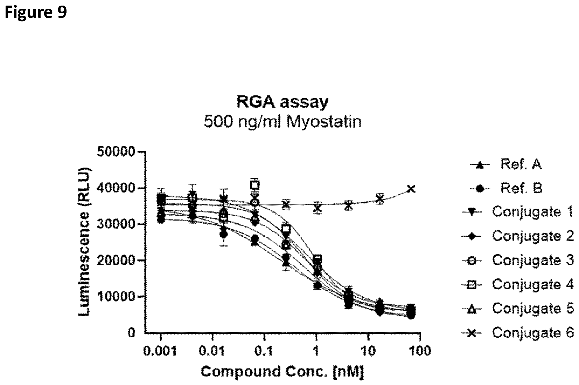

Figure 9 shows results of the measurements of antagonistic activity in an ActRIIA/B reporter cell assays (Myostatin). Reference A = unconjugated antibody with variable heavy chain of SEQ ID No. 28 and variable light chain of SEQ ID No. 29. Reference B: unconjugated antibody with variable heavy chain of SEQ ID No. 18 and variable light chain of SEQ ID No. 19.
Figure 10 shows results of the affinity measurements of the conjugates of the present inventions to mouse GLPR.

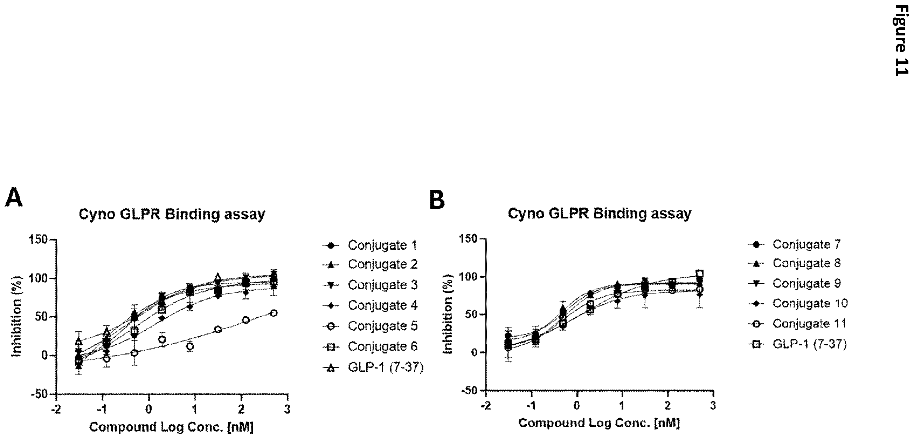

Figure 11 shows results of the affinity measurements of the conjugates of the present inventions to cynomolgus monkey GLPR.

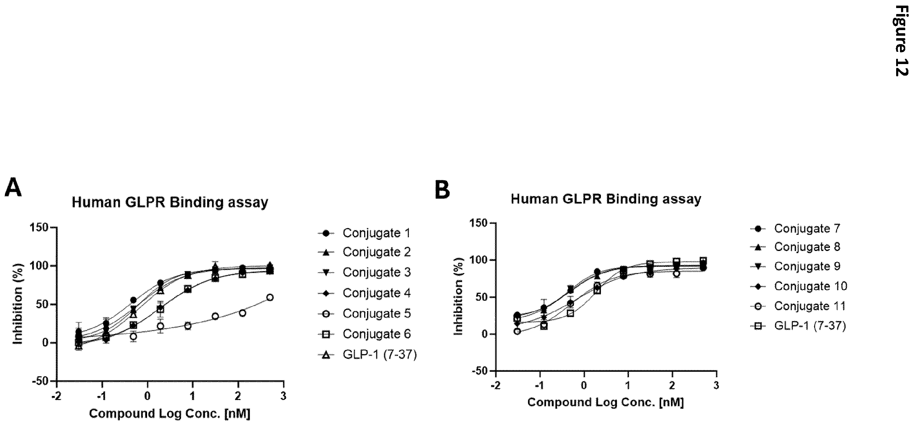

Figure 12 shows results of the affinity measurements of the conjugates of the present inventions to human GLPR.
Figure 13 shows results of the measurements of functional GLPR activity in a cAMP assay using mouse GLP-1R.

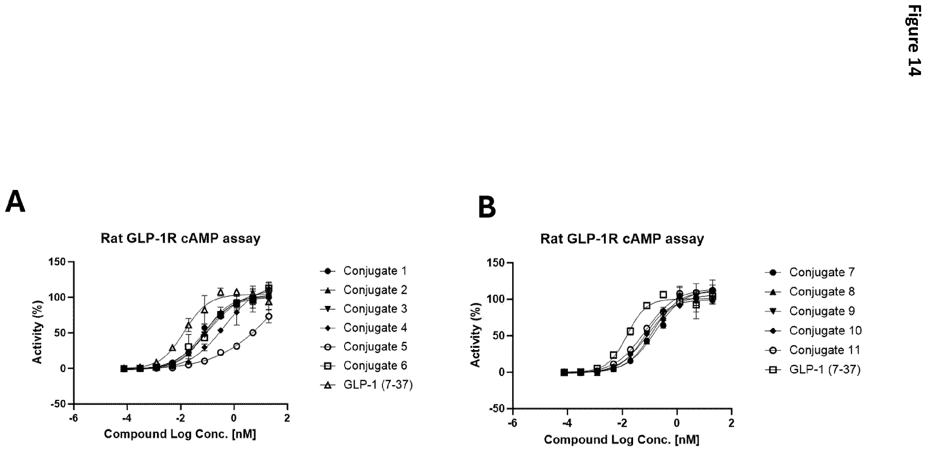

Figure 14 shows results of the measurements of functional GLPR activity in a cAMP assay using rat GLP-1R.

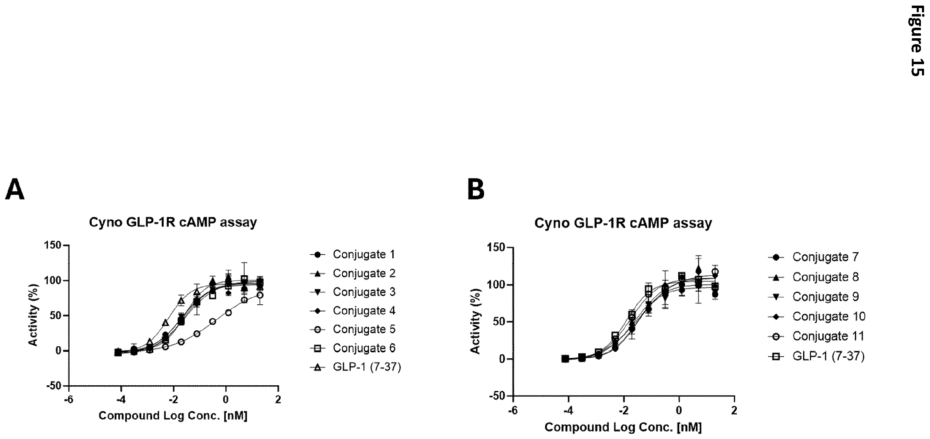

Figure 15 shows results of the measurements of functional GLPR activity in a cAMP assay using cynomolgus monkey GLP-1R.

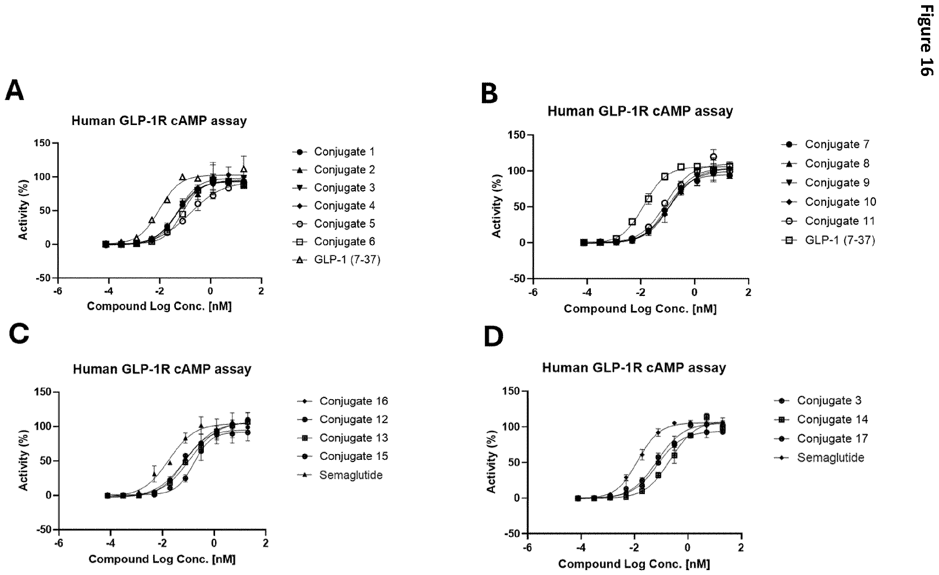

Figure 16 shows results of the measurements of functional GLPR activity in a cAMP assay using human GLP-1R.
Figure 17 and 18 show the results of the measurements of functional GLPR activity in a Reporter Gene Assay.
Figure 19 shows in vivo efficacy on food intake of Conjugate 1-5 vs vehicle, Semaglutide, unconjugated reference antibody and Conjugate 6. Reference A = unconjugated antibody with variable heavy chain of SEQ ID No. 28 and variable light chain of SEQ ID No. 29.
Figure 20 shows in vivo efficacy on body weight lowering and body composition of Conjugate 1-5 vs vehicle, Semaglutide, unconjugated reference antibody and Conjugate 6. Reference A = unconjugated antibody with variable heavy chain of SEQ ID No. 28 and variable light chain of SEQ ID No. 29.
Figure 21 shows an alternative representation of the in vivo efficacy on body weight lowering and body composition of Conjugate 1-5 vs vehicle, Semaglutide, unconjugated reference antibody and Conjugate 6. Reference A = unconjugated antibody with variable heavy chain of SEQ ID No. 28 and variable light chain of SEQ ID No. 29.
Figure 22 shows in vivo efficacy on food intake of Conjugate 3, & 17 vs unconjugated reference antibody and Conjugate 14. Reference A = unconjugated antibody with variable heavy chain of SEQ ID No. 28 and variable light chain of SEQ ID No. 29.
Figure 23 shows in vivo efficacy on fat mass reduction and lean mass impact of Conjugate 3, & 17 vs unconjugated reference antibody and Conjugate 14. Reference A = unconjugated antibody with variable heavy chain of SEQ ID No. 28 and variable light chain of SEQ ID No. 29.
Figure 24 shows the molecular structure of Compounds 1 ( top), 2 (middle) and 3 (bottom) after conjugation to the antibody.
Figure 25 shows the molecular structure of Compounds 4 ( top), 5 (middle) and 6 (bottom) after conjugation to the antibody.
Figure 26 shows the molecular structure of Compounds 7 ( top), 8 (middle) and 9 (bottom) after conjugation to the antibody.
Figure 27 shows the molecular structure of Compounds 10 ( top), 11 (middle) and 12 (bottom) after conjugation to the antibody.
Figure 28 shows the molecular structure of Compounds 13 ( top), 14 (middle) and 15 (bottom) after conjugation to the antibody.

### Summary of the invention

The present invention relates to an immunoconjugate comprising an ActRII receptor antibody and an agonistic GLP-1 peptide.

In certain embodiments, said immunoconjugate comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 30 (CDRH2), and SEQ ID NO: 22 (CDRH3) and a variable light chain comprising the CDR amino acid sequences of SEQ ID NO: 31 (CDRL1), SEQ ID NO: 32 (CDRL2), and SEQ ID NO: 33 (CDRL3), and wherein said agonistic GLP-1 peptide comprises the amino acid sequence of SEQ ID No. 79.

In certain embodiments, said agonistic GLP-1 peptide is comprised in a compound comprising the amino acid sequence of SEQ ID No. 64 or 69.

In certain embodiments, said ActRII receptor antibody comprises a variable heavy chain comprising the amino acid sequences of SEQ ID NO: 28 and a variable light chain comprising the amino acid sequences of SEQ ID NO: 29.

In certain embodiments, said ActRII receptor antibody specifically binds to a polypeptide of SEQ ID No. 58 and a polypeptide of SEQ ID No. 59.

In certain embodiments, said agonistic GLP-1 peptide or said compounds is conjugated to said ActRII receptor antibody via an engineered cysteine in the Fc region of said ActRII receptor antibody. In certain embodiments, said engineered cysteine is at position 272 of the constant region of the heavy chain (EU numbering).

In certain embodiments, said ActRII receptor antibody comprises the mutations L234A, L235A and P329A in the Fc region (EU numbering).

In certain embodiments, said ActRII receptor antibody comprises a heavy chain comprising the amino acid sequences of SEQ ID NO: 26 and a variable light chain comprising the amino acid sequences of SEQ ID NO: 27.

In certain embodiments, the present invention relates to a pharmaceutical composition comprising any one of aforementioned immunoconjugates.

In certain embodiments, the present invention relates to any one of aforementioned immunoconjugates or aforementioned pharmaceutical composition for use in medicine.

In certain embodiments, the present invention relates to a pharmaceutical composition comprising any one of aforementioned immunoconjugates.

In certain embodiments, the present invention relates to any one of aforementioned immunoconjugates or aforementioned pharmaceutical composition for use in medicine.

In certain embodiments, said use in medicine is the use in the treatment of a metabolic disorder.

In certain embodiments, said metabolic disorder is selected from the group consisting of: obesity, diabetes, metabolic syndrome, anti-psychotic drug-associated obesity, glucocorticoid-induced obesity, hypothalamic obesity associated with craniopharyngioma, Prader-Willi syndrome, and a monogenetic disorder associated with obesity.

In certain embodiments, said treatment is useful for an obesity related co-morbidity, wherein the condition is selected from the group of: glucose intolerance, prediabetes, insulin resistance, high triglycerides, overweight associated physical impairment, osteoporosis, renal disease, obstructive sleep apnea, sexual hormones impairment, endocrine reproductive disorders, osteoarthritis, gastrointestinal cancers, dyslipidemia, hypertension, heart failure, coronary heart disease, stroke, and/or gallstones.

### Embodiments of the invention

### ActRII antibody

The term "**antibody**" as used herein refers to a protein comprising at least two heavy (H) chains and two light (L) chains inter-connected by disulfide bonds, which interacts with an antigen. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs arranged from amino-terminus to carboxy-terminus in the following order: FR1, CDR1, FR2, CDR2, FR3, CDR3, and FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system. The term "antibody" includes for example, monoclonal antibodies, human antibodies, humanized antibodies, camelised antibodies and chimeric antibodies. The antibodies can be of any isotype (e.g., IgG, IgE, IgM, IgD, IgA and IgY), class (e.g., IgD , IgG2, IgG3, IgG4, IgA1 and IgA2) or subclass. Both the light and heavy chains are divided into regions of structural and functional homology.

The terms "**binds specifically to**", "**specifically binds to**", is "specific **to/for**" or "**specifically recognizes**" in the context of an antibody, refers to an antibody that is specific for an antigen and is able to discriminate between the target antigen and one or more reference antigen(s). Specificity is not an absolute, but a relative property. For example, a standard ELISA assay or standard flow cytometry assay can be carried out. The scoring may be carried out by standard color development (e.g. secondary antibody with horseradish peroxide and tetramethyl benzidine with hydrogen peroxide) or by binding of a secondary antibody labeled with PE or another dye or marker. The reaction in certain wells is scored by the optical density (OD), for example, at 450 nm or by mean fluorescence intensity (MFI) in flow cytometry. Typical background (=negative reaction) may be 0.1 OD; typical positive reaction may be 1 OD. Background and positive reaction MFI are highly dependent on instrument settings. The difference positive/negative can be more than 10-fold. Typically, determination of binding specificity is performed by using not a single reference antigen, but a set of about three to five unrelated antigens, such as milk powder, BSA, transferrin or the like. For flow cytometry various antigen-negative cells can be used. An antibody that specifically binds to an antigen may however have cross-reactivity to the respective orthologous antigen from other species (e.g., species homologs). In certain embodiments such cross-reactivity to an orthologous antigen is even preferred.

As used herein, an antibody has "**cross-reactivity**" or is "**cross-reactive**" if it binds to a closely related antigen or the same antigen from other species. In the present disclosure the term is used for an antibody that is specific for ActRllB but that also binds to ActRIIA.

The term "**ActRII receptor antibody**" as used herein refers to an antibody specific for ActRIIA, antibodies specific for ActRllB, and antibodies specific for ActRIIA and ActRllB, i.e. antibodies with a cross-reactivity between ActRIIA and ActRIIB.

The terms "**ActRIIA**" and "**ACVR2A**" as used herein refers to the human protein with UniProt ID P27037. ActRIIA has the following amino acid sequence:

MGAAAKLAFAVFLISCSSGAILGRSETQECLFFNANWEKDRTNQTGVEPCYGDKDKRRHC
FATWKNISGSIEIVKQGCWLDDINCYDRTDCVEKKDSPEVYFCCCEGNMCNEKFSYFPEM
EVTQPTSNPVTPKPPYYNILLYSLVPLMLIAGIVICAFWVYRHHKMAYPPVLVPTQDPGP
PPPSPLLGLKPLQLLEVKARGRFGCVWKAQLLNEYVAVKIFPIQDKQSWQNEYEVYSLPG
MKHENILQFIGAEKRGTSVDVDLWLITAFHEKGSLSDFLKANVVSWNELCHIAETMARGL
AYLHEDIPGLKDGHKPAISHRDIKSKNVLLKNNLTACIADFGLALKFEAGKSAGDTHGQV
GTRRYMAPEVLEGAINFQRDAFLRIDMYAMGLVLWELASRCTAADGPVDEYMLPFEEEIG
QHPSLEDMQEVVVHKKKRPVLRDYWQKHAGMAMLCETIEECWDHDAEARLSAGCVGERIT
QMQRLTNIITTEDIVTVVTMVTNVDFPPKESSL (SEQ ID No. 58)

The terms "**ActRIIB** and "**ACVR2B**" as used herein refers to the human protein with UniProt ID Q13705.









 (SEQ ID No. 59)

Activin receptor II B (ActRIIB) is a receptor for myostatin, activin, and bone morphogenetic proteins (BMPs). The interaction between myostatin and this receptor regulates the inhibition of skeletal muscle differentiation via the Smad-dependent pathway. It is thought that by inhibiting or preventing myostatin from binding to ActRllB, e.g, via an ActRII receptor antibody, the formation of skeletal muscle can be induced. Regulation of the activin receptor II A (ActRIIA) also plays a role in the regulation of muscle growth (Morvan et al. 2017). ActRII receptor antibodies are known in the art (e.g. WO2010/125003, WO2013/063536, WO2013/188448, WO2014/172448, WO2017/156488, WO2020/243448 and WO2021/174198).

In certain embodiments, the ActRII receptor antibody comprised in the immunoconjugates of the present disclosure comprises a variable heavy chain comprising the CDR amino acid sequences of SEQ ID NO: 20 (CDRH1), SEQ ID NO: 21 (CDRH2), and SEQ ID NO: 22 (CDRH3), and a variable light chain comprising the CDR amino acid sequences of SEQ ID NO: 23 (CDRL1), SEQ ID NO: 24 (CDRL2), and SEQ ID NO: 25 (CDRL3).

In certain embodiments, the ActRII receptor antibody comprised in the immunoconjugates of the present disclosure comprises a variable heavy chain comprising the CDR amino acid sequences of SEQ ID NO: 20 (CDRH1), SEQ ID NO: 30 (CDRH2), and SEQ ID NO: 22 (CDRH3), and a variable light chain comprising the CDR amino acid sequences of SEQ ID NO: 31 (CDRL1), SEQ ID NO: 32 (CDRL2), and SEQ ID NO: 33 (CDRL3).

In certain embodiments, the ActRII receptor antibody comprised in the immunoconjugates of the present disclosure comprises a variable heavy chain comprising the CDR amino acid sequences of SEQ ID NO: 20 (CDRH1), SEQ ID NO: 30 (CDRH2), and SEQ ID NO: 22 (CDRH3), and a variable light chain comprising the CDR amino acid sequences of SEQ ID NO: 37 (CDRL1), SEQ ID NO: 38 (CDRL2), and SEQ ID NO: 33 (CDRL3).

In certain embodiments, the ActRII receptor antibody comprised in the immunoconjugates of the present disclosure comprises a variable heavy chain comprising the CDR amino acid sequences of SEQ ID NO: 20 (CDRH1), SEQ ID NO: 21 (CDRH2), and SEQ ID NO: 22 (CDRH3), and a variable light chain comprising the CDR amino acid sequences of SEQ ID NO: 43 (CDRL1), SEQ ID NO: 32 (CDRL2), and SEQ ID NO: 44 (CDRL3).

In certain embodiments, the ActRII receptor antibody comprised in the immunoconjugates of the present disclosure comprises a variable heavy chain consisting of the CDR amino acid sequences of SEQ ID NO: 20 (CDRH1), SEQ ID NO: 21 (CDRH2), and SEQ ID NO: 22 (CDRH3), and a variable light chain consisting of the CDR amino acid sequences of SEQ ID NO: 23 (CDRL1), SEQ ID NO: 24 (CDRL2), and SEQ ID NO: 25 (CDRL3).

In certain embodiments, the ActRII receptor antibody comprised in the immunoconjugates of the present disclosure comprises a variable heavy chain consisting of the CDR amino acid sequences of SEQ ID NO: 20 (CDRH1), SEQ ID NO: 30 (CDRH2), and SEQ ID NO: 22 (CDRH3), and a variable light chain consisting of the CDR amino acid sequences of SEQ ID NO: 31 (CDRL1), SEQ ID NO: 32 (CDRL2), and SEQ ID NO: 33 (CDRL3).

In certain embodiments, the ActRII receptor antibody comprised in the immunoconjugates of the present disclosure comprises a variable heavy chain consisting of the CDR amino acid sequences of SEQ ID NO: 20 (CDRH1), SEQ ID NO: 30 (CDRH2), and SEQ ID NO: 22 (CDRH3), and a variable light chain consisting of the CDR amino acid sequences of SEQ ID NO: 37 (CDRL1), SEQ ID NO: 38 (CDRL2), and SEQ ID NO: 33 (CDRL3).

In certain embodiments, the ActRII receptor antibody comprised in the immunoconjugates of the present disclosure comprises a variable heavy chain consisting of the CDR amino acid sequences of SEQ ID NO: 20 (CDRH1), SEQ ID NO: 21 (CDRH2), and SEQ ID NO: 22 (CDRH3), and a variable light chain consisting of the CDR amino acid sequences of SEQ ID NO: 43 (CDRL1), SEQ ID NO: 32 (CDRL2), and SEQ ID NO: 44 (CDRL3).

In certain embodiments, the ActRII receptor antibody comprised in the immunoconjugates of the present disclosure comprises a variable heavy chain comprising the amino acid sequences of SEQ ID NO: 18 and a variable light chain comprising the amino acid sequences of SEQ ID NO: 19.

In certain embodiments, the ActRII receptor antibody comprised in the immunoconjugates of the present disclosure comprises a variable heavy chain comprising the amino acid sequences of SEQ ID NO: 28 and a variable light chain comprising the amino acid sequences of SEQ ID NO: 29.

In certain embodiments, the ActRII receptor antibody comprised in the immunoconjugates of the present disclosure comprises a variable heavy chain comprising the amino acid sequences of SEQ ID NO: 28 and a variable light chain comprising the amino acid sequences of SEQ ID NO: 36.

In certain embodiments, the ActRII receptor antibody comprised in the immunoconjugates of the present disclosure comprises a variable heavy chain comprising the amino acid sequences of SEQ ID NO: 41 and a variable light chain comprising the amino acid sequences of SEQ ID NO: 42.

In certain embodiments, the ActRII receptor antibody comprised in the immunoconjugates of the present disclosure comprises a variable heavy chain consisting of the amino acid sequences of SEQ ID NO: 18 and a variable light chain consisting of the amino acid sequences of SEQ ID NO: 19.In certain embodiments, the ActRII receptor antibody comprised in the immunoconjugates of the present disclosure comprises a variable heavy chain consisting of the amino acid sequences of SEQ ID NO: 28 and a variable light chain consisting of the amino acid sequences of SEQ ID NO: 29.

In certain embodiments, the ActRII receptor antibody comprised in the immunoconjugates of the present disclosure comprises a variable heavy chain consisting of the amino acid sequences of SEQ ID NO: 28 and a variable light chain consisting of the amino acid sequences of SEQ ID NO: 36.

In certain embodiments, the ActRII receptor antibody comprised in the immunoconjugates of the present disclosure comprises a variable heavy chain consisting of the amino acid sequences of SEQ ID NO: 41 and a variable light chain consisting of the amino acid sequences of SEQ ID NO: 42.

In certain embodiments, the ActRII receptor antibody comprised in the immunoconjugates of the present disclosure comprises a full length heavy chain comprising the amino acid sequences of SEQ ID NO: 18 and a full length light chain comprising the amino acid sequences of SEQ ID NO: 19.

In certain embodiments, the ActRII receptor antibody comprised in the immunoconjugates of the present disclosure comprises a full length heavy chain comprising the amino acid sequences of SEQ ID NO: 26 and a full length light chain comprising the amino acid sequences of SEQ ID NO: 27.

In certain embodiments, the ActRII receptor antibody comprised in the immunoconjugates of the present disclosure comprises a full length heavy chain comprising the amino acid sequences of SEQ ID NO: 28 and a full length light chain comprising the amino acid sequences of SEQ ID NO: 36.

In certain embodiments, the ActRII receptor antibody comprised in the immunoconjugates of the present disclosure comprises a full length heavy chain comprising the amino acid sequences of SEQ ID NO: 41 and a full length light chain comprising the amino acid sequences of SEQ ID NO: 42.

In certain embodiments, the ActRII receptor antibody comprised in the immunoconjugates of the present disclosure comprises a full length heavy chain consisting of the amino acid sequences of SEQ ID NO: 18 and a full length light chain consisting of the amino acid sequences of SEQ ID NO: 19.

In certain embodiments, the ActRII receptor antibody comprised in the immunoconjugates of the present disclosure comprises a full length heavy chain consisting of the amino acid sequences of SEQ ID NO: 26 and a full length light chain consisting of the amino acid sequences of SEQ ID NO: 27.

In certain embodiments, the ActRII receptor antibody comprised in the immunoconjugates of the present disclosure comprises a full length heavy chain consisting of the amino acid sequences of SEQ ID NO: 28 and a full length light chain consisting of the amino acid sequences of SEQ ID NO: 36.

In certain embodiments, the ActRII receptor antibody comprised in the immunoconjugates of the present disclosure comprises a full length heavy chain consisting of the amino acid sequences of SEQ ID NO: 41 and a full length light chain consisting of the amino acid sequences of SEQ ID NO: 42.

In certain embodiments, the ActRII receptor antibody comprised in the immunoconjugates of the present disclosure comprises a variable heavy chain comprising the amino acid sequence of SEQ ID NO: 18, or an amino acid sequence of at least 80 percent, 81 percent, 82 percent, 83 percent, 84 percent, 85 percent, 86 percent, 87 percent, 88 percent, 89 percent, 90 percent, 91 percent, 92 percent, 93 percent, 94 percent, 95 percent, 96 percent, 97 percent, 98 percent, or 99 percent sequence identity thereto; and/or a variable light chain comprising the amino acid sequence of SEQ ID NO: 19, or an amino acid sequence of at least 90 percent, 91 percent, 92 percent, 93 percent, 94 percent, 95 percent, 96 percent, 97 percent, 98 percent, or 99 percent sequence identity thereto.

In certain embodiments, the ActRII receptor antibody comprised in the immunoconjugates of the present disclosure comprises a variable heavy chain comprising the amino acid sequence of SEQ ID NO: 28, or an amino acid sequence of at least 80 percent, 81 percent, 82 percent, 83 percent, 84 percent, 85 percent, 86 percent, 87 percent, 88 percent, 89 percent, 90 percent, 91 percent, 92 percent, 93 percent, 94 percent, 95 percent, 96 percent, 97 percent, 98 percent, or 99 percent sequence identity thereto; and/or a variable light chain comprising the amino acid sequence of SEQ ID NO: 29, or an amino acid sequence of at least 90 percent, 91 percent, 92 percent, 93 percent, 94 percent, 95 percent, 96 percent, 97 percent, 98 percent, or 99 percent sequence identity thereto.

In certain embodiments, the ActRII receptor antibody comprised in the immunoconjugates of the present disclosure comprises a variable heavy chain comprising the amino acid sequence of SEQ ID NO: 28, or an amino acid sequence of at least 80 percent, 81 percent, 82 percent, 83 percent, 84 percent, 85 percent, 86 percent, 87 percent, 88 percent, 89 percent, 90 percent, 91 percent, 92 percent, 93 percent, 94 percent, 95 percent, 96 percent, 97 percent, 98 percent, or 99 percent sequence identity thereto; and/or a variable light chain comprising the amino acid sequence of SEQ ID NO: 36, or an amino acid sequence of at least 90 percent, 91 percent, 92 percent, 93 percent, 94 percent, 95 percent, 96 percent, 97 percent, 98 percent, or 99 percent sequence identity thereto.

In certain embodiments, the ActRII receptor antibody comprised in the immunoconjugates of the present disclosure comprises a variable heavy chain comprising the amino acid sequence of SEQ ID NO: 41, or an amino acid sequence of at least 80 percent, 81 percent, 82 percent, 83 percent, 84 percent, 85 percent, 86 percent, 87 percent, 88 percent, 89 percent, 90 percent, 91 percent, 92 percent, 93 percent, 94 percent, 95 percent, 96 percent, 97 percent, 98 percent, or 99 percent sequence identity thereto; and/or a variable light chain comprising the amino acid sequence of SEQ ID NO: 42, or an amino acid sequence of at least 90 percent, 91 percent, 92 percent, 93 percent, 94 percent, 95 percent, 96 percent, 97 percent, 98 percent, or 99 percent sequence identity thereto.

In certain embodiments, the ActRII receptor antibody comprised in the immunoconjugates of the present disclosure comprises a heavy chain comprising the amino acid sequence of SEQ ID NO: 16, or an amino acid sequence of at least 80 percent, 81 percent, 82 percent, 83 percent, 84 percent, 85 percent, 86 percent, 87 percent, 88 percent, 89 percent, 90 percent, 91 percent, 92 percent, 93 percent, 94 percent, 95 percent, 96 percent, 97 percent, 98 percent, or 99 percent sequence identity thereto; and/or a light chain comprising the amino acid sequence of SEQ ID NO: 17, or an amino acid sequence of at least 90 percent, 91 percent, 92 percent, 93 percent, 94 percent, 95 percent, 96 percent, 97 percent, 98 percent, or 99 percent sequence identity thereto.

In certain embodiments, the ActRII receptor antibody comprised in the immunoconjugates of the present disclosure comprises a heavy chain comprising the amino acid sequence of SEQ ID NO: 26, or an amino acid sequence of at least 80 percent, 81 percent, 82 percent, 83 percent, 84 percent, 85 percent, 86 percent, 87 percent, 88 percent, 89 percent, 90 percent, 91 percent, 92 percent, 93 percent, 94 percent, 95 percent, 96 percent, 97 percent, 98 percent, or 99 percent sequence identity thereto; and/or a light chain comprising the amino acid sequence of SEQ ID NO: 27, or an amino acid sequence of at least 90 percent, 91 percent, 92 percent, 93 percent, 94 percent, 95 percent, 96 percent, 97 percent, 98 percent, or 99 percent sequence identity thereto.

In certain embodiments, the ActRII receptor antibody comprised in the immunoconjugates of the present disclosure comprises a heavy chain comprising the amino acid sequence of SEQ ID NO: 34, or an amino acid sequence of at least 80 percent, 81 percent, 82 percent, 83 percent, 84 percent, 85 percent, 86 percent, 87 percent, 88 percent, 89 percent, 90 percent, 91 percent, 92 percent, 93 percent, 94 percent, 95 percent, 96 percent, 97 percent, 98 percent, or 99 percent sequence identity thereto; and/or a light chain comprising the amino acid sequence of SEQ ID NO: 35, or an amino acid sequence of at least 90 percent, 91 percent, 92 percent, 93 percent, 94 percent, 95 percent, 96 percent, 97 percent, 98 percent, or 99 percent sequence identity thereto.

In certain embodiments, the ActRII receptor antibody comprised in the immunoconjugates of the present disclosure comprises a heavy chain comprising the amino acid sequence of SEQ ID NO: 39, or an amino acid sequence of at least 80 percent, 81 percent, 82 percent, 83 percent, 84 percent, 85 percent, 86 percent, 87 percent, 88 percent, 89 percent, 90 percent, 91 percent, 92 percent, 93 percent, 94 percent, 95 percent, 96 percent, 97 percent, 98 percent, or 99 percent sequence identity thereto; and/or a light chain comprising the amino acid sequence of SEQ ID NO: 40, or an amino acid sequence of at least 90 percent, 91 percent, 92 percent, 93 percent, 94 percent, 95 percent, 96 percent, 97 percent, 98 percent, or 99 percent sequence identity thereto.

In certain embodiments, the ActRII receptor antibody comprised in the immunoconjugates of the present disclosure specifically binds to ActRIIA. In certain embodiments, the ActRII receptor antibody comprised in the immunoconjugates of the present disclosure specifically binds to a polypeptide of SEQ ID No. 58. In certain embodiments, the ActRII receptor antibody comprised in the immunoconjugates of the present disclosure specifically binds to ActRIIB. In certain embodiments, the ActRII receptor antibody comprised in the immunoconjugates of the present disclosure specifically binds to a polypeptide of SEQ ID No. 59.

In certain embodiments, the ActRII receptor antibody comprised in the immunoconjugates of the present disclosure specifically binds to ActRIIA and ActRIIB. In certain embodiments, the ActRII receptor antibody comprised in the immunoconjugates of the present disclosure specifically binds to a polypeptide of SEQ ID No. 58 and a polypeptide of SEQ ID No. 59.

In certain embodiments, the ActRII receptor antibody comprised in the immunoconjugates of the present disclosure binds to ActRllB with a KD of 100 nM or less, 10 nM or less, 1 nM or less. Preferably, an ActRII receptor antibody binds to ActRllB with an affinity of 100 pM or less (i.e. 100 pM, 50 pM, 10 pM, 1 pM or less). In certain embodiments, the ActRII receptor antibody binds to ActRllB with an affinity of between 10 and 20 pM.

In certain embodiments, the ActRII receptor antibody comprised in the immunoconjugates of the present disclosure binds to a polypeptide of SEQ ID No. 59 with a KD of 100 nM or less, 10 nM or less, 1 nM or less. Preferably, the ActRII receptor antibody binds to a polypeptide of SEQ ID No. 59 with an affinity of 100 pM or less (i.e. 100 pM, 50 pM, 10 pM, 1 pM or less). In certain embodiments, the ActRII receptor antibody binds to a polypeptide of SEQ ID No. 59 with an affinity of between 10 and 20 pM.

In certain embodiments, the ActRII receptor antibody comprised in the immunoconjugates of the present disclosure binds to ActRllB with a 5 -fold greater affinity than to ActRIIA, more preferably 10-fold, still more preferably 50-fold, still more preferably 100-fold. In some embodiments, the ActRII receptor antibody binds to ActRIIA with an affinity of 100 pM or more (i.e. 250 pM, 500 pM, 1 nM, 5 nM or more).

In certain embodiments, the ActRII receptor antibody comprised in the immunoconjugates of the present disclosure binds to a polypeptide of SEQ ID No. 59 with a 5 -fold greater affinity than to a polypeptide of SEQ ID No. 58, more preferably 10-fold, still more preferably 50-fold, still more preferably 100-fold. In some embodiments, the ActRII receptor antibody binds to a polypeptide of SEQ ID No. 58 with an affinity of 100 pM or more (i.e. 250 pM, 500 pM, 1 nM, 5 nM or more).

The ActRII receptor comprised in the immunoconjugates of the present disclosure has a Fc domain. Said Fc domain may for example be a human IgG1 Fc domain, a human IgG2 Fc domain, a human IgG3, Fc domain, or a human IgG4 Fc domain. Preferably, said Fc domain may for example be a human IgG1 Fc domain,

In certain embodiments, the ActRII receptor antibody comprised in the immunoconjugates of the present disclosure contains an engineered cysteine. In certain embodiments, the ActRII receptor antibody comprised in the immunoconjugates of the present disclosure contains an engineered cysteine at position 272, 361, 384, 389 or 434. In certain embodiments, the ActRII receptor antibody comprised in the immunoconjugates of the present disclosure contains an engineered cysteine at position 272. In certain embodiments, the ActRII receptor antibody comprised in the immunoconjugates of the present disclosure contains a engineered cysteine at position 272, 361, 384, 389 or 434 (EU numbering). In certain embodiments, the ActRII receptor antibody comprised in the immunoconjugates of the present disclosure contains an engineered cysteine at position 272 (EU numbering).

The terms "**silent**", "**silenced**" as used herein in the context of a silenced antibody or an antibody comprising a silencing mutation refers to a mutation in the Fc domain of such antibody which decreases, partially or wholly, binding to one or more cell surface Fcgamma receptors, thereby reducing or dampening, and in some embodiments abrogating substantially completely, one or more Fc-mediated antibody effector functions, such as ADCC, ADCP, and CDC complement response (see, e.g., Kang and Jung, Experimental and Molecular Medicine (2019) 51:138). Silenced effector functions can be obtained by mutation in the Fc region of the antibody and have been described in the art (e.g., Strohl, Biotechnology 20: 685-91 for LALA and N297A; Baudino et al., J. Immunol. 181: 6664-69 for D265A). Other exemplary Fc silencing mutations include amino acid substitutions at one or more of positions E233, L234, L235, G236, N297, P331 and P329 (see e.g. U.S. Pat. Nos.6,737,056, 7,332,581; WO 2004/056312, WO2021/234402, and Shields, R. L. et al., J. Biol. Chem.276 (2001) 6591-6604). Silencing mutations also include (numbering according EU index) the LALA (L234A/L235A), the PA-LALA (L234A/L235A/P329A) and the PG-LALA (L234A/L235A/P329G) mutations, as well as the AEASS mutations (L234A/L235E/G237A/A330S/P331S).

In certain embodiments, the ActRII receptor antibody comprised in the immunoconjugates of the present disclosure comprises a silencing Fc modification. In certain embodiments, the ActRII receptor antibody comprised in the immunoconjugates of the present disclosure comprises a silencing Fc modification selected from a LALA mutation, a PA-LALA mutation, a PG-LALA mutation and a AEASS mutation. In certain embodiments, the ActRII receptor antibody comprised in the immunoconjugates of the present disclosure comprises a silencing Fc modification, wherein said silencing Fc modification is a PA-LALA mutation.

In certain embodiments, the ActRII receptor antibody comprised in the immunoconjugates of the present disclosure contains a L234A, a L235A and a P329A mutation in the Fc region. In certain embodiments, the ActRII receptor antibody comprised in the immunoconjugates of the present disclosure contains a L234A, a L235A and a P329A mutation (EU numbering) in the Fc region. In certain embodiments, the ActRII receptor antibody comprised in the immunoconjugates of the present disclosure comprises the mutations L234A, L235A and P329A in the Fc region. In certain embodiments, the ActRII receptor antibody comprised in the immunoconjugates of the present disclosure comprises the mutations L234A, L235A and P329A (EU numbering) in the Fc region.

In certain embodiments, the Fc domain of the ActRII receptor antibody comprised in the immunoconjugates of the present disclosure is modified to increase serum half-life.

In certain embodiments, the ActRII receptor antibody comprised in the immunoconjugates of the present disclosure comprises modifications that increase serum half-life selected from one or more of substitution of the following residues: 2591, 252Y, 307Q, 308F, 428L, 434H, 434F, 434Y, 434A, 434M, and 434S (EU numbering). In In certain embodiments, the ActRII receptor antibody comprised in the immunoconjugates of the present disclosure comprises modifications selected from 259I/434S, 308F/434S, 308F/428L/434S, 259I/308F/434S, 307Q/308F/434S, 250I/308F/434S, and 308F1319L/434S (EU numbering scheme).

In certain embodiments, the ActRII receptor antibody comprised in the immunoconjugates of the present disclosure comprise the mutations M252Y, S254T and T256E (EU numbering). This mutation is referred to as "**YTE mutations**". Antibodies carrying a YTE mutation are reported to increase the half-life of the antibody (WO2002/060919)

In certain embodiments, the ActRII receptor antibody comprised in the immunoconjugates of the present disclosure comprises a variable heavy chain comprising the CDR amino acid sequences of SEQ ID NO: 20 (CDRH1), SEQ ID NO: 21 (CDRH2), and SEQ ID NO: 22 (CDRH3), a variable light chain comprising the CDR amino acid sequences of SEQ ID NO: 23 (CDRL1), SEQ ID NO: 24 (CDRL2), and SEQ ID NO: 25 (CDRL3), and the mutations L234A, L235A and P329A (EU numbering) in the Fc region.

In certain embodiments, the ActRII receptor antibody comprised in the immunoconjugates of the present disclosure comprises a variable heavy chain comprising the CDR amino acid sequences of SEQ ID NO: 20 (CDRH1), SEQ ID NO: 30 (CDRH2), and SEQ ID NO: 22 (CDRH3), a variable light chain comprising the CDR amino acid sequences of SEQ ID NO: 31 (CDRL1), SEQ ID NO: 32 (CDRL2), and SEQ ID NO: 33 (CDRL3), and the mutations L234A, L235A and P329A (EU numbering) in the Fc region.

In certain embodiments, the ActRII receptor antibody comprised in the immunoconjugates of the present disclosure comprises a variable heavy chain comprising the CDR amino acid sequences of SEQ ID NO: 20 (CDRH1), SEQ ID NO: 30 (CDRH2), and SEQ ID NO: 22 (CDRH3), a variable light chain comprising the CDR amino acid sequences of SEQ ID NO: 37 (CDRL1), SEQ ID NO: 38 (CDRL2), and SEQ ID NO: 33 (CDRL3), and the mutations L234A, L235A and P329A (EU numbering) in the Fc region.

In certain embodiments, the ActRII receptor antibody comprised in the immunoconjugates of the present disclosure comprises a variable heavy chain comprising the CDR amino acid sequences of SEQ ID NO: 20 (CDRH1), SEQ ID NO: 21 (CDRH2), and SEQ ID NO: 22 (CDRH3), a variable light chain comprising the CDR amino acid sequences of SEQ ID NO: 43 (CDRL1), SEQ ID NO: 32 (CDRL2), and SEQ ID NO: 44 (CDRL3), and the mutations L234A, L235A and P329A (EU numbering) in the Fc region.

In certain embodiments, the ActRII receptor antibody comprised in the immunoconjugates of the present disclosure comprises a variable heavy chain consisting of the CDR amino acid sequences of SEQ ID NO: 20 (CDRH1), SEQ ID NO: 21 (CDRH2), and SEQ ID NO: 22 (CDRH3), a variable light chain consisting of the CDR amino acid sequences of SEQ ID NO: 23 (CDRL1), SEQ ID NO: 24 (CDRL2), and SEQ ID NO: 25 (CDRL3), and the mutations L234A, L235A and P329A (EU numbering) in the Fc region.

In certain embodiments, the ActRII receptor antibody comprised in the immunoconjugates of the present disclosure comprises a variable heavy chain consisting of the CDR amino acid sequences of SEQ ID NO: 20 (CDRH1), SEQ ID NO: 30 (CDRH2), and SEQ ID NO: 22 (CDRH3), a variable light chain consisting of the CDR amino acid sequences of SEQ ID NO: 31 (CDRL1), SEQ ID NO: 32 (CDRL2), and SEQ ID NO: 33 (CDRL3), and the mutations L234A, L235A and P329A (EU numbering) in the Fc region.

In certain embodiments, the ActRII receptor antibody comprised in the immunoconjugates of the present disclosure comprises a variable heavy chain consisting of the CDR amino acid sequences of SEQ ID NO: 20 (CDRH1), SEQ ID NO: 30 (CDRH2), and SEQ ID NO: 22 (CDRH3), a variable light chain consisting of the CDR amino acid sequences of SEQ ID NO: 37 (CDRL1), SEQ ID NO: 38 (CDRL2), and SEQ ID NO: 33 (CDRL3), and the mutations L234A, L235A and P329A (EU numbering) in the Fc region.

In certain embodiments, the ActRII receptor antibody comprised in the immunoconjugates of the present disclosure comprises a variable heavy chain consisting of the CDR amino acid sequences of SEQ ID NO: 20 (CDRH1), SEQ ID NO: 21 (CDRH2), and SEQ ID NO: 22 (CDRH3), a variable light chain consisting of the CDR amino acid sequences of SEQ ID NO: 43 (CDRL1), SEQ ID NO: 32 (CDRL2), and SEQ ID NO: 44 (CDRL3), and the mutations L234A, L235A and P329A (EU numbering) in the Fc region.

In certain embodiments, the ActRII receptor antibody comprised in the immunoconjugates of the present disclosure comprises a variable heavy chain comprising the amino acid sequences of SEQ ID NO: 18, a variable light chain comprising the amino acid sequences of SEQ ID NO: 19, and the mutations L234A, L235A and P329A (EU numbering) in the Fc region.

In certain embodiments, the ActRII receptor antibody comprised in the immunoconjugates of the present disclosure comprises a variable heavy chain comprising the amino acid sequences of SEQ ID NO: 28, a variable light chain comprising the amino acid sequences of SEQ ID NO: 29, and the mutations L234A, L235A and P329A (EU numbering) in the Fc region.

In certain embodiments, the ActRII receptor antibody comprised in the immunoconjugates of the present disclosure comprises a variable heavy chain comprising the amino acid sequences of SEQ ID NO: 28, a variable light chain comprising the amino acid sequences of SEQ ID NO: 36, and the mutations L234A, L235A and P329A (EU numbering) in the Fc region.

In certain embodiments, the ActRII receptor antibody comprised in the immunoconjugates of the present disclosure comprises a variable heavy chain comprising the amino acid sequences of SEQ ID NO: 41, a variable light chain comprising the amino acid sequences of SEQ ID NO: 42, and the mutations L234A, L235A and P329A (EU numbering) in the Fc region.

In certain embodiments, the ActRII receptor antibody comprised in the immunoconjugates of the present disclosure comprises a variable heavy chain consisting of the amino acid sequences of SEQ ID NO: 18, a variable light chain consisting of the amino acid sequences of SEQ ID NO: 19, and the mutations L234A, L235A and P329A (EU numbering) in the Fc region.

In certain embodiments, the ActRII receptor antibody comprised in the immunoconjugates of the present disclosure comprises a variable heavy chain consisting of the amino acid sequences of SEQ ID NO: 28, a variable light chain consisting of the amino acid sequences of SEQ ID NO: 29, and the mutations L234A, L235A and P329A (EU numbering) in the Fc region.

In certain embodiments, the ActRII receptor antibody comprised in the immunoconjugates of the present disclosure comprises a variable heavy chain consisting of the amino acid sequences of SEQ ID NO: 28, a variable light chain consisting of the amino acid sequences of SEQ ID NO: 36, and the mutations L234A, L235A and P329A (EU numbering) in the Fc region.

In certain embodiments, the ActRII receptor antibody comprised in the immunoconjugates of the present disclosure comprises a variable heavy chain consisting of the amino acid sequences of SEQ ID NO: 41, a variable light chain consisting of the amino acid sequences of SEQ ID NO: 42, and the mutations L234A, L235A and P329A (EU numbering) in the Fc region.

In certain embodiments, the ActRII receptor antibody comprised in the immunoconjugates of the present disclosure comprises a variable heavy chain comprising the CDR amino acid sequences of SEQ ID NO: 20 (CDRH1), SEQ ID NO: 21 (CDRH2), and SEQ ID NO: 22 (CDRH3), a variable light chain comprising the CDR amino acid sequences of SEQ ID NO: 23 (CDRL1), SEQ ID NO: 24 (CDRL2), and SEQ ID NO: 25 (CDRL3), and an engineered cysteine at position 272 (EU numbering) in the Fc region.

In certain embodiments, the ActRII receptor antibody comprised in the immunoconjugates of the present disclosure comprises a variable heavy chain comprising the CDR amino acid sequences of SEQ ID NO: 20 (CDRH1), SEQ ID NO: 30 (CDRH2), and SEQ ID NO: 22 (CDRH3), a variable light chain comprising the CDR amino acid sequences of SEQ ID NO: 31 (CDRL1), SEQ ID NO: 32 (CDRL2), and SEQ ID NO: 33 (CDRL3), and an engineered cysteine at position 272 (EU numbering) in the Fc region.

In certain embodiments, the ActRII receptor antibody comprised in the immunoconjugates of the present disclosure comprises a variable heavy chain comprising the CDR amino acid sequences of SEQ ID NO: 20 (CDRH1), SEQ ID NO: 30 (CDRH2), and SEQ ID NO: 22 (CDRH3), a variable light chain comprising the CDR amino acid sequences of SEQ ID NO: 37 (CDRL1), SEQ ID NO: 38 (CDRL2), and SEQ ID NO: 33 (CDRL3), and an engineered cysteine at position 272 (EU numbering) in the Fc region.

In certain embodiments, the ActRII receptor antibody comprised in the immunoconjugates of the present disclosure comprises a variable heavy chain comprising the CDR amino acid sequences of SEQ ID NO: 20 (CDRH1), SEQ ID NO: 21 (CDRH2), and SEQ ID NO: 22 (CDRH3), a variable light chain comprising the CDR amino acid sequences of SEQ ID NO: 43 (CDRL1), SEQ ID NO: 32 (CDRL2), and SEQ ID NO: 44 (CDRL3), and an engineered cysteine at position 272 (EU numbering) in the Fc region.

In certain embodiments, the ActRII receptor antibody comprised in the immunoconjugates of the present disclosure comprises a variable heavy chain consisting of the CDR amino acid sequences of SEQ ID NO: 20 (CDRH1), SEQ ID NO: 21 (CDRH2), and SEQ ID NO: 22 (CDRH3), a variable light chain consisting of the CDR amino acid sequences of SEQ ID NO: 23 (CDRL1), SEQ ID NO: 24 (CDRL2), and SEQ ID NO: 25 (CDRL3), and an engineered cysteine at position 272 (EU numbering) in the Fc region.

In certain embodiments, the ActRII receptor antibody comprised in the immunoconjugates of the present disclosure comprises a variable heavy chain consisting of the CDR amino acid sequences of SEQ ID NO: 20 (CDRH1), SEQ ID NO: 30 (CDRH2), and SEQ ID NO: 22 (CDRH3), a variable light chain consisting of the CDR amino acid sequences of SEQ ID NO: 31 (CDRL1), SEQ ID NO: 32 (CDRL2), and SEQ ID NO: 33 (CDRL3), and an engineered cysteine at position 272 (EU numbering) in the Fc region.

In certain embodiments, the ActRII receptor antibody comprised in the immunoconjugates of the present disclosure comprises a variable heavy chain consisting of the CDR amino acid sequences of SEQ ID NO: 20 (CDRH1), SEQ ID NO: 30 (CDRH2), and SEQ ID NO: 22 (CDRH3), a variable light chain consisting of the CDR amino acid sequences of SEQ ID NO: 37 (CDRL1), SEQ ID NO: 38 (CDRL2), and SEQ ID NO: 33 (CDRL3), and an engineered cysteine at position 272 (EU numbering) in the Fc region.

In certain embodiments, the ActRII receptor antibody comprised in the immunoconjugates of the present disclosure comprises a variable heavy chain consisting of the CDR amino acid sequences of SEQ ID NO: 20 (CDRH1), SEQ ID NO: 21 (CDRH2), and SEQ ID NO: 22 (CDRH3), a variable light chain consisting of the CDR amino acid sequences of SEQ ID NO: 43 (CDRL1), SEQ ID NO: 32 (CDRL2), and SEQ ID NO: 44 (CDRL3), and an engineered cysteine at position 272 (EU numbering) in the Fc region.

In certain embodiments, the ActRII receptor antibody comprised in the immunoconjugates of the present disclosure comprises a variable heavy chain comprising the amino acid sequences of SEQ ID NO: 18, a variable light chain comprising the amino acid sequences of SEQ ID NO: 19, and an engineered cysteine at position 272 (EU numbering) in the Fc region.

In certain embodiments, the ActRII receptor antibody comprised in the immunoconjugates of the present disclosure comprises a variable heavy chain comprising the amino acid sequences of SEQ ID NO: 28, a variable light chain comprising the amino acid sequences of SEQ ID NO: 29, and an engineered cysteine at position 272 (EU numbering) in the Fc region.

In certain embodiments, the ActRII receptor antibody comprised in the immunoconjugates of the present disclosure comprises a variable heavy chain comprising the amino acid sequences of SEQ ID NO: 28, a variable light chain comprising the amino acid sequences of SEQ ID NO: 36, and an engineered cysteine at position 272 (EU numbering) in the Fc region.

In certain embodiments, the ActRII receptor antibody comprised in the immunoconjugates of the present disclosure comprises a variable heavy chain comprising the amino acid sequences of SEQ ID NO: 41, a variable light chain comprising the amino acid sequences of SEQ ID NO: 42, and an engineered cysteine at position 272 (EU numbering) in the Fc region.

In certain embodiments, the ActRII receptor antibody comprised in the immunoconjugates of the present disclosure comprises a variable heavy chain consisting of the amino acid sequences of SEQ ID NO: 18, a variable light chain consisting of the amino acid sequences of SEQ ID NO: 19, and an engineered cysteine at position 272 (EU numbering) in the Fc region.

In certain embodiments, the ActRII receptor antibody comprised in the immunoconjugates of the present disclosure comprises a variable heavy chain consisting of the amino acid sequences of SEQ ID NO: 28, a variable light chain consisting of the amino acid sequences of SEQ ID NO: 29, and an engineered cysteine at position 272 (EU numbering) in the Fc region.

In certain embodiments, the ActRII receptor antibody comprised in the immunoconjugates of the present disclosure comprises a variable heavy chain consisting of the amino acid sequences of SEQ ID NO: 28, a variable light chain consisting of the amino acid sequences of SEQ ID NO: 36, and an engineered cysteine at position 272 (EU numbering) in the Fc region.

In certain embodiments, the ActRII receptor antibody comprised in the immunoconjugates of the present disclosure comprises a variable heavy chain consisting of the amino acid sequences of SEQ ID NO: 41, a variable light chain consisting of the amino acid sequences of SEQ ID NO: 42, and an engineered cysteine at position 272 (EU numbering) in the Fc region.

In certain embodiments, the ActRII receptor antibody comprised in the immunoconjugates of the present disclosure comprises a variable heavy chain comprising the CDR amino acid sequences of SEQ ID NO: 20 (CDRH1), SEQ ID NO: 21 (CDRH2), and SEQ ID NO: 22 (CDRH3), a variable light chain comprising the CDR amino acid sequences of SEQ ID NO: 23 (CDRL1), SEQ ID NO: 24 (CDRL2), and SEQ ID NO: 25 (CDRL3), the mutations L234A, L235A and P329A (EU numbering) in the Fc region, and an engineered cysteine at position 272 (EU numbering) in the Fc region.

In certain embodiments, the ActRII receptor antibody comprised in the immunoconjugates of the present disclosure comprises a variable heavy chain comprising the CDR amino acid sequences of SEQ ID NO: 20 (CDRH1), SEQ ID NO: 30 (CDRH2), and SEQ ID NO: 22 (CDRH3), a variable light chain comprising the CDR amino acid sequences of SEQ ID NO: 31 (CDRL1), SEQ ID NO: 32 (CDRL2), and SEQ ID NO: 33 (CDRL3), the mutations L234A, L235A and P329A (EU numbering) in the Fc region, and an engineered cysteine at position 272 (EU numbering) in the Fc region.

In certain embodiments, the ActRII receptor antibody comprised in the immunoconjugates of the present disclosure comprises a variable heavy chain comprising the CDR amino acid sequences of SEQ ID NO: 20 (CDRH1), SEQ ID NO: 30 (CDRH2), and SEQ ID NO: 22 (CDRH3), a variable light chain comprising the CDR amino acid sequences of SEQ ID NO: 37 (CDRL1), SEQ ID NO: 38 (CDRL2), and SEQ ID NO: 33 (CDRL3), the mutations L234A, L235A and P329A (EU numbering) in the Fc region, and an engineered cysteine at position 272 (EU numbering) in the Fc region.

In certain embodiments, the ActRII receptor antibody comprised in the immunoconjugates of the present disclosure comprises a variable heavy chain comprising the CDR amino acid sequences of SEQ ID NO: 20 (CDRH1), SEQ ID NO: 21 (CDRH2), and SEQ ID NO: 22 (CDRH3), a variable light chain comprising the CDR amino acid sequences of SEQ ID NO: 43 (CDRL1), SEQ ID NO: 32 (CDRL2), and SEQ ID NO: 44 (CDRL3), the mutations L234A, L235A and P329A (EU numbering) in the Fc region, and an engineered cysteine at position 272 (EU numbering) in the Fc region.

In certain embodiments, the ActRII receptor antibody comprised in the immunoconjugates of the present disclosure comprises a variable heavy chain consisting of the CDR amino acid sequences of SEQ ID NO: 20 (CDRH1), SEQ ID NO: 21 (CDRH2), and SEQ ID NO: 22 (CDRH3), a variable light chain consisting of the CDR amino acid sequences of SEQ ID NO: 23 (CDRL1), SEQ ID NO: 24 (CDRL2), and SEQ ID NO: 25 (CDRL3), the mutations L234A, L235A and P329A (EU numbering) in the Fc region, and an engineered cysteine at position 272 (EU numbering) in the Fc region.

In certain embodiments, the ActRII receptor antibody comprised in the immunoconjugates of the present disclosure comprises a variable heavy chain consisting of the CDR amino acid sequences of SEQ ID NO: 20 (CDRH1), SEQ ID NO: 30 (CDRH2), and SEQ ID NO: 22 (CDRH3), a variable light chain consisting of the CDR amino acid sequences of SEQ ID NO: 31 (CDRL1), SEQ ID NO: 32 (CDRL2), and SEQ ID NO: 33 (CDRL3), the mutations L234A, L235A and P329A (EU numbering) in the Fc region, and an engineered cysteine at position 272 (EU numbering) in the Fc region.

In certain embodiments, the ActRII receptor antibody comprised in the immunoconjugates of the present disclosure comprises a variable heavy chain consisting of the CDR amino acid sequences of SEQ ID NO: 20 (CDRH1), SEQ ID NO: 30 (CDRH2), and SEQ ID NO: 22 (CDRH3), a variable light chain consisting of the CDR amino acid sequences of SEQ ID NO: 37 (CDRL1), SEQ ID NO: 38 (CDRL2), and SEQ ID NO: 33 (CDRL3), the mutations L234A, L235A and P329A (EU numbering) in the Fc region, and an engineered cysteine at position 272 (EU numbering) in the Fc region.

In certain embodiments, the ActRII receptor antibody comprised in the immunoconjugates of the present disclosure comprises a variable heavy chain consisting of the CDR amino acid sequences of SEQ ID NO: 20 (CDRH1), SEQ ID NO: 21 (CDRH2), and SEQ ID NO: 22 (CDRH3), a variable light chain consisting of the CDR amino acid sequences of SEQ ID NO: 43 (CDRL1), SEQ ID NO: 32 (CDRL2), and SEQ ID NO: 44 (CDRL3), the mutations L234A, L235A and P329A (EU numbering) in the Fc region, and an engineered cysteine at position 272 (EU numbering) in the Fc region.

In certain embodiments, the ActRII receptor antibody comprised in the immunoconjugates of the present disclosure comprises a variable heavy chain comprising the amino acid sequences of SEQ ID NO: 18, a variable light chain comprising the amino acid sequences of SEQ ID NO: 19, the mutations L234A, L235A and P329A (EU numbering) in the Fc region, and an engineered cysteine at position 272 (EU numbering) in the Fc region.

In certain embodiments, the ActRII receptor antibody comprised in the immunoconjugates of the present disclosure comprises a variable heavy chain comprising the amino acid sequences of SEQ ID NO: 28, a variable light chain comprising the amino acid sequences of SEQ ID NO: 29, the mutations L234A, L235A and P329A (EU numbering) in the Fc region, and an engineered cysteine at position 272 (EU numbering) in the Fc region.

In certain embodiments, the ActRII receptor antibody comprised in the immunoconjugates of the present disclosure comprises a variable heavy chain comprising the amino acid sequences of SEQ ID NO: 28, a variable light chain comprising the amino acid sequences of SEQ ID NO: 36, the mutations L234A, L235A and P329A (EU numbering) in the Fc region, and an engineered cysteine at position 272 (EU numbering) in the Fc region.

In certain embodiments, the ActRII receptor antibody comprised in the immunoconjugates of the present disclosure comprises a variable heavy chain comprising the amino acid sequences of SEQ ID NO: 41, a variable light chain comprising the amino acid sequences of SEQ ID NO: 42, the mutations L234A, L235A and P329A (EU numbering) in the Fc region, and an engineered cysteine at position 272 (EU numbering) in the Fc region.

In certain embodiments, the ActRII receptor antibody comprised in the immunoconjugates of the present disclosure comprises a variable heavy chain consisting of the amino acid sequences of SEQ ID NO: 18, a variable light chain consisting of the amino acid sequences of SEQ ID NO: 19, the mutations L234A, L235A and P329A (EU numbering) in the Fc region, and an engineered cysteine at position 272 (EU numbering) in the Fc region.

In certain embodiments, the ActRII receptor antibody comprised in the immunoconjugates of the present disclosure comprises a variable heavy chain consisting of the amino acid sequences of SEQ ID NO: 28, a variable light chain consisting of the amino acid sequences of SEQ ID NO: 29, the mutations L234A, L235A and P329A (EU numbering) in the Fc region, and an engineered cysteine at position 272 (EU numbering) in the Fc region.

In certain embodiments, the ActRII receptor antibody comprised in the immunoconjugates of the present disclosure comprises a variable heavy chain consisting of the amino acid sequences of SEQ ID NO: 28, a variable light chain consisting of the amino acid sequences of SEQ ID NO: 36, the mutations L234A, L235A and P329A (EU numbering) in the Fc region, and an engineered cysteine at position 272 (EU numbering) in the Fc region.

In certain embodiments, the ActRII receptor antibody comprised in the immunoconjugates of the present disclosure comprises a variable heavy chain consisting of the amino acid sequences of SEQ ID NO: 41, a variable light chain consisting of the amino acid sequences of SEQ ID NO: 42, the mutations L234A, L235A and P329A (EU numbering) in the Fc region, and an engineered cysteine at position 272 (EU numbering) in the Fc region.

### GLP-1 agonists

The glucagon-like peptide-1 receptor is mainly recognized as a pancreatic beta cell receptor that stimulates insulin secretion upon binding of the hormone glucagon-like peptide-1 (GLP-1). GLP-1 is a type of incretin hormone, all of which regulate insulin release in response to blood sugar. GLP-1 agonists mimic the action of GLP-1 peptide and activate the GLP-1 receptor upon binding, stimulating insulin secretion. Besides its glucose-regulatory effects GLP-1 has also been identified to act in other tissues such as the brain, stomach, intestine, heart and muscle.

However, GLP-1 agonists exhibit significant side effects and tolerability issues at effective doses. GLP-1 agonists have also been shown to decrease lean mass of a subject during treatment. Warnings and precautions for GLP-1 agonists (e.g., semaglutide) include but are not limited to: thyroid C-cell tumors, acute pancreatitis, acute gallbladder disease, hypoglycemia, acute kidney injury, severe adverse gastrointestinal reactions, hypersensitivity, e.g., anaphylactic reactions and angioedema, diabetic retinopathy complications in subjects Type 2 Diabetes, heart rate increase, and suicidal behavior and ideation. Adverse reactions reported in at least 5 percent of subjects treated with a GLP-1 agonist (e.g., semaglutide), include nausea, diarrhea, vomiting, constipation, abdominal pain, headache, fatigue, dyspepsia, dizziness, abdominal distension, eructation, hypoglycemia in patients with type 2 diabetes, flatulence, gastroenteritis, and gastroesophageal reflux disease. Thus, there is a need for optimized dosage and treatments with GLP-1 agonists.

The term "**GLP-1 agonist**" as used herein refers to a compound, which fully or partially activates the human GLP-1 receptor. Principally, a GLP-1 agonist may be a peptide, antibody, small molecule, or an aptamer. Peptidic GLP-1 agonist or peptide analog, include but not limited to: exenatide, exenatide extended-release, dulaglutide, liraglutide, lixisenatide or semaglutide. An exemplary small molecule non-peptidic GLP-1 agonist is danuglipron. Other GLP-1 agonists are described in the literature. Compounds 1-15 disclosed herein are also exemplary GLP-1 agonists.

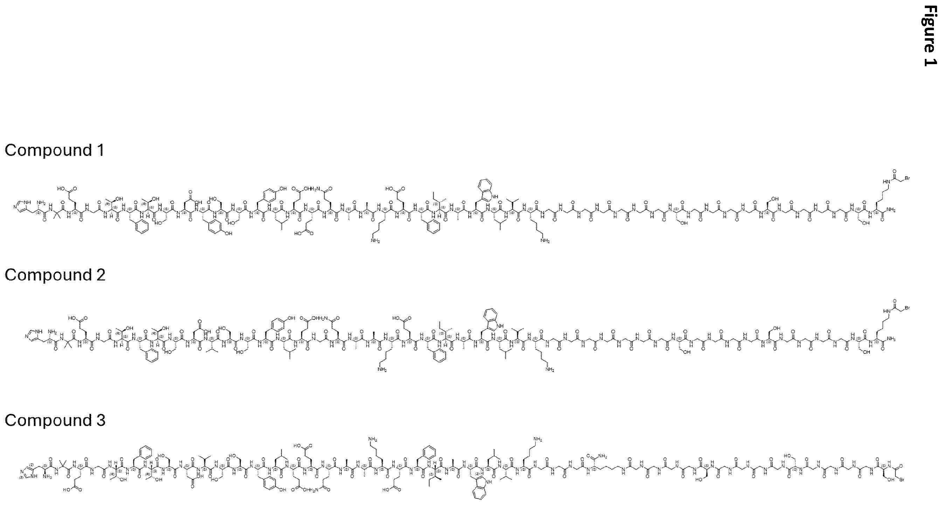

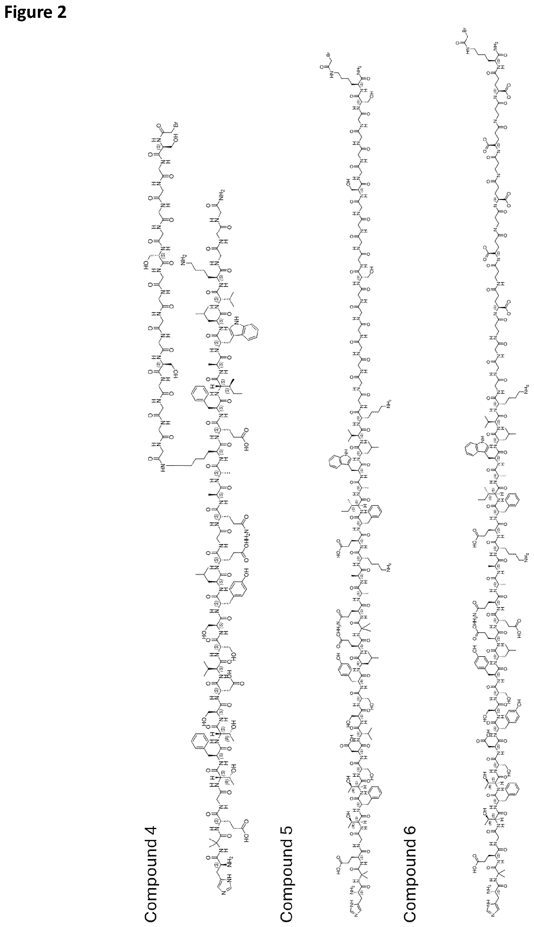

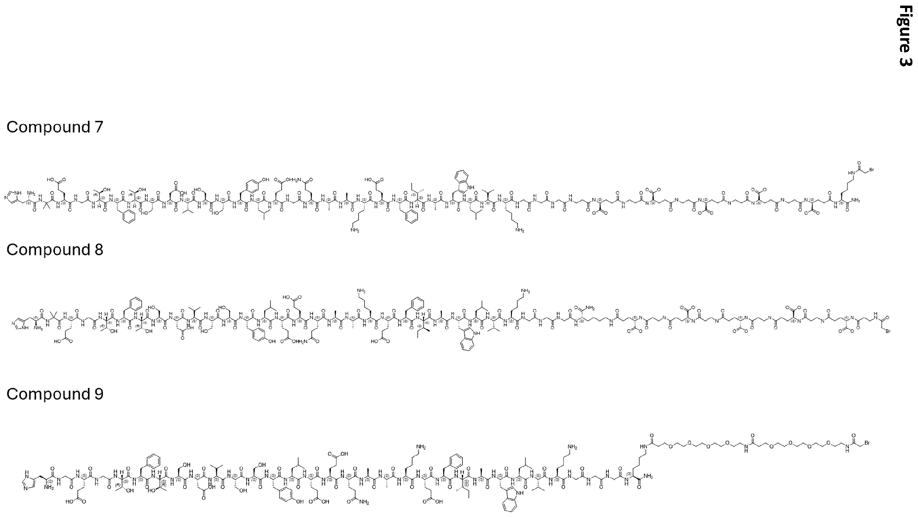

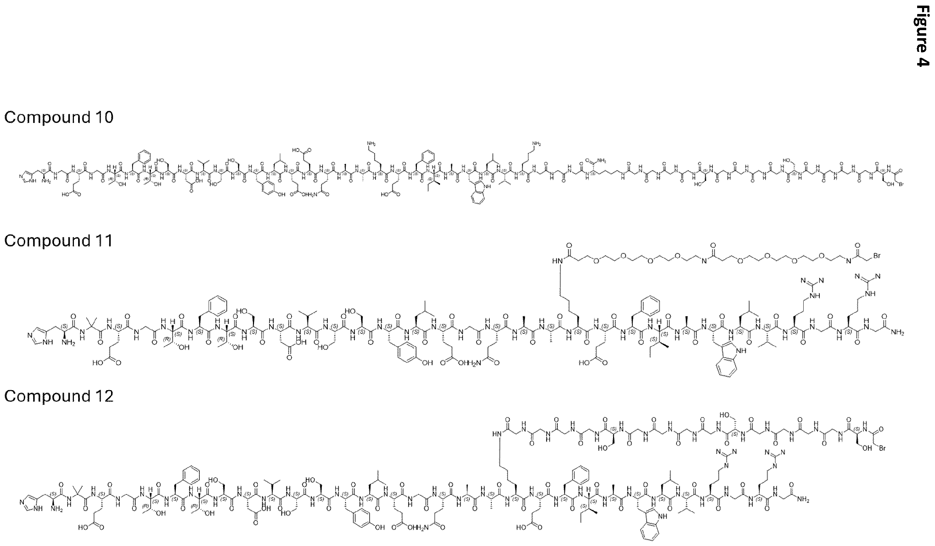

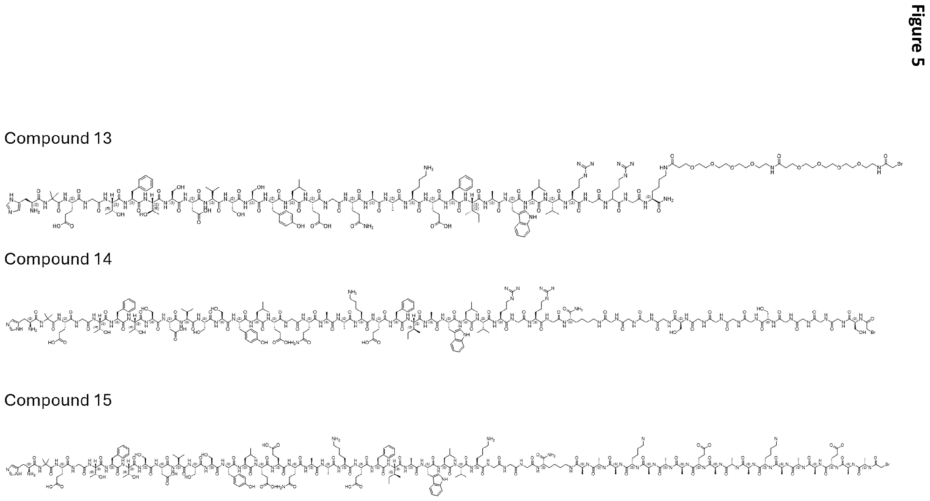

The following compounds were generated and tested as further described herein:
Compound 1:
   Structure see Figure 1 (top)
   HAibEGTFTSDYSSYLEEQAAKEFIAWLVKGGGGGGGSGGGGSGGGGSK(AcBr) (SEQ ID No. 1)
Compound 2:
   Structure see Figure 1 (middle)
   HAibEGTFTSDVSSYLEGQAAKEFIAWLVKGGGGGGGSGGGGSGGGGSK(AcBr) (SEQ ID No. 2)
Compound 3:
   Structure see Figure 1 (bottom)
   HAibEGTFTSDVSSYLEEQAAKEFIAWLVKGGGK(GGGGSGGGGSGGGGSAcBr) (SEQ ID No. 3)
Compound 4:
   Structure see Figure 2 (top)
   HAibEGTFTSDVSSYLEGQAAK(GGGGSGGGGSGGGGSAcBr)EFIAWLVKGGG (SEQ ID No. 4)
Compound 5:
   Structure see Figure 2 (middle)
   HAibEGTFTSDVSSylEAibQAAKEFIAWLVKGGGGGGGSGGGGSGGGGSK(AcBr) (SEQ ID No. 5)
Compound 6:
   Structure see Figure 2 (bottom)
   HAibEGTFTSDYSSYLEEQAAKEFIAWLVKGGGβAγEβAγEβAγEβAγEβAγEK(AcBr) (SEQ ID No. 6)
Compound 7:
   Structure see Figure 3 (top)
   HAibEGTFTSDVSSYLEGQAAKEFIAWLVKGGGβAγEβAγEβAγEβAγEβAγEK(AcBr) (SEQ ID No. 7)
Compound 8:
   Structure see Figure 3 (middle)
   HAibEGTFTSDVSSYLEEQAAKEFIAWLVKGGGK(γEβAγEβAγEβAγEβAγEβAAcBr) (SEQ ID No. 8)
Compound 9:
   Structure see Figure 3 (bottom)
   HGEGTFTSDVSSYLEEQAAKEFIAWLVKGGGK(MiniPEGAcBr) (SEQ ID No. 9)
Compound 10:
   Structure see Figure 4 (top)
   HGEGTFTSDVSSYLEEQAAKEFIAWLVKGGGK(GGGGSGGGGSGGGGSAcBr) (SEQ ID No. 10)
Compound 11:
   Structure see Figure 4 (middle)
   HAibEGTFTSDVSSYLEGQAAK(MiniPEGAcBr)EFIAWLVRGRG (SEQ ID No. 11)
Compound 12:
   Structure see Figure 4 (bottom)
   HAibEGTFTSDVSSYLEGQAAK(GGGGSGGGGSGGGGSAcBr)EFIAWLVRGRG (SEQ ID No. 12)
Compound 13:
   Structure see Figure 5 (top)
   HAibEGTFTSDVSSYLEGQAAKEFIAWLVRGRGK(MiniPEGAcBr) (SEQ ID No. 13)
Compound 14:
   Structure see Figure 5 (middle)
   HAibEGTFTSDVSSYLEGQAAKEFIAWLVRGRGK(GGGGSGGGGSGGGGSAcBr) (SEQ ID No. 14)
Compound 15:
   Structure see Figure 5 (bottom)
   HAibEGTFTSDVSSYLEEQAAKEFIAWLVKGGGK(AAAKAAAEAAAKAAAEAAAcBr) (SEQ ID No. 15)

Abbreviations:
Aib: Aminoisobutyric acid
BrAc: Bromoacetic acid
βA: β-Alanine
γE: γ-Glutamic acid
MiniPEG: 1-Amino-3,6,9,12-tetraoxapentadecan-15-oic acid - CAS 581065-95-4
MBHA Resin: 4-Methylbenzhydrylaminhydrochloride
DDE: 2-Acetyldimedone
MPA: 3-Mercaptopropionic acid
DIC: N,N'-Diisopropylcarbodiimid
TIS: Triisopropylsilane
HBTU: (2-(1H-Benzotriazol-1-yl)-1,1,3,3-tetramethyluronium-hexafluorophosphate

In certain embodiments, the agonistic GLP-1 peptide comprised in the immunoconjugates of the present disclosure comprises an agonistic GLP-1 peptide comprising an amino acid sequence selected from any one of SEQ ID No.'s 1-15.

In certain embodiments, the agonistic GLP-1 peptide comprised in the immunoconjugates of the present disclosure comprises an agonistic GLP-1 peptide comprising the amino acid sequence of SEQ ID No. 3. In certain embodiments, the agonistic GLP-1 peptide comprised in the immunoconjugates of the present disclosure comprises an agonistic GLP-1 peptide consisting of the amino acid sequence of SEQ ID No. 3.

In certain embodiments, the agonistic GLP-1 peptide comprised in the immunoconjugates of the present disclosure comprises an agonistic GLP-1 peptide comprising the amino acid sequence of SEQ ID No. 8. In certain embodiments, the agonistic GLP-1 peptide comprised in the immunoconjugates of the present disclosure comprises an agonistic GLP-1 peptide consisting of the amino acid sequence of SEQ ID No. 8.

In certain embodiments, the immunoconjugate of the present disclosure comprises an ActRII receptor antibody comprising a variable heavy chain comprising the CDR amino acid sequences of SEQ ID NO: 20 (CDRH1), SEQ ID NO: 21 (CDRH2), and SEQ ID NO: 22 (CDRH3), a variable light chain comprising the CDR amino acid sequences of SEQ ID NO: 23 (CDRL1), SEQ ID NO: 24 (CDRL2), and SEQ ID NO: 25 (CDRL3), and an agonistic GLP-1 peptide comprising an amino acid sequence selected from any one of SEQ ID No.'s 77-82.

In certain embodiments, the immunoconjugate of the present disclosure comprises an ActRII receptor antibody comprising a variable heavy chain comprising the CDR amino acid sequences of SEQ ID NO: 20 (CDRH1), SEQ ID NO: 30 (CDRH2), and SEQ ID NO: 22 (CDRH3), a variable light chain comprising the CDR amino acid sequences of SEQ ID NO: 31 (CDRL1), SEQ ID NO: 32 (CDRL2), and SEQ ID NO: 33 (CDRL3), and an agonistic GLP-1 peptide comprising an amino acid sequence selected from any one of SEQ ID No.'s 77-82.

In certain embodiments, the immunoconjugate of the present disclosure comprises an ActRII receptor antibody comprising a variable heavy chain comprising the CDR amino acid sequences of SEQ ID NO: 20 (CDRH1), SEQ ID NO: 30 (CDRH2), and SEQ ID NO: 22 (CDRH3), a variable light chain comprising the CDR amino acid sequences of SEQ ID NO: 37 (CDRL1), SEQ ID NO: 38 (CDRL2), and SEQ ID NO: 33 (CDRL3), and an agonistic GLP-1 peptide comprising an amino acid sequence selected from any one of SEQ ID No.'s 77-82.

In certain embodiments, the immunoconjugate of the present disclosure comprises an ActRII receptor antibody comprising a variable heavy chain comprising the CDR amino acid sequences of SEQ ID NO: 20 (CDRH1), SEQ ID NO: 21 (CDRH2), and SEQ ID NO: 22 (CDRH3), a variable light chain comprising the CDR amino acid sequences of SEQ ID NO: 43 (CDRL1), SEQ ID NO: 32 (CDRL2), and SEQ ID NO: 44 (CDRL3), and an agonistic GLP-1 peptide comprising an amino acid sequence selected from any one of SEQ ID No.'s 77-82.

In certain embodiments, the immunoconjugate of the present disclosure comprises an ActRII receptor antibody comprising a variable heavy chain comprising the CDR amino acid sequences of SEQ ID NO: 20 (CDRH1), SEQ ID NO: 21 (CDRH2), and SEQ ID NO: 22 (CDRH3), a variable light chain comprising the CDR amino acid sequences of SEQ ID NO: 23 (CDRL1), SEQ ID NO: 24 (CDRL2), and SEQ ID NO: 25 (CDRL3), and an agonistic GLP-1 peptide comprising the amino acid sequence of SEQ ID No. 79.

In certain embodiments, the immunoconjugate of the present disclosure comprises an ActRII receptor antibody comprising a variable heavy chain comprising the CDR amino acid sequences of SEQ ID NO: 20 (CDRH1), SEQ ID NO: 30 (CDRH2), and SEQ ID NO: 22 (CDRH3), a variable light chain comprising the CDR amino acid sequences of SEQ ID NO: 31 (CDRL1), SEQ ID NO: 32 (CDRL2), and SEQ ID NO: 33 (CDRL3), and an agonistic GLP-1 peptide comprising the amino acid sequence of SEQ ID No. 79.

In certain embodiments, the immunoconjugate of the present disclosure comprises an ActRII receptor antibody comprising a variable heavy chain comprising the CDR amino acid sequences of SEQ ID NO: 20 (CDRH1), SEQ ID NO: 30 (CDRH2), and SEQ ID NO: 22 (CDRH3), a variable light chain comprising the CDR amino acid sequences of SEQ ID NO: 37 (CDRL1), SEQ ID NO: 38 (CDRL2), and SEQ ID NO: 33 (CDRL3), and an agonistic GLP-1 peptide comprising the amino acid sequence of SEQ ID No. 79.

In certain embodiments, the immunoconjugate of the present disclosure comprises an ActRII receptor antibody comprising a variable heavy chain comprising the CDR amino acid sequences of SEQ ID NO: 20 (CDRH1), SEQ ID NO: 21 (CDRH2), and SEQ ID NO: 22 (CDRH3), a variable light chain comprising the CDR amino acid sequences of SEQ ID NO: 43 (CDRL1), SEQ ID NO: 32 (CDRL2), and SEQ ID NO: 44 (CDRL3), and an agonistic GLP-1 peptide comprising the amino acid sequence of SEQ ID No. 79.

In certain embodiments, the immunoconjugate of the present disclosure comprises an ActRII receptor antibody comprising a variable heavy chain comprising the amino acid sequences of SEQ ID NO: 18, a variable light chain comprising the amino acid sequences of SEQ ID NO: 19, and an agonistic GLP-1 peptide comprising an amino acid sequence selected from any one of SEQ ID No.'s 77-82.

In certain embodiments, the immunoconjugate of the present disclosure comprises an ActRII receptor antibody comprising a variable heavy chain comprising the amino acid sequences of SEQ ID NO: 28, a variable light chain comprising the amino acid sequences of SEQ ID NO: 29, and an agonistic GLP-1 peptide comprising an amino acid sequence selected from any one of SEQ ID No.'s 77-82.

In certain embodiments, the immunoconjugate of the present disclosure comprises an ActRII receptor antibody comprising a variable heavy chain comprising the amino acid sequences of SEQ ID NO: 28, a variable light chain comprising the amino acid sequences of SEQ ID NO: 36, and an agonistic GLP-1 peptide comprising an amino acid sequence selected from any one of SEQ ID No.'s 77-82.

In certain embodiments, the immunoconjugate of the present disclosure comprises an ActRII receptor antibody comprising a variable heavy chain comprising the amino acid sequences of SEQ ID NO: 41, a variable light chain comprising the amino acid sequences of SEQ ID NO: 42, and an agonistic GLP-1 peptide comprising an amino acid sequence selected from any one of SEQ ID No.'s 77-82.

In certain embodiments, the immunoconjugate of the present disclosure comprises an ActRII receptor antibody comprising a variable heavy chain comprising the amino acid sequences of SEQ ID NO: 18, a variable light chain comprising the amino acid sequences of SEQ ID NO: 19, and an agonistic GLP-1 peptide comprising the amino acid sequence of SEQ ID No. 79.

In certain embodiments, the immunoconjugate of the present disclosure comprises an ActRII receptor antibody comprising a variable heavy chain comprising the amino acid sequences of SEQ ID NO: 28, a variable light chain comprising the amino acid sequences of SEQ ID NO: 29, and an agonistic GLP-1 peptide comprising the amino acid sequence of SEQ ID No. 79.

In certain embodiments, the immunoconjugate of the present disclosure comprises an ActRII receptor antibody comprising a variable heavy chain comprising the amino acid sequences of SEQ ID NO: 28, a variable light chain comprising the amino acid sequences of SEQ ID NO: 36, and an agonistic GLP-1 peptide comprising the amino acid sequence of SEQ ID No. 79.

In certain embodiments, the immunoconjugate of the present disclosure comprises an ActRII receptor antibody comprising a variable heavy chain comprising the amino acid sequences of SEQ ID NO: 41, a variable light chain comprising the amino acid sequences of SEQ ID NO: 42, and an agonistic GLP-1 peptide comprising the amino acid sequence of SEQ ID No. 79.

In certain embodiments, the immunoconjugate of the present disclosure comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 21 (CDRH2), and SEQ ID NO: 22 (CDRH3) and a variable light chain comprising the CDR amino acid sequences of SEQ ID NO: 23 (CDRL1), SEQ ID NO: 24 (CDRL2), and SEQ ID NO: 25 (CDRL3), and wherein said agonistic GLP-1 peptide comprises the amino acid sequence of SEQ ID No. 79.

In certain embodiments, the immunoconjugate of the present disclosure comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 30 (CDRH2), and SEQ ID NO: 22 (CDRH3) and a variable light chain comprising the CDR amino acid sequences of SEQ ID NO: 31 (CDRL1), SEQ ID NO: 32 (CDRL2), and SEQ ID NO: 33 (CDRL3), and wherein said agonistic GLP-1 peptide comprises the amino acid sequence of SEQ ID No. 79.

In certain embodiments, the immunoconjugate of the present disclosure comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 30 (CDRH2), and SEQ ID NO: 22 (CDRH3) and a variable light chain comprising the CDR amino acid sequences of SEQ ID NO: 37 (CDRL1), SEQ ID NO: 38 (CDRL2), and SEQ ID NO: 33 (CDRL3), and wherein said agonistic GLP-1 peptide comprises the amino acid sequence of SEQ ID No. 79.

In certain embodiments, the immunoconjugate of the present disclosure comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 21 (CDRH2), and SEQ ID NO: 22 (CDRH3) and a variable light chain comprising the CDR amino acid sequences of SEQ ID NO: 43 (CDRL1), SEQ ID NO: 32 (CDRL2), and SEQ ID NO: 44 (CDRL3), and wherein said agonistic GLP-1 peptide comprises the amino acid sequence of SEQ ID No. 79.

In certain embodiments, the immunoconjugate of the present disclosure comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 21 (CDRH2), and SEQ ID NO: 22 (CDRH3) and a variable light chain comprising the CDR amino acid sequences of SEQ ID NO: 23 (CDRL1), SEQ ID NO: 24 (CDRL2), and SEQ ID NO: 25 (CDRL3), wherein said agonistic GLP-1 peptide comprises the amino acid sequence of SEQ ID No. 79, and wherein said agonistic GLP-1 peptide is comprised in a compound comprising the amino acid sequence of SEQ ID No. 64 or 69.

In certain embodiments, the immunoconjugate of the present disclosure comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 30 (CDRH2), and SEQ ID NO: 22 (CDRH3) and a variable light chain comprising the CDR amino acid sequences of SEQ ID NO: 31 (CDRL1), SEQ ID NO: 32 (CDRL2), and SEQ ID NO: 33 (CDRL3), wherein said agonistic GLP-1 peptide comprises the amino acid sequence of SEQ ID No. 79, and wherein said agonistic GLP-1 peptide is comprised in a compound comprising the amino acid sequence of SEQ ID No. 64 or 69.

In certain embodiments, the immunoconjugate of the present disclosure comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 30 (CDRH2), and SEQ ID NO: 22 (CDRH3) and a variable light chain comprising the CDR amino acid sequences of SEQ ID NO: 37 (CDRL1), SEQ ID NO: 38 (CDRL2), and SEQ ID NO: 33 (CDRL3), wherein said agonistic GLP-1 peptide comprises the amino acid sequence of SEQ ID No. 79, and wherein said agonistic GLP-1 peptide is comprised in a compound comprising the amino acid sequence of SEQ ID No. 64 or 69.

In certain embodiments, the immunoconjugate of the present disclosure comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 21 (CDRH2), and SEQ ID NO: 22 (CDRH3) and a variable light chain comprising the CDR amino acid sequences of SEQ ID NO: 43 (CDRL1), SEQ ID NO: 32 (CDRL2), and SEQ ID NO: 44 (CDRL3), wherein said agonistic GLP-1 peptide comprises the amino acid sequence of SEQ ID No. 79, and wherein said agonistic GLP-1 peptide is comprised in a compound comprising the amino acid sequence of SEQ ID No. 64 or 69.

In certain embodiments, the immunoconjugate of the present disclosure comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 21 (CDRH2), and SEQ ID NO: 22 (CDRH3) and a variable light chain comprising the CDR amino acid sequences of SEQ ID NO: 23 (CDRL1), SEQ ID NO: 24 (CDRL2), and SEQ ID NO: 25 (CDRL3), wherein said agonistic GLP-1 peptide comprises the amino acid sequence of SEQ ID No. 79, and wherein said agonistic GLP-1 peptide is comprised in a compound comprising the amino acid sequence of SEQ ID No. 64.

In certain embodiments, the immunoconjugate of the present disclosure comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 30 (CDRH2), and SEQ ID NO: 22 (CDRH3) and a variable light chain comprising the CDR amino acid sequences of SEQ ID NO: 31 (CDRL1), SEQ ID NO: 32 (CDRL2), and SEQ ID NO: 33 (CDRL3), wherein said agonistic GLP-1 peptide comprises the amino acid sequence of SEQ ID No. 79, and wherein said agonistic GLP-1 peptide is comprised in a compound comprising the amino acid sequence of SEQ ID No. 64.

In certain embodiments, the immunoconjugate of the present disclosure comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 30 (CDRH2), and SEQ ID NO: 22 (CDRH3) and a variable light chain comprising the CDR amino acid sequences of SEQ ID NO: 37 (CDRL1), SEQ ID NO: 38 (CDRL2), and SEQ ID NO: 33 (CDRL3), wherein said agonistic GLP-1 peptide comprises the amino acid sequence of SEQ ID No. 79, and wherein said agonistic GLP-1 peptide is comprised in a compound comprising the amino acid sequence of SEQ ID No. 64.

In certain embodiments, the immunoconjugate of the present disclosure comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 21 (CDRH2), and SEQ ID NO: 22 (CDRH3) and a variable light chain comprising the CDR amino acid sequences of SEQ ID NO: 43 (CDRL1), SEQ ID NO: 32 (CDRL2), and SEQ ID NO: 44 (CDRL3), wherein said agonistic GLP-1 peptide comprises the amino acid sequence of SEQ ID No. 79, and wherein said agonistic GLP-1 peptide is comprised in a compound comprising the amino acid sequence of SEQ ID No. 64.

In certain embodiments, the immunoconjugate of the present disclosure comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 21 (CDRH2), and SEQ ID NO: 22 (CDRH3) and a variable light chain comprising the CDR amino acid sequences of SEQ ID NO: 23 (CDRL1), SEQ ID NO: 24 (CDRL2), and SEQ ID NO: 25 (CDRL3), wherein said agonistic GLP-1 peptide comprises the amino acid sequence of SEQ ID No. 79, and wherein said agonistic GLP-1 peptide is comprised in a compound comprising the amino acid sequence of SEQ ID No. 69.

In certain embodiments, the immunoconjugate of the present disclosure comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 30 (CDRH2), and SEQ ID NO: 22 (CDRH3) and a variable light chain comprising the CDR amino acid sequences of SEQ ID NO: 31 (CDRL1), SEQ ID NO: 32 (CDRL2), and SEQ ID NO: 33 (CDRL3), wherein said agonistic GLP-1 peptide comprises the amino acid sequence of SEQ ID No. 79, and wherein said agonistic GLP-1 peptide is comprised in a compound comprising the amino acid sequence of SEQ ID No. 69.

In certain embodiments, the immunoconjugate of the present disclosure comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 30 (CDRH2), and SEQ ID NO: 22 (CDRH3) and a variable light chain comprising the CDR amino acid sequences of SEQ ID NO: 37 (CDRL1), SEQ ID NO: 38 (CDRL2), and SEQ ID NO: 33 (CDRL3), wherein said agonistic GLP-1 peptide comprises the amino acid sequence of SEQ ID No. 79, and wherein said agonistic GLP-1 peptide is comprised in a compound comprising the amino acid sequence of SEQ ID No. 69.

In certain embodiments, the immunoconjugate of the present disclosure comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 21 (CDRH2), and SEQ ID NO: 22 (CDRH3) and a variable light chain comprising the CDR amino acid sequences of SEQ ID NO: 43 (CDRL1), SEQ ID NO: 32 (CDRL2), and SEQ ID NO: 44 (CDRL3), wherein said agonistic GLP-1 peptide comprises the amino acid sequence of SEQ ID No. 79, and wherein said agonistic GLP-1 peptide is comprised in a compound comprising the amino acid sequence of SEQ ID No. 69.

In certain embodiments, the immunoconjugate of the present disclosure comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 21 (CDRH2), and SEQ ID NO: 22 (CDRH3) and a variable light chain comprising the CDR amino acid sequences of SEQ ID NO: 23 (CDRL1), SEQ ID NO: 24 (CDRL2), and SEQ ID NO: 25 (CDRL3), wherein said agonistic GLP-1 peptide comprises the amino acid sequence of SEQ ID No. 79, and wherein said agonistic GLP-1 peptide is comprised in a compound comprising the amino acid sequence of SEQ ID No. 64 or 69.

In certain embodiments, the immunoconjugate of the present disclosure comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 30 (CDRH2), and SEQ ID NO: 22 (CDRH3) and a variable light chain comprising the CDR amino acid sequences of SEQ ID NO: 31 (CDRL1), SEQ ID NO: 32 (CDRL2), and SEQ ID NO: 33 (CDRL3), wherein said agonistic GLP-1 peptide comprises the amino acid sequence of SEQ ID No. 79, and wherein said agonistic GLP-1 peptide is comprised in a compound comprising the amino acid sequence of SEQ ID No. 64 or 69.

In certain embodiments, the immunoconjugate of the present disclosure comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 30 (CDRH2), and SEQ ID NO: 22 (CDRH3) and a variable light chain comprising the CDR amino acid sequences of SEQ ID NO: 37 (CDRL1), SEQ ID NO: 38 (CDRL2), and SEQ ID NO: 33 (CDRL3), wherein said agonistic GLP-1 peptide comprises the amino acid sequence of SEQ ID No. 79, and wherein said agonistic GLP-1 peptide is comprised in a compound comprising the amino acid sequence of SEQ ID No. 64 or 69.

In certain embodiments, the immunoconjugate of the present disclosure comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 21 (CDRH2), and SEQ ID NO: 22 (CDRH3) and a variable light chain comprising the CDR amino acid sequences of SEQ ID NO: 43 (CDRL1), SEQ ID NO: 32 (CDRL2), and SEQ ID NO: 44 (CDRL3), wherein said agonistic GLP-1 peptide comprises the amino acid sequence of SEQ ID No. 79, and wherein said agonistic GLP-1 peptide is comprised in a compound comprising the amino acid sequence of SEQ ID No. 64 or 69.

In certain embodiments, the immunoconjugate of the present disclosure comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 21 (CDRH2), and SEQ ID NO: 22 (CDRH3) and a variable light chain comprising the CDR amino acid sequences of SEQ ID NO: 23 (CDRL1), SEQ ID NO: 24 (CDRL2), and SEQ ID NO: 25 (CDRL3), wherein said agonistic GLP-1 peptide comprises the amino acid sequence of SEQ ID No. 79, and wherein said agonistic GLP-1 peptide is comprised in a compound comprising the amino acid sequence of SEQ ID No. 64.

In certain embodiments, the immunoconjugate of the present disclosure comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 30 (CDRH2), and SEQ ID NO: 22 (CDRH3) and a variable light chain comprising the CDR amino acid sequences of SEQ ID NO: 31 (CDRL1), SEQ ID NO: 32 (CDRL2), and SEQ ID NO: 33 (CDRL3), wherein said agonistic GLP-1 peptide comprises the amino acid sequence of SEQ ID No. 79, and wherein said agonistic GLP-1 peptide is comprised in a compound comprising the amino acid sequence of SEQ ID No. 64.

In certain embodiments, the immunoconjugate of the present disclosure comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 30 (CDRH2), and SEQ ID NO: 22 (CDRH3) and a variable light chain comprising the CDR amino acid sequences of SEQ ID NO: 37 (CDRL1), SEQ ID NO: 38 (CDRL2), and SEQ ID NO: 33 (CDRL3), wherein said agonistic GLP-1 peptide comprises the amino acid sequence of SEQ ID No. 79, and wherein said agonistic GLP-1 peptide is comprised in a compound comprising the amino acid sequence of SEQ ID No. 64.

In certain embodiments, the immunoconjugate of the present disclosure comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 21 (CDRH2), and SEQ ID NO: 22 (CDRH3) and a variable light chain comprising the CDR amino acid sequences of SEQ ID NO: 43 (CDRL1), SEQ ID NO: 32 (CDRL2), and SEQ ID NO: 44 (CDRL3), wherein said agonistic GLP-1 peptide comprises the amino acid sequence of SEQ ID No. 79, and wherein said agonistic GLP-1 peptide is comprised in a compound comprising the amino acid sequence of SEQ ID No. 64.

In certain embodiments, the immunoconjugate of the present disclosure comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 21 (CDRH2), and SEQ ID NO: 22 (CDRH3) and a variable light chain comprising the CDR amino acid sequences of SEQ ID NO: 23 (CDRL1), SEQ ID NO: 24 (CDRL2), and SEQ ID NO: 25 (CDRL3), wherein said agonistic GLP-1 peptide comprises the amino acid sequence of SEQ ID No. 79, and wherein said agonistic GLP-1 peptide is comprised in a compound comprising the amino acid sequence of SEQ ID No. 69.

In certain embodiments, the immunoconjugate of the present disclosure comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 30 (CDRH2), and SEQ ID NO: 22 (CDRH3) and a variable light chain comprising the CDR amino acid sequences of SEQ ID NO: 31 (CDRL1), SEQ ID NO: 32 (CDRL2), and SEQ ID NO: 33 (CDRL3), wherein said agonistic GLP-1 peptide comprises the amino acid sequence of SEQ ID No. 79, and wherein said agonistic GLP-1 peptide is comprised in a compound comprising the amino acid sequence of SEQ ID No. 69.

In certain embodiments, the immunoconjugate of the present disclosure comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 30 (CDRH2), and SEQ ID NO: 22 (CDRH3) and a variable light chain comprising the CDR amino acid sequences of SEQ ID NO: 37 (CDRL1), SEQ ID NO: 38 (CDRL2), and SEQ ID NO: 33 (CDRL3), wherein said agonistic GLP-1 peptide comprises the amino acid sequence of SEQ ID No. 79, and wherein said agonistic GLP-1 peptide is comprised in a compound comprising the amino acid sequence of SEQ ID No. 69.

In certain embodiments, the immunoconjugate of the present disclosure comprises an ActRII receptor antibody and an agonistic GLP-1 peptide, wherein said ActRII receptor antibody comprises a variable heavy chain comprising the CDR amino acid sequences of SEQ ID NO: 20 (CDRH1), SEQ ID NO: 21 (CDRH2), and SEQ ID NO: 22 (CDRH3) and a variable light chain comprising the CDR amino acid sequences of SEQ ID NO: 43 (CDRL1), SEQ ID NO: 32 (CDRL2), and SEQ ID NO: 44 (CDRL3), wherein said agonistic GLP-1 peptide comprises the amino acid sequence of SEQ ID No. 79, and wherein said agonistic GLP-1 peptide is comprised in a compound comprising the amino acid sequence of SEQ ID No. 69.

In certain embodiments, the immunoconjugate of the present disclosure comprises an ActRII receptor antibody and an agonistic GLP-1 peptide, wherein said ActRII receptor antibody comprises a variable heavy chain comprising the amino acid sequences of SEQ ID NO: 18 and a variable light chain comprising the amino acid sequences of SEQ ID NO: 19, wherein said agonistic GLP-1 peptide comprises the amino acid sequence of SEQ ID No. 79, and wherein said agonistic GLP-1 peptide is comprised in a compound comprising the amino acid sequence of SEQ ID No. 64 or 69.

In certain embodiments, the immunoconjugate of the present disclosure comprises an ActRII receptor antibody and an agonistic GLP-1 peptide, wherein said ActRII receptor antibody comprises a variable heavy chain comprising the amino acid sequences of SEQ ID NO: 28 and a variable light chain comprising the amino acid sequences of SEQ ID NO: 29, wherein said agonistic GLP-1 peptide comprises the amino acid sequence of SEQ ID No. 79, and wherein said agonistic GLP-1 peptide is comprised in a compound comprising the amino acid sequence of SEQ ID No. 64 or 69.

In certain embodiments, the immunoconjugate of the present disclosure comprises an ActRII receptor antibody and an agonistic GLP-1 peptide, wherein said ActRII receptor antibody comprises a variable heavy chain comprising the amino acid sequences of SEQ ID NO: 28 and a variable light chain comprising the amino acid sequences of SEQ ID NO: 36, wherein said agonistic GLP-1 peptide comprises the amino acid sequence of SEQ ID No. 79, and wherein said agonistic GLP-1 peptide is comprised in a compound comprising the amino acid sequence of SEQ ID No. 64 or 69.

In certain embodiments, the immunoconjugate of the present disclosure comprises an ActRII receptor antibody and an agonistic GLP-1 peptide, wherein said ActRII receptor antibody comprises a variable heavy chain comprising the amino acid sequences of SEQ ID NO: 41 and a variable light chain comprising the amino acid sequences of SEQ ID NO: 42, wherein said agonistic GLP-1 peptide comprises the amino acid sequence of SEQ ID No. 79, and wherein said agonistic GLP-1 peptide is comprised in a compound comprising the amino acid sequence of SEQ ID No. 64 or 69.

In certain embodiments, the immunoconjugate of the present disclosure comprises an ActRII receptor antibody and an agonistic GLP-1 peptide, wherein said ActRII receptor antibody comprises a variable heavy chain comprising the amino acid sequences of SEQ ID NO: 18 and a variable light chain comprising the amino acid sequences of SEQ ID NO: 19, wherein said agonistic GLP-1 peptide comprises the amino acid sequence of SEQ ID No. 79, and wherein said agonistic GLP-1 peptide is comprised in a compound comprising the amino acid sequence of SEQ ID No. 64.

In certain embodiments, the immunoconjugate of the present disclosure comprises an ActRII receptor antibody and an agonistic GLP-1 peptide, wherein said ActRII receptor antibody comprises a variable heavy chain comprising the amino acid sequences of SEQ ID NO: 28 and a variable light chain comprising the amino acid sequences of SEQ ID NO: 29, wherein said agonistic GLP-1 peptide comprises the amino acid sequence of SEQ ID No. 79, and wherein said agonistic GLP-1 peptide is comprised in a compound comprising the amino acid sequence of SEQ ID No. 64.

In certain embodiments, the immunoconjugate of the present disclosure comprises an ActRII receptor antibody and an agonistic GLP-1 peptide, wherein said ActRII receptor antibody comprises a variable heavy chain comprising the amino acid sequences of SEQ ID NO: 28 and a variable light chain comprising the amino acid sequences of SEQ ID NO: 36, wherein said agonistic GLP-1 peptide comprises the amino acid sequence of SEQ ID No. 79, and wherein said agonistic GLP-1 peptide is comprised in a compound comprising the amino acid sequence of SEQ ID No. 64.

In certain embodiments, the immunoconjugate of the present disclosure comprises an ActRII receptor antibody and an agonistic GLP-1 peptide, wherein said ActRII receptor antibody comprises a variable heavy chain comprising the amino acid sequences of SEQ ID NO: 41 and a variable light chain comprising the amino acid sequences of SEQ ID NO: 42, wherein said agonistic GLP-1 peptide comprises the amino acid sequence of SEQ ID No. 79, and wherein said agonistic GLP-1 peptide is comprised in a compound comprising the amino acid sequence of SEQ ID No. 64.

In certain embodiments, the immunoconjugate of the present disclosure comprises an ActRII receptor antibody and an agonistic GLP-1 peptide, wherein said ActRII receptor antibody comprises a variable heavy chain comprising the amino acid sequences of SEQ ID NO: 18 and a variable light chain comprising the amino acid sequences of SEQ ID NO: 19, wherein said agonistic GLP-1 peptide comprises the amino acid sequence of SEQ ID No. 79, and wherein said agonistic GLP-1 peptide is comprised in a compound comprising the amino acid sequence of SEQ ID No. 69.

In certain embodiments, the immunoconjugate of the present disclosure comprises an ActRII receptor antibody and an agonistic GLP-1 peptide, wherein said ActRII receptor antibody comprises a variable heavy chain comprising the amino acid sequences of SEQ ID NO: 28 and a variable light chain comprising the amino acid sequences of SEQ ID NO: 29, wherein said agonistic GLP-1 peptide comprises the amino acid sequence of SEQ ID No. 79, and wherein said agonistic GLP-1 peptide is comprised in a compound comprising the amino acid sequence of SEQ ID No. 69.

In certain embodiments, the immunoconjugate of the present disclosure comprises an ActRII receptor antibody and an agonistic GLP-1 peptide, wherein said ActRII receptor antibody comprises a variable heavy chain comprising the amino acid sequences of SEQ ID NO: 28 and a variable light chain comprising the amino acid sequences of SEQ ID NO: 36, wherein said agonistic GLP-1 peptide comprises the amino acid sequence of SEQ ID No. 79, and wherein said agonistic GLP-1 peptide is comprised in a compound comprising the amino acid sequence of SEQ ID No. 69.

In certain embodiments, the immunoconjugate of the present disclosure comprises an ActRII receptor antibody and an agonistic GLP-1 peptide, wherein said ActRII receptor antibody comprises a variable heavy chain comprising the amino acid sequences of SEQ ID NO: 41 and a variable light chain comprising the amino acid sequences of SEQ ID NO: 42, wherein said agonistic GLP-1 peptide comprises the amino acid sequence of SEQ ID No. 79, and wherein said agonistic GLP-1 peptide is comprised in a compound comprising the amino acid sequence of SEQ ID No. 69.

In certain embodiments, the immunoconjugate of the present disclosure comprises an ActRII receptor antibody and an agonistic GLP-1 peptide, wherein said ActRII receptor antibody comprises a variable heavy chain comprising the amino acid sequences of SEQ ID NO: 18 and a variable light chain comprising the amino acid sequences of SEQ ID NO: 19, wherein said agonistic GLP-1 peptide comprises the amino acid sequence of SEQ ID No. 79, and wherein said agonistic GLP-1 peptide is comprised in a compound comprising the amino acid sequence of SEQ ID No. 64 or 69.

In certain embodiments, the immunoconjugate of the present disclosure comprises an ActRII receptor antibody and an agonistic GLP-1 peptide, wherein said ActRII receptor antibody comprises a variable heavy chain comprising the amino acid sequences of SEQ ID NO: 28 and a variable light chain comprising the amino acid sequences of SEQ ID NO: 29, wherein said agonistic GLP-1 peptide comprises the amino acid sequence of SEQ ID No. 79, and wherein said agonistic GLP-1 peptide is comprised in a compound comprising the amino acid sequence of SEQ ID No. 64 or 69.

In certain embodiments, the immunoconjugate of the present disclosure comprises an ActRII receptor antibody and an agonistic GLP-1 peptide, wherein said ActRII receptor antibody comprises a variable heavy chain comprising the amino acid sequences of SEQ ID NO: 28 and a variable light chain comprising the amino acid sequences of SEQ ID NO: 36, wherein said agonistic GLP-1 peptide comprises the amino acid sequence of SEQ ID No. 79, and wherein said agonistic GLP-1 peptide is comprised in a compound comprising the amino acid sequence of SEQ ID No. 64 or 69.

In certain embodiments, the immunoconjugate of the present disclosure comprises an ActRII receptor antibody and an agonistic GLP-1 peptide, wherein said ActRII receptor antibody comprises a variable heavy chain comprising the amino acid sequences of SEQ ID NO: 41 and a variable light chain comprising the amino acid sequences of SEQ ID NO: 42, wherein said agonistic GLP-1 peptide comprises the amino acid sequence of SEQ ID No. 79, and wherein said agonistic GLP-1 peptide is comprised in a compound comprising the amino acid sequence of SEQ ID No. 64 or 69.

In certain embodiments, the immunoconjugate of the present disclosure comprises an ActRII receptor antibody and an agonistic GLP-1 peptide, wherein said ActRII receptor antibody comprises a variable heavy chain comprising the amino acid sequences of SEQ ID NO: 18 and a variable light chain comprising the amino acid sequences of SEQ ID NO: 19, wherein said agonistic GLP-1 peptide comprises the amino acid sequence of SEQ ID No. 79, and wherein said agonistic GLP-1 peptide is comprised in a compound comprising the amino acid sequence of SEQ ID No. 64.

In certain embodiments, the immunoconjugate of the present disclosure comprises an ActRII receptor antibody and an agonistic GLP-1 peptide, wherein said ActRII receptor antibody comprises a variable heavy chain comprising the amino acid sequences of SEQ ID NO: 28 and a variable light chain comprising the amino acid sequences of SEQ ID NO: 29, wherein said agonistic GLP-1 peptide comprises the amino acid sequence of SEQ ID No. 79, and wherein said agonistic GLP-1 peptide is comprised in a compound comprising the amino acid sequence of SEQ ID No. 64.

In certain embodiments, the immunoconjugate of the present disclosure comprises an ActRII receptor antibody and an agonistic GLP-1 peptide, wherein said ActRII receptor antibody comprises a variable heavy chain comprising the amino acid sequences of SEQ ID NO: 28 and a variable light chain comprising the amino acid sequences of SEQ ID NO: 36, wherein said agonistic GLP-1 peptide comprises the amino acid sequence of SEQ ID No. 79, and wherein said agonistic GLP-1 peptide is comprised in a compound comprising the amino acid sequence of SEQ ID No. 64.

In certain embodiments, the immunoconjugate of the present disclosure comprises an ActRII receptor antibody and an agonistic GLP-1 peptide, wherein said ActRII receptor antibody comprises a variable heavy chain comprising the amino acid sequences of SEQ ID NO: 41 and a variable light chain comprising the amino acid sequences of SEQ ID NO: 42, wherein said agonistic GLP-1 peptide comprises the amino acid sequence of SEQ ID No. 79, and wherein said agonistic GLP-1 peptide is comprised in a compound comprising the amino acid sequence of SEQ ID No. 64.

In certain embodiments, the immunoconjugate of the present disclosure comprises an ActRII receptor antibody and an agonistic GLP-1 peptide, wherein said ActRII receptor antibody comprises a variable heavy chain comprising the amino acid sequences of SEQ ID NO: 18 and a variable light chain comprising the amino acid sequences of SEQ ID NO: 19, wherein said agonistic GLP-1 peptide comprises the amino acid sequence of SEQ ID No. 79, and wherein said agonistic GLP-1 peptide is comprised in a compound comprising the amino acid sequence of SEQ ID No. 69.

In certain embodiments, the immunoconjugate of the present disclosure comprises an ActRII receptor antibody and an agonistic GLP-1 peptide, wherein said ActRII receptor antibody comprises a variable heavy chain comprising the amino acid sequences of SEQ ID NO: 28 and a variable light chain comprising the amino acid sequences of SEQ ID NO: 29, wherein said agonistic GLP-1 peptide comprises the amino acid sequence of SEQ ID No. 79, and wherein said agonistic GLP-1 peptide is comprised in a compound comprising the amino acid sequence of SEQ ID No. 69.

In certain embodiments, the immunoconjugate of the present disclosure comprises an ActRII receptor antibody and an agonistic GLP-1 peptide, wherein said ActRII receptor antibody comprises a variable heavy chain comprising the amino acid sequences of SEQ ID NO: 28 and a variable light chain comprising the amino acid sequences of SEQ ID NO: 36, wherein said agonistic GLP-1 peptide comprises the amino acid sequence of SEQ ID No. 79, and wherein said agonistic GLP-1 peptide is comprised in a compound comprising the amino acid sequence of SEQ ID No. 69.

In certain embodiments, the immunoconjugate of the present disclosure comprises an ActRII receptor antibody and an agonistic GLP-1 peptide, wherein said ActRII receptor antibody comprises a variable heavy chain comprising the amino acid sequences of SEQ ID NO: 41 and a variable light chain comprising the amino acid sequences of SEQ ID NO: 42, wherein said agonistic GLP-1 peptide comprises the amino acid sequence of SEQ ID No. 79, and wherein said agonistic GLP-1 peptide is comprised in a compound comprising the amino acid sequence of SEQ ID No. 69.

### Conjugation

Various technologies exist, by which peptides or modified peptides can be conjugated to antibodies. This includes cysteine engineering of antibodies. In this technology peptides are conjugate to cysteines that are artificially introduced into the antibody, typically the Fc part.

The term "cysteine engineered" or "engineered cysteine" as used herein in the context of antibodies, refers to an antibody construct that is engineered to introduce at least one cysteine insertion mutation. A "cysteine mutation" in this context refers to a non-native cysteine residue that is introduced into the parental antibody construct sequence. The inserted cysteine residue(s) may be used as a site for conjugation of one or more active agents. In the present disclosure such active agent is an agonistic GLP-1 peptide.

Various positions in antibodies were reported as being amenable for cysteine engineering, including but not limited to positions 241, 243, 251, 253, 258, 264, 269, 271, 272, 274, 280, 281, 285, 288, 291, 293, 294, 296, 301, 307, 309, 311, 318, 329, 340, 341, 345, 357, 361, 384, 385, 386, 387, 389, 401, 402, 411, 417, 433, 434, 435, and 439 (from WO2015157595).

Peptides can be coupled to the cysteine engineered antibodies via Michael addition to, e.g., maleimide-modified peptides, or via halogen-substitution of accordingly modified peptides, e.g. Bromo acetylated peptides. Chemically modified peptides can for example be conjugated to cysteine engineered antibodies as described in ACS Chem Biol. 2017 Sep 15;12(9):2427-2435.

Other conjugation technologies are known as well that can be used to generate a conjugate comprising an ActRII receptor antibody and an agonistic GLP-1 peptide. Such additional conjugation technologies include conjugation via lysine-linked peptides or acetylene/azide modified peptides (via click chemistry).

In certain embodiments, the present disclosure relates to an immunoconjugate comprising an ActRII receptor antibody and an agonistic GLP-1 peptide, wherein said ActRII receptor antibody is conjugated to said agonistic GLP-1 peptide via cysteine engineering.

In certain embodiments, the present disclosure relates to an immunoconjugate comprising an ActRII receptor antibody and an agonistic GLP-1 peptide, wherein said ActRII receptor antibody is conjugated to said agonistic GLP-1 peptide via cysteine engineering, and wherein said conjugation technology employs bromo acetylated peptides.

In certain embodiments, the present disclosure relates to an immunoconjugate comprising an ActRII receptor antibody and an agonistic GLP-1 peptide, wherein said agonistic GLP-1 peptide is conjugated to said ActRII receptor antibody at position 272, 361, 384, 389 or 434 of the ActRII receptor antibody. Said agonistic GLP-1 peptide may also be conjugated to said ActRII receptor antibody via a cysteine residue located in the hinge region.

Furthermore, spacers of various length and rigidity may be introduced to further optimize the immunoconjugates of the present disclosure. Commonly used spacers include polyamides or polyethylene glycol (PEG).

In certain embodiments, the immunoconjugate of the present disclosure comprise an ActRII receptor antibody and an agonistic GLP-1 peptide, wherein said agonistic GLP-1 peptide is conjugated to said ActRII receptor antibody via an engineered cysteine. In certain embodiments, the immunoconjugate of the present disclosure comprise an ActRII receptor antibody and an agonistic GLP-1 peptide, wherein said agonistic GLP-1 peptide is conjugated to said ActRII receptor antibody via an engineered cysteine in the Fc region of said ActRII receptor antibody.

In certain embodiments, the immunoconjugate of the present disclosure comprise an ActRII receptor antibody and an agonistic GLP-1 peptide, wherein said agonistic GLP-1 peptide is conjugated to said ActRII receptor antibody via an engineered cysteine at position 272 of the constant region of the heavy chain of said ActRII receptor antibody. In certain embodiments, the immunoconjugate of the present disclosure comprise an ActRII receptor antibody and an agonistic GLP-1 peptide, wherein said agonistic GLP-1 peptide is conjugated to said ActRII receptor antibody via an engineered cysteine at position 272 of the constant region of the heavy chain of said ActRII receptor antibody (EU numbering).

In certain embodiments, the immunoconjugate of the present disclosure comprise an ActRII receptor antibody comprising a variable heavy chain comprising the CDR amino acid sequences of SEQ ID NO: 20 (CDRH1), SEQ ID NO: 21 (CDRH2), and SEQ ID NO: 22 (CDRH3), a variable light chain comprising the CDR amino acid sequences of SEQ ID NO: 23 (CDRL1), SEQ ID NO: 24 (CDRL2), and SEQ ID NO: 25 (CDRL3), and an agonistic GLP-1 peptide comprising the amino acid sequence of SEQ ID No. 79, wherein said agonistic GLP-1 peptide is conjugated to said ActRII receptor antibody via an engineered cysteine at position 272 of the constant region of the heavy chain of said ActRII receptor antibody (EU numbering).

In certain embodiments, the immunoconjugate of the present disclosure comprise an ActRII receptor antibody comprising a variable heavy chain comprising the CDR amino acid sequences of SEQ ID NO: 20 (CDRH1), SEQ ID NO: 30 (CDRH2), and SEQ ID NO: 22 (CDRH3), a variable light chain comprising the CDR amino acid sequences of SEQ ID NO: 31 (CDRL1), SEQ ID NO: 32 (CDRL2), and SEQ ID NO: 33 (CDRL3), and an agonistic GLP-1 peptide comprising the amino acid sequence of SEQ ID No. 79, wherein said agonistic GLP-1 peptide is conjugated to said ActRII receptor antibody via an engineered cysteine at position 272 of the constant region of the heavy chain of said ActRII receptor antibody (EU numbering).

In certain embodiments, the immunoconjugate of the present disclosure comprise an ActRII receptor antibody comprising a variable heavy chain comprising the CDR amino acid sequences of SEQ ID NO: 20 (CDRH1), SEQ ID NO: 30 (CDRH2), and SEQ ID NO: 22 (CDRH3), a variable light chain comprising the CDR amino acid sequences of SEQ ID NO: 37 (CDRL1), SEQ ID NO: 38 (CDRL2), and SEQ ID NO: 33 (CDRL3), and an agonistic GLP-1 peptide comprising the amino acid sequence of SEQ ID No. 79, wherein said agonistic GLP-1 peptide is conjugated to said ActRII receptor antibody via an engineered cysteine at position 272 of the constant region of the heavy chain of said ActRII receptor antibody (EU numbering).

In certain embodiments, the immunoconjugate of the present disclosure comprise an ActRII receptor antibody comprising a variable heavy chain comprising the CDR amino acid sequences of SEQ ID NO: 20 (CDRH1), SEQ ID NO: 21 (CDRH2), and SEQ ID NO: 22 (CDRH3), a variable light chain comprising the CDR amino acid sequences of SEQ ID NO: 43 (CDRL1), SEQ ID NO: 32 (CDRL2), and SEQ ID NO: 44 (CDRL3), and an agonistic GLP-1 peptide comprising the amino acid sequence of SEQ ID No. 79, wherein said agonistic GLP-1 peptide is conjugated to said ActRII receptor antibody via an engineered cysteine at position 272 of the constant region of the heavy chain of said ActRII receptor antibody (EU numbering).

In certain embodiments, the immunoconjugate of the present disclosure comprise an ActRII receptor antibody comprising a variable heavy chain comprising the CDR amino acid sequences of SEQ ID NO: 20 (CDRH1), SEQ ID NO: 21 (CDRH2), and SEQ ID NO: 22 (CDRH3), a variable light chain comprising the CDR amino acid sequences of SEQ ID NO: 23 (CDRL1), SEQ ID NO: 24 (CDRL2), and SEQ ID NO: 25 (CDRL3), and an agonistic GLP-1 peptide comprising the amino acid sequence of SEQ ID No. 79, wherein said agonistic GLP-1 peptide is comprised in a compound comprising the amino acid sequence of SEQ ID No. 64, wherein said agonistic GLP-1 peptide or said compound is conjugated to said ActRII receptor antibody via an engineered cysteine at position 272 of the constant region of the heavy chain of said ActRII receptor antibody (EU numbering).

In certain embodiments, the immunoconjugate of the present disclosure comprise an ActRII receptor antibody comprising a variable heavy chain comprising the CDR amino acid sequences of SEQ ID NO: 20 (CDRH1), SEQ ID NO: 30 (CDRH2), and SEQ ID NO: 22 (CDRH3), a variable light chain comprising the CDR amino acid sequences of SEQ ID NO: 31 (CDRL1), SEQ ID NO: 32 (CDRL2), and SEQ ID NO: 33 (CDRL3), and an agonistic GLP-1 peptide comprising the amino acid sequence of SEQ ID No. 79, wherein said agonistic GLP-1 peptide is comprised in a compound comprising the amino acid sequence of SEQ ID No. 64, wherein said agonistic GLP-1 peptide or said compound is conjugated to said ActRII receptor antibody via an engineered cysteine at position 272 of the constant region of the heavy chain of said ActRII receptor antibody (EU numbering).

In certain embodiments, the immunoconjugate of the present disclosure comprise an ActRII receptor antibody comprising a variable heavy chain comprising the CDR amino acid sequences of SEQ ID NO: 20 (CDRH1), SEQ ID NO: 30 (CDRH2), and SEQ ID NO: 22 (CDRH3), a variable light chain comprising the CDR amino acid sequences of SEQ ID NO: 37 (CDRL1), SEQ ID NO: 38 (CDRL2), and SEQ ID NO: 33 (CDRL3), and an agonistic GLP-1 peptide comprising the amino acid sequence of SEQ ID No. 79, wherein said agonistic GLP-1 peptide is comprised in a compound comprising the amino acid sequence of SEQ ID No. 64, wherein said agonistic GLP-1 peptide or said compound is conjugated to said ActRII receptor antibody via an engineered cysteine at position 272 of the constant region of the heavy chain of said ActRII receptor antibody (EU numbering).

In certain embodiments, the immunoconjugate of the present disclosure comprise an ActRII receptor antibody comprising a variable heavy chain comprising the CDR amino acid sequences of SEQ ID NO: 20 (CDRH1), SEQ ID NO: 21 (CDRH2), and SEQ ID NO: 22 (CDRH3), a variable light chain comprising the CDR amino acid sequences of SEQ ID NO: 43 (CDRL1), SEQ ID NO: 32 (CDRL2), and SEQ ID NO: 44 (CDRL3), and an agonistic GLP-1 peptide comprising the amino acid sequence of SEQ ID No. 79, wherein said agonistic GLP-1 peptide is comprised in a compound comprising the amino acid sequence of SEQ ID No. 64, wherein said agonistic GLP-1 peptide or said compound is conjugated to said ActRII receptor antibody via an engineered cysteine at position 272 of the constant region of the heavy chain of said ActRII receptor antibody (EU numbering).

In certain embodiments, the immunoconjugate of the present disclosure comprise an ActRII receptor antibody comprising a variable heavy chain comprising the CDR amino acid sequences of SEQ ID NO: 20 (CDRH1), SEQ ID NO: 21 (CDRH2), and SEQ ID NO: 22 (CDRH3), a variable light chain comprising the CDR amino acid sequences of SEQ ID NO: 23 (CDRL1), SEQ ID NO: 24 (CDRL2), and SEQ ID NO: 25 (CDRL3), and an agonistic GLP-1 peptide comprising the amino acid sequence of SEQ ID No. 79, wherein said agonistic GLP-1 peptide is comprised in a compound comprising the amino acid sequence of SEQ ID No. 69, wherein said agonistic GLP-1 peptide or said compound is conjugated to said ActRII receptor antibody via an engineered cysteine at position 272 of the constant region of the heavy chain of said ActRII receptor antibody (EU numbering).

In certain embodiments, the immunoconjugate of the present disclosure comprise an ActRII receptor antibody comprising a variable heavy chain comprising the CDR amino acid sequences of SEQ ID NO: 20 (CDRH1), SEQ ID NO: 30 (CDRH2), and SEQ ID NO: 22 (CDRH3), a variable light chain comprising the CDR amino acid sequences of SEQ ID NO: 31 (CDRL1), SEQ ID NO: 32 (CDRL2), and SEQ ID NO: 33 (CDRL3), and an agonistic GLP-1 peptide comprising the amino acid sequence of SEQ ID No. 79, wherein said agonistic GLP-1 peptide is comprised in a compound comprising the amino acid sequence of SEQ ID No. 69, wherein said agonistic GLP-1 peptide or said compound is conjugated to said ActRII receptor antibody via an engineered cysteine at position 272 of the constant region of the heavy chain of said ActRII receptor antibody (EU numbering).

In certain embodiments, the immunoconjugate of the present disclosure comprise an ActRII receptor antibody comprising a variable heavy chain comprising the CDR amino acid sequences of SEQ ID NO: 20 (CDRH1), SEQ ID NO: 30 (CDRH2), and SEQ ID NO: 22 (CDRH3), a variable light chain comprising the CDR amino acid sequences of SEQ ID NO: 37 (CDRL1), SEQ ID NO: 38 (CDRL2), and SEQ ID NO: 33 (CDRL3), and an agonistic GLP-1 peptide comprising the amino acid sequence of SEQ ID No. 79, wherein said agonistic GLP-1 peptide is comprised in a compound comprising the amino acid sequence of SEQ ID No. 69, wherein said agonistic GLP-1 peptide or said compound is conjugated to said ActRII receptor antibody via an engineered cysteine at position 272 of the constant region of the heavy chain of said ActRII receptor antibody (EU numbering).

In certain embodiments, the immunoconjugate of the present disclosure comprise an ActRII receptor antibody comprising a variable heavy chain comprising the CDR amino acid sequences of SEQ ID NO: 20 (CDRH1), SEQ ID NO: 21 (CDRH2), and SEQ ID NO: 22 (CDRH3), a variable light chain comprising the CDR amino acid sequences of SEQ ID NO: 43 (CDRL1), SEQ ID NO: 32 (CDRL2), and SEQ ID NO: 44 (CDRL3), and an agonistic GLP-1 peptide comprising the amino acid sequence of SEQ ID No. 79, wherein said agonistic GLP-1 peptide is comprised in a compound comprising the amino acid sequence of SEQ ID No. 69, wherein said agonistic GLP-1 peptide or said compound is conjugated to said ActRII receptor antibody via an engineered cysteine at position 272 of the constant region of the heavy chain of said ActRII receptor antibody (EU numbering).

In certain embodiments, the immunoconjugate of the present disclosure comprise an ActRII receptor antibody comprising a variable heavy chain comprising the amino acid sequences of SEQ ID NO: 18, a variable light chain comprising the amino acid sequences of SEQ ID NO: 19, and an agonistic GLP-1 peptide comprising the amino acid sequence of SEQ ID No. 79, wherein said agonistic GLP-1 peptide is conjugated to said ActRII receptor antibody via an engineered cysteine at position 272 of the constant region of the heavy chain of said ActRII receptor antibody (EU numbering).

In certain embodiments, the immunoconjugate of the present disclosure comprise an ActRII receptor antibody comprising a variable heavy chain comprising the amino acid sequences of SEQ ID NO: 28, a variable light chain comprising the amino acid sequences of SEQ ID NO: 29, and an agonistic GLP-1 peptide comprising the amino acid sequence of SEQ ID No. 79, wherein said agonistic GLP-1 peptide is conjugated to said ActRII receptor antibody via an engineered cysteine at position 272 of the constant region of the heavy chain of said ActRII receptor antibody (EU numbering).

In certain embodiments, the immunoconjugate of the present disclosure comprise an ActRII receptor antibody comprising a variable heavy chain comprising the amino acid sequences of SEQ ID NO: 28, a variable light chain comprising the amino acid sequences of SEQ ID NO: 36, and an agonistic GLP-1 peptide comprising the amino acid sequence of SEQ ID No. 79, wherein said agonistic GLP-1 peptide is conjugated to said ActRII receptor antibody via an engineered cysteine at position 272 of the constant region of the heavy chain of said ActRII receptor antibody (EU numbering).

In certain embodiments, the immunoconjugate of the present disclosure comprise an ActRII receptor antibody comprising a variable heavy chain comprising the amino acid sequences of SEQ ID NO: 41, a variable light chain comprising the amino acid sequences of SEQ ID NO: 42, and an agonistic GLP-1 peptide comprising the amino acid sequence of SEQ ID No. 79, wherein said agonistic GLP-1 peptide is conjugated to said ActRII receptor antibody via an engineered cysteine at position 272 of the constant region of the heavy chain of said ActRII receptor antibody (EU numbering).

In certain embodiments, the immunoconjugate of the present disclosure comprise an ActRII receptor antibody comprising a variable heavy chain comprising the amino acid sequences of SEQ ID NO: 18, a variable light chain comprising the amino acid sequences of SEQ ID NO: 19, and an agonistic GLP-1 peptide comprising the amino acid sequence of SEQ ID No. 79, wherein said agonistic GLP-1 peptide is comprised in a compound comprising the amino acid sequence of SEQ ID No. 64, wherein said agonistic GLP-1 peptide or said compound is conjugated to said ActRII receptor antibody via an engineered cysteine at position 272 of the constant region of the heavy chain of said ActRII receptor antibody (EU numbering).

In certain embodiments, the immunoconjugate of the present disclosure comprise an ActRII receptor antibody comprising a variable heavy chain comprising the amino acid sequences of SEQ ID NO: 28, a variable light chain comprising the amino acid sequences of SEQ ID NO: 29, and an agonistic GLP-1 peptide comprising the amino acid sequence of SEQ ID No. 79, wherein said agonistic GLP-1 peptide is comprised in a compound comprising the amino acid sequence of SEQ ID No. 64, wherein said agonistic GLP-1 peptide or said compound is conjugated to said ActRII receptor antibody via an engineered cysteine at position 272 of the constant region of the heavy chain of said ActRII receptor antibody (EU numbering).In certain embodiments, the immunoconjugate of the present disclosure comprise an ActRII receptor antibody comprising a variable heavy chain comprising the amino acid sequences of SEQ ID NO: 28, a variable light chain comprising the amino acid sequences of SEQ ID NO: 36, and an agonistic GLP-1 peptide comprising the amino acid sequence of SEQ ID No. 79, wherein said agonistic GLP-1 peptide is comprised in a compound comprising the amino acid sequence of SEQ ID No. 64, wherein said agonistic GLP-1 peptide or said compound is conjugated to said ActRII receptor antibody via an engineered cysteine at position 272 of the constant region of the heavy chain of said ActRII receptor antibody (EU numbering).

In certain embodiments, the immunoconjugate of the present disclosure comprise an ActRII receptor antibody comprising a variable heavy chain comprising the amino acid sequences of SEQ ID NO: 41, a variable light chain comprising the amino acid sequences of SEQ ID NO: 42, and an agonistic GLP-1 peptide comprising the amino acid sequence of SEQ ID No. 79, wherein said agonistic GLP-1 peptide is comprised in a compound comprising the amino acid sequence of SEQ ID No. 64, wherein said agonistic GLP-1 peptide or said compound is conjugated to said ActRII receptor antibody via an engineered cysteine at position 272 of the constant region of the heavy chain of said ActRII receptor antibody (EU numbering).

In certain embodiments, the immunoconjugate of the present disclosure comprise an ActRII receptor antibody comprising a variable heavy chain comprising the amino acid sequences of SEQ ID NO: 18, a variable light chain comprising the amino acid sequences of SEQ ID NO: 19, and an agonistic GLP-1 peptide comprising the amino acid sequence of SEQ ID No. 79, wherein said agonistic GLP-1 peptide is comprised in a compound comprising the amino acid sequence of SEQ ID No. 69, wherein said agonistic GLP-1 peptide or said compound is conjugated to said ActRII receptor antibody via an engineered cysteine at position 272 of the constant region of the heavy chain of said ActRII receptor antibody (EU numbering).

In certain embodiments, the immunoconjugate of the present disclosure comprise an ActRII receptor antibody comprising a variable heavy chain comprising the amino acid sequences of SEQ ID NO: 28, a variable light chain comprising the amino acid sequences of SEQ ID NO: 29, and an agonistic GLP-1 peptide comprising the amino acid sequence of SEQ ID No. 79, wherein said agonistic GLP-1 peptide is comprised in a compound comprising the amino acid sequence of SEQ ID No. 69, wherein said agonistic GLP-1 peptide or said compound is conjugated to said ActRII receptor antibody via an engineered cysteine at position 272 of the constant region of the heavy chain of said ActRII receptor antibody (EU numbering).

In certain embodiments, the immunoconjugate of the present disclosure comprise an ActRII receptor antibody comprising a variable heavy chain comprising the amino acid sequences of SEQ ID NO: 28, a variable light chain comprising the amino acid sequences of SEQ ID NO: 36, and an agonistic GLP-1 peptide comprising the amino acid sequence of SEQ ID No. 79, wherein said agonistic GLP-1 peptide is comprised in a compound comprising the amino acid sequence of SEQ ID No. 69, wherein said agonistic GLP-1 peptide or said compound is conjugated to said ActRII receptor antibody via an engineered cysteine at position 272 of the constant region of the heavy chain of said ActRII receptor antibody (EU numbering).

In certain embodiments, the immunoconjugate of the present disclosure comprise an ActRII receptor antibody comprising a variable heavy chain comprising the amino acid sequences of SEQ ID NO: 41, a variable light chain comprising the amino acid sequences of SEQ ID NO: 42, and an agonistic GLP-1 peptide comprising the amino acid sequence of SEQ ID No. 79, wherein said agonistic GLP-1 peptide is comprised in a compound comprising the amino acid sequence of SEQ ID No. 69, wherein said agonistic GLP-1 peptide or said compound is conjugated to said ActRII receptor antibody via an engineered cysteine at position 272 of the constant region of the heavy chain of said ActRII receptor antibody (EU numbering).

In certain embodiments, the immunoconjugate of the present disclosure comprise an ActRII receptor antibody comprising a variable heavy chain comprising the CDR amino acid sequences of SEQ ID NO: 20 (CDRH1), SEQ ID NO: 21 (CDRH2), and SEQ ID NO: 22 (CDRH3), a variable light chain comprising the CDR amino acid sequences of SEQ ID NO: 23 (CDRL1), SEQ ID NO: 24 (CDRL2), and SEQ ID NO: 25 (CDRL3), and an agonistic GLP-1 peptide comprising the amino acid sequence of SEQ ID No. 79, wherein said agonistic GLP-1 peptide is conjugated to said ActRII receptor antibody via an engineered cysteine at position 272 of the constant region of the heavy chain of said ActRII receptor antibody (EU numbering), and wherein said ActRII receptor antibody comprises the mutations L234A, L235A and P329A in the Fc region (EU numbering).

In certain embodiments, the immunoconjugate of the present disclosure comprise an ActRII receptor antibody comprising a variable heavy chain comprising the CDR amino acid sequences of SEQ ID NO: 20 (CDRH1), SEQ ID NO: 30 (CDRH2), and SEQ ID NO: 22 (CDRH3), a variable light chain comprising the CDR amino acid sequences of SEQ ID NO: 31 (CDRL1), SEQ ID NO: 32 (CDRL2), and SEQ ID NO: 33 (CDRL3), and an agonistic GLP-1 peptide comprising the amino acid sequence of SEQ ID No. 79, wherein said agonistic GLP-1 peptide is conjugated to said ActRII receptor antibody via an engineered cysteine at position 272 of the constant region of the heavy chain of said ActRII receptor antibody (EU numbering) , and wherein said ActRII receptor antibody comprises the mutations L234A, L235A and P329A in the Fc region (EU numbering).

In certain embodiments, the immunoconjugate of the present disclosure comprise an ActRII receptor antibody comprising a variable heavy chain comprising the CDR amino acid sequences of SEQ ID NO: 20 (CDRH1), SEQ ID NO: 30 (CDRH2), and SEQ ID NO: 22 (CDRH3), a variable light chain comprising the CDR amino acid sequences of SEQ ID NO: 37 (CDRL1), SEQ ID NO: 38 (CDRL2), and SEQ ID NO: 33 (CDRL3), and an agonistic GLP-1 peptide comprising the amino acid sequence of SEQ ID No. 79, wherein said agonistic GLP-1 peptide is conjugated to said ActRII receptor antibody via an engineered cysteine at position 272 of the constant region of the heavy chain of said ActRII receptor antibody (EU numbering) , and wherein said ActRII receptor antibody comprises the mutations L234A, L235A and P329A in the Fc region (EU numbering).

In certain embodiments, the immunoconjugate of the present disclosure comprise an ActRII receptor antibody comprising a variable heavy chain comprising the CDR amino acid sequences of SEQ ID NO: 20 (CDRH1), SEQ ID NO: 21 (CDRH2), and SEQ ID NO: 22 (CDRH3), a variable light chain comprising the CDR amino acid sequences of SEQ ID NO: 43 (CDRL1), SEQ ID NO: 32 (CDRL2), and SEQ ID NO: 44 (CDRL3), and an agonistic GLP-1 peptide comprising the amino acid sequence of SEQ ID No. 79, wherein said agonistic GLP-1 peptide is conjugated to said ActRII receptor antibody via an engineered cysteine at position 272 of the constant region of the heavy chain of said ActRII receptor antibody (EU numbering), and wherein said ActRII receptor antibody comprises the mutations L234A, L235A and P329A in the Fc region (EU numbering).

In certain embodiments, the immunoconjugate of the present disclosure comprise an ActRII receptor antibody comprising a variable heavy chain comprising the CDR amino acid sequences of SEQ ID NO: 20 (CDRH1), SEQ ID NO: 21 (CDRH2), and SEQ ID NO: 22 (CDRH3), a variable light chain comprising the CDR amino acid sequences of SEQ ID NO: 23 (CDRL1), SEQ ID NO: 24 (CDRL2), and SEQ ID NO: 25 (CDRL3), and an agonistic GLP-1 peptide comprising the amino acid sequence of SEQ ID No. 79, wherein said agonistic GLP-1 peptide is comprised in a compound comprising the amino acid sequence of SEQ ID No. 64, wherein said agonistic GLP-1 peptide or said compound is conjugated to said ActRII receptor antibody via an engineered cysteine at position 272 of the constant region of the heavy chain of said ActRII receptor antibody (EU numbering), and wherein said ActRII receptor antibody comprises the mutations L234A, L235A and P329A in the Fc region (EU numbering).

In certain embodiments, the immunoconjugate of the present disclosure comprise an ActRII receptor antibody comprising a variable heavy chain comprising the CDR amino acid sequences of SEQ ID NO: 20 (CDRH1), SEQ ID NO: 30 (CDRH2), and SEQ ID NO: 22 (CDRH3), a variable light chain comprising the CDR amino acid sequences of SEQ ID NO: 31 (CDRL1), SEQ ID NO: 32 (CDRL2), and SEQ ID NO: 33 (CDRL3), and an agonistic GLP-1 peptide comprising the amino acid sequence of SEQ ID No. 79, wherein said agonistic GLP-1 peptide is comprised in a compound comprising the amino acid sequence of SEQ ID No. 64, wherein said agonistic GLP-1 peptide or said compound is conjugated to said ActRII receptor antibody via an engineered cysteine at position 272 of the constant region of the heavy chain of said ActRll receptor antibody (EU numbering), and wherein said ActRll receptor antibody comprises the mutations L234A, L235A and P329A in the Fc region (EU numbering).

In certain embodiments, the immunoconjugate of the present disclosure comprise an ActRII receptor antibody comprising a variable heavy chain comprising the CDR amino acid sequences of SEQ ID NO: 20 (CDRH1), SEQ ID NO: 30 (CDRH2), and SEQ ID NO: 22 (CDRH3), a variable light chain comprising the CDR amino acid sequences of SEQ ID NO: 37 (CDRL1), SEQ ID NO: 38 (CDRL2), and SEQ ID NO: 33 (CDRL3), and an agonistic GLP-1 peptide comprising the amino acid sequence of SEQ ID No. 79, wherein said agonistic GLP-1 peptide is comprised in a compound comprising the amino acid sequence of SEQ ID No. 64, wherein said agonistic GLP-1 peptide or said compound is conjugated to said ActRII receptor antibody via an engineered cysteine at position 272 of the constant region of the heavy chain of said ActRII receptor antibody (EU numbering), and wherein said ActRII receptor antibody comprises the mutations L234A, L235A and P329A in the Fc region (EU numbering).

In certain embodiments, the immunoconjugate of the present disclosure comprise an ActRII receptor antibody comprising a variable heavy chain comprising the CDR amino acid sequences of SEQ ID NO: 20 (CDRH1), SEQ ID NO: 21 (CDRH2), and SEQ ID NO: 22 (CDRH3), a variable light chain comprising the CDR amino acid sequences of SEQ ID NO: 43 (CDRL1), SEQ ID NO: 32 (CDRL2), and SEQ ID NO: 44 (CDRL3), and an agonistic GLP-1 peptide comprising the amino acid sequence of SEQ ID No. 79, wherein said agonistic GLP-1 peptide is comprised in a compound comprising the amino acid sequence of SEQ ID No. 64, wherein said agonistic GLP-1 peptide or said compound is conjugated to said ActRII receptor antibody via an engineered cysteine at position 272 of the constant region of the heavy chain of said ActRII receptor antibody (EU numbering), and wherein said ActRII receptor antibody comprises the mutations L234A, L235A and P329A in the Fc region (EU numbering).

In certain embodiments, the immunoconjugate of the present disclosure comprise an ActRII receptor antibody comprising a variable heavy chain comprising the CDR amino acid sequences of SEQ ID NO: 20 (CDRH1), SEQ ID NO: 21 (CDRH2), and SEQ ID NO: 22 (CDRH3), a variable light chain comprising the CDR amino acid sequences of SEQ ID NO: 23 (CDRL1), SEQ ID NO: 24 (CDRL2), and SEQ ID NO: 25 (CDRL3), and an agonistic GLP-1 peptide comprising the amino acid sequence of SEQ ID No. 79, wherein said agonistic GLP-1 peptide is comprised in a compound comprising the amino acid sequence of SEQ ID No. 69, wherein said agonistic GLP-1 peptide or said compound is conjugated to said ActRII receptor antibody via an engineered cysteine at position 272 of the constant region of the heavy chain of said ActRII receptor antibody (EU numbering), and wherein said ActRII receptor antibody comprises the mutations L234A, L235A and P329A in the Fc region (EU numbering).

In certain embodiments, the immunoconjugate of the present disclosure comprise an ActRll receptor antibody comprising a variable heavy chain comprising the CDR amino acid sequences of SEQ ID NO: 20 (CDRH1), SEQ ID NO: 30 (CDRH2), and SEQ ID NO: 22 (CDRH3), a variable light chain comprising the CDR amino acid sequences of SEQ ID NO: 31 (CDRL1), SEQ ID NO: 32 (CDRL2), and SEQ ID NO: 33 (CDRL3), and an agonistic GLP-1 peptide comprising the amino acid sequence of SEQ ID No. 79, wherein said agonistic GLP-1 peptide is comprised in a compound comprising the amino acid sequence of SEQ ID No. 69, wherein said agonistic GLP-1 peptide or said compound is conjugated to said ActRll receptor antibody via an engineered cysteine at position 272 of the constant region of the heavy chain of said ActRll receptor antibody (EU numbering), and wherein said ActRll receptor antibody comprises the mutations L234A, L235A and P329A in the Fc region (EU numbering).

In certain embodiments, the immunoconjugate of the present disclosure comprise an ActRll receptor antibody comprising a variable heavy chain comprising the CDR amino acid sequences of SEQ ID NO: 20 (CDRH1), SEQ ID NO: 30 (CDRH2), and SEQ ID NO: 22 (CDRH3), a variable light chain comprising the CDR amino acid sequences of SEQ ID NO: 37 (CDRL1), SEQ ID NO: 38 (CDRL2), and SEQ ID NO: 33 (CDRL3), and an agonistic GLP-1 peptide comprising the amino acid sequence of SEQ ID No. 79, wherein said agonistic GLP-1 peptide is comprised in a compound comprising the amino acid sequence of SEQ ID No. 69, wherein said agonistic GLP-1 peptide or said compound is conjugated to said ActRll receptor antibody via an engineered cysteine at position 272 of the constant region of the heavy chain of said ActRll receptor antibody (EU numbering), and wherein said ActRll receptor antibody comprises the mutations L234A, L235A and P329A in the Fc region (EU numbering).

In certain embodiments, the immunoconjugate of the present disclosure comprise an ActRll receptor antibody comprising a variable heavy chain comprising the CDR amino acid sequences of SEQ ID NO: 20 (CDRH1), SEQ ID NO: 21 (CDRH2), and SEQ ID NO: 22 (CDRH3), a variable light chain comprising the CDR amino acid sequences of SEQ ID NO: 43 (CDRL1), SEQ ID NO: 32 (CDRL2), and SEQ ID NO: 44 (CDRL3), and an agonistic GLP-1 peptide comprising the amino acid sequence of SEQ ID No. 79, wherein said agonistic GLP-1 peptide is comprised in a compound comprising the amino acid sequence of SEQ ID No. 69, wherein said agonistic GLP-1 peptide or said compound is conjugated to said ActRll receptor antibody via an engineered cysteine at position 272 of the constant region of the heavy chain of said ActRll receptor antibody (EU numbering), and wherein said ActRll receptor antibody comprises the mutations L234A, L235A and P329A in the Fc region (EU numbering).

In certain embodiments, the immunoconjugate of the present disclosure comprise an ActRll receptor antibody comprising a variable heavy chain comprising the amino acid sequences of SEQ ID NO: 18, a variable light chain comprising the amino acid sequences of SEQ ID NO: 19, and an agonistic GLP-1 peptide comprising the amino acid sequence of SEQ ID No. 79, wherein said agonistic GLP-1 peptide is conjugated to said ActRll receptor antibody via an engineered cysteine at position 272 of the constant region of the heavy chain of said ActRll receptor antibody (EU numbering), and wherein said ActRll receptor antibody comprises the mutations L234A, L235A and P329A in the Fc region (EU numbering).

In certain embodiments, the immunoconjugate of the present disclosure comprise an ActRll receptor antibody comprising a variable heavy chain comprising the amino acid sequences of SEQ ID NO: 28, a variable light chain comprising the amino acid sequences of SEQ ID NO: 29, and an agonistic GLP-1 peptide comprising the amino acid sequence of SEQ ID No. 79, wherein said agonistic GLP-1 peptide is conjugated to said ActRll receptor antibody via an engineered cysteine at position 272 of the constant region of the heavy chain of said ActRll receptor antibody (EU numbering), and wherein said ActRll receptor antibody comprises the mutations L234A, L235A and P329A in the Fc region (EU numbering).

In certain embodiments, the immunoconjugate of the present disclosure comprise an ActRll receptor antibody comprising a variable heavy chain comprising the amino acid sequences of SEQ ID NO: 28, a variable light chain comprising the amino acid sequences of SEQ ID NO: 36, and an agonistic GLP-1 peptide comprising the amino acid sequence of SEQ ID No. 79, wherein said agonistic GLP-1 peptide is conjugated to said ActRll receptor antibody via an engineered cysteine at position 272 of the constant region of the heavy chain of said ActRll receptor antibody (EU numbering), and wherein said ActRll receptor antibody comprises the mutations L234A, L235A and P329A in the Fc region (EU numbering).

In certain embodiments, the immunoconjugate of the present disclosure comprise an ActRll receptor antibody comprising a variable heavy chain comprising the amino acid sequences of SEQ ID NO: 41, a variable light chain comprising the amino acid sequences of SEQ ID NO: 42, and an agonistic GLP-1 peptide comprising the amino acid sequence of SEQ ID No. 79, wherein said agonistic GLP-1 peptide is conjugated to said ActRll receptor antibody via an engineered cysteine at position 272 of the constant region of the heavy chain of said ActRll receptor antibody (EU numbering), and wherein said ActRll receptor antibody comprises the mutations L234A, L235A and P329A in the Fc region (EU numbering).

In certain embodiments, the immunoconjugate of the present disclosure comprise an ActRll receptor antibody comprising a variable heavy chain comprising the amino acid sequences of SEQ ID NO: 18, a variable light chain comprising the amino acid sequences of SEQ ID NO: 19, and an agonistic GLP-1 peptide comprising the amino acid sequence of SEQ ID No. 79, wherein said agonistic GLP-1 peptide is comprised in a compound comprising the amino acid sequence of SEQ ID No. 64, wherein said agonistic GLP-1 peptide or said compound is conjugated to said ActRll receptor antibody via an engineered cysteine at position 272 of the constant region of the heavy chain of said ActRll receptor antibody (EU numbering), and wherein said ActRll receptor antibody comprises the mutations L234A, L235A and P329A in the Fc region (EU numbering).

In certain embodiments, the immunoconjugate of the present disclosure comprise an ActRll receptor antibody comprising a variable heavy chain comprising the amino acid sequences of SEQ ID NO: 28, a variable light chain comprising the amino acid sequences of SEQ ID NO: 29, and an agonistic GLP-1 peptide comprising the amino acid sequence of SEQ ID No. 79, wherein said agonistic GLP-1 peptide is comprised in a compound comprising the amino acid sequence of SEQ ID No. 64, wherein said agonistic GLP-1 peptide or said compound is conjugated to said ActRll receptor antibody via an engineered cysteine at position 272 of the constant region of the heavy chain of said ActRll receptor antibody (EU numbering), and wherein said ActRll receptor antibody comprises the mutations L234A, L235A and P329A in the Fc region (EU numbering).

In certain embodiments, the immunoconjugate of the present disclosure comprise an ActRll receptor antibody comprising a variable heavy chain comprising the amino acid sequences of SEQ ID NO: 28, a variable light chain comprising the amino acid sequences of SEQ ID NO: 36, and an agonistic GLP-1 peptide comprising the amino acid sequence of SEQ ID No. 79, wherein said agonistic GLP-1 peptide is comprised in a compound comprising the amino acid sequence of SEQ ID No. 64, wherein said agonistic GLP-1 peptide or said compound is conjugated to said ActRll receptor antibody via an engineered cysteine at position 272 of the constant region of the heavy chain of said ActRll receptor antibody (EU numbering), and wherein said ActRll receptor antibody comprises the mutations L234A, L235A and P329A in the Fc region (EU numbering).

In certain embodiments, the immunoconjugate of the present disclosure comprise an ActRll receptor antibody comprising a variable heavy chain comprising the amino acid sequences of SEQ ID NO: 41, a variable light chain comprising the amino acid sequences of SEQ ID NO: 42, and an agonistic GLP-1 peptide comprising the amino acid sequence of SEQ ID No. 79, wherein said agonistic GLP-1 peptide is comprised in a compound comprising the amino acid sequence of SEQ ID No. 64, wherein said agonistic GLP-1 peptide or said compound is conjugated to said ActRll receptor antibody via an engineered cysteine at position 272 of the constant region of the heavy chain of said ActRll receptor antibody (EU numbering), and wherein said ActRll receptor antibody comprises the mutations L234A, L235A and P329A in the Fc region (EU numbering).

In certain embodiments, the immunoconjugate of the present disclosure comprise an ActRll receptor antibody comprising a variable heavy chain comprising the amino acid sequences of SEQ ID NO: 18, a variable light chain comprising the amino acid sequences of SEQ ID NO: 19, and an agonistic GLP-1 peptide comprising the amino acid sequence of SEQ ID No. 79, wherein said agonistic GLP-1 peptide is comprised in a compound comprising the amino acid sequence of SEQ ID No. 69, wherein said agonistic GLP-1 peptide or said compound is conjugated to said ActRll receptor antibody via an engineered cysteine at position 272 of the constant region of the heavy chain of said ActRll receptor antibody (EU numbering), and wherein said ActRll receptor antibody comprises the mutations L234A, L235A and P329A in the Fc region (EU numbering).

In certain embodiments, the immunoconjugate of the present disclosure comprise an ActRll receptor antibody comprising a variable heavy chain comprising the amino acid sequences of SEQ ID NO: 28, a variable light chain comprising the amino acid sequences of SEQ ID NO: 29, and an agonistic GLP-1 peptide comprising the amino acid sequence of SEQ ID No. 79, wherein said agonistic GLP-1 peptide is comprised in a compound comprising the amino acid sequence of SEQ ID No. 69, wherein said agonistic GLP-1 peptide or said compound is conjugated to said ActRll receptor antibody via an engineered cysteine at position 272 of the constant region of the heavy chain of said ActRll receptor antibody (EU numbering), and wherein said ActRll receptor antibody comprises the mutations L234A, L235A and P329A in the Fc region (EU numbering).

In certain embodiments, the immunoconjugate of the present disclosure comprise an ActRll receptor antibody comprising a variable heavy chain comprising the amino acid sequences of SEQ ID NO: 28, a variable light chain comprising the amino acid sequences of SEQ ID NO: 36, and an agonistic GLP-1 peptide comprising the amino acid sequence of SEQ ID No. 79, wherein said agonistic GLP-1 peptide is comprised in a compound comprising the amino acid sequence of SEQ ID No. 69, wherein said agonistic GLP-1 peptide or said compound is conjugated to said ActRll receptor antibody via an engineered cysteine at position 272 of the constant region of the heavy chain of said ActRll receptor antibody (EU numbering), and wherein said ActRll receptor antibody comprises the mutations L234A, L235A and P329A in the Fc region (EU numbering).

In certain embodiments, the immunoconjugate of the present disclosure comprise an ActRll receptor antibody comprising a variable heavy chain comprising the amino acid sequences of SEQ ID NO: 41, a variable light chain comprising the amino acid sequences of SEQ ID NO: 42, and an agonistic GLP-1 peptide comprising the amino acid sequence of SEQ ID No. 79, wherein said agonistic GLP-1 peptide is comprised in a compound comprising the amino acid sequence of SEQ ID No. 69, wherein said agonistic GLP-1 peptide or said compound is conjugated to said ActRll receptor antibody via an engineered cysteine at position 272 of the constant region of the heavy chain of said ActRll receptor antibody (EU numbering), and wherein said ActRll receptor antibody comprises the mutations L234A, L235A and P329A in the Fc region (EU numbering).

In certain embodiments, the immunoconjugate of the present disclosure comprise an ActRll receptor antibody comprising a heavy chain comprising the amino acid sequences of SEQ ID NO: 16 and a variable light chain comprising the amino acid sequences of SEQ ID NO: 17, and an agonistic GLP-1 peptide comprising the amino acid sequence of SEQ ID No. 79, wherein said agonistic GLP-1 peptide is conjugated to said ActRll receptor antibody via an engineered cysteine at position 272 of the constant region of the heavy chain of said ActRll receptor antibody (EU numbering).

In certain embodiments, the immunoconjugate of the present disclosure comprise an ActRll receptor antibody comprising a heavy chain comprising the amino acid sequences of SEQ ID NO: 26 and a variable light chain comprising the amino acid sequences of SEQ ID NO: 27, and an agonistic GLP-1 peptide comprising the amino acid sequence of SEQ ID No. 79, wherein said agonistic GLP-1 peptide is conjugated to said ActRll receptor antibody via an engineered cysteine at position 272 of the constant region of the heavy chain of said ActRll receptor antibody (EU numbering).

In certain embodiments, the immunoconjugate of the present disclosure comprise an ActRll receptor antibody comprising a heavy chain comprising the amino acid sequences of SEQ ID NO: 34 and a variable light chain comprising the amino acid sequences of SEQ ID NO: 35, and an agonistic GLP-1 peptide comprising the amino acid sequence of SEQ ID No. 79, wherein said agonistic GLP-1 peptide is conjugated to said ActRll receptor antibody via an engineered cysteine at position 272 of the constant region of the heavy chain of said ActRll receptor antibody (EU numbering).

In certain embodiments, the immunoconjugate of the present disclosure comprise an ActRll receptor antibody comprising a heavy chain comprising the amino acid sequences of SEQ ID NO: 39 and a variable light chain comprising the amino acid sequences of SEQ ID NO: 40, and an agonistic GLP-1 peptide comprising the amino acid sequence of SEQ ID No. 79, wherein said agonistic GLP-1 peptide is conjugated to said ActRll receptor antibody via an engineered cysteine at position 272 of the constant region of the heavy chain of said ActRll receptor antibody (EU numbering).

In certain embodiments, the immunoconjugate of the present disclosure comprise an ActRll receptor antibody comprising a heavy chain comprising the amino acid sequences of SEQ ID NO: 16 and a variable light chain comprising the amino acid sequences of SEQ ID NO: 17, and an agonistic GLP-1 peptide comprising the amino acid sequence of SEQ ID No. 79, wherein said agonistic GLP-1 peptide is comprised in a compound comprising the amino acid sequence of SEQ ID No. 64, wherein said agonistic GLP-1 peptide or said compound is conjugated to said ActRll receptor antibody via an engineered cysteine at position 272 of the constant region of the heavy chain of said ActRll receptor antibody (EU numbering).

In certain embodiments, the immunoconjugate of the present disclosure comprise an ActRll receptor antibody comprising a variable heavy chain comprising a heavy chain comprising the amino acid sequences of SEQ ID NO: 26 and a variable light chain comprising the amino acid sequences of SEQ ID NO: 27, and an agonistic GLP-1 peptide comprising the amino acid sequence of SEQ ID No. 79, wherein said agonistic GLP-1 peptide is comprised in a compound comprising the amino acid sequence of SEQ ID No. 64, wherein said agonistic GLP-1 peptide or said compound is conjugated to said ActRII receptor antibody via an engineered cysteine at position 272 of the constant region of the heavy chain of said ActRll receptor antibody (EU numbering).

In certain embodiments, the immunoconjugate of the present disclosure comprise an ActRll receptor antibody comprising a heavy chain comprising the amino acid sequences of SEQ ID NO: 34 and a variable light chain comprising the amino acid sequences of SEQ ID NO: 35, and an agonistic GLP-1 peptide comprising the amino acid sequence of SEQ ID No. 79, wherein said agonistic GLP-1 peptide is comprised in a compound comprising the amino acid sequence of SEQ ID No. 64, wherein said agonistic GLP-1 peptide or said compound is conjugated to said ActRll receptor antibody via an engineered cysteine at position 272 of the constant region of the heavy chain of said ActRll receptor antibody (EU numbering).

In certain embodiments, the immunoconjugate of the present disclosure comprise an ActRll receptor antibody comprising a variable heavy chain comprising a heavy chain comprising the amino acid sequences of SEQ ID NO: 39 and a variable light chain comprising the amino acid sequences of SEQ ID NO: 40, and an agonistic GLP-1 peptide comprising the amino acid sequence of SEQ ID No. 79, wherein said agonistic GLP-1 peptide is comprised in a compound comprising the amino acid sequence of SEQ ID No. 64, wherein said agonistic GLP-1 peptide or said compound is conjugated to said ActRII receptor antibody via an engineered cysteine at position 272 of the constant region of the heavy chain of said ActRll receptor antibody (EU numbering).

In certain embodiments, the immunoconjugate of the present disclosure comprise an ActRll receptor antibody comprising a variable heavy chain comprising a heavy chain comprising the amino acid sequences of SEQ ID NO: 16 and a variable light chain comprising the amino acid sequences of SEQ ID NO: 17, and an agonistic GLP-1 peptide comprising the amino acid sequence of SEQ ID No. 79, wherein said agonistic GLP-1 peptide is comprised in a compound comprising the amino acid sequence of SEQ ID No. 69, wherein said agonistic GLP-1 peptide or said compound is conjugated to said ActRII receptor antibody via an engineered cysteine at position 272 of the constant region of the heavy chain of said ActRll receptor antibody (EU numbering).

In certain embodiments, the immunoconjugate of the present disclosure comprise an ActRll receptor antibody comprising a variable heavy chain comprising a heavy chain comprising the amino acid sequences of SEQ ID NO: 26 and a variable light chain comprising the amino acid sequences of SEQ ID NO: 27, and an agonistic GLP-1 peptide comprising the amino acid sequence of SEQ ID No. 79, wherein said agonistic GLP-1 peptide is comprised in a compound comprising the amino acid sequence of SEQ ID No. 69, wherein said agonistic GLP-1 peptide or said compound is conjugated to said ActRII receptor antibody via an engineered cysteine at position 272 of the constant region of the heavy chain of said ActRll receptor antibody (EU numbering).

In certain embodiments, the immunoconjugate of the present disclosure comprise an ActRll receptor antibody comprising a variable heavy chain comprising a heavy chain comprising the amino acid sequences of SEQ ID NO: 34 and a variable light chain comprising the amino acid sequences of SEQ ID NO: 35, and an agonistic GLP-1 peptide comprising the amino acid sequence of SEQ ID No. 79, wherein said agonistic GLP-1 peptide is comprised in a compound comprising the amino acid sequence of SEQ ID No. 69, wherein said agonistic GLP-1 peptide or said compound is conjugated to said ActRII receptor antibody via an engineered cysteine at position 272 of the constant region of the heavy chain of said ActRll receptor antibody (EU numbering).

In certain embodiments, the immunoconjugate of the present disclosure comprise an ActRll receptor antibody comprising a heavy chain comprising the amino acid sequences of SEQ ID NO: 39 and a variable light chain comprising the amino acid sequences of SEQ ID NO: 40, and an agonistic GLP-1 peptide comprising the amino acid sequence of SEQ ID No. 79, wherein said agonistic GLP-1 peptide is comprised in a compound comprising the amino acid sequence of SEQ ID No. 69, wherein said agonistic GLP-1 peptide or said compound is conjugated to said ActRll receptor antibody via an engineered cysteine at position 272 of the constant region of the heavy chain of said ActRll receptor antibody (EU numbering).

### Treatment

The terms **"treatment", "treating"** and the like are used herein to generally mean obtaining a desired pharmacologic and/or physiologic effect with a therapeutic agent. "Treatment" as used herein covers any treatment of a disease in a mammal, and includes inhibiting or slowing the onset or development of the disease or relieving the disease, e.g., causing regression of the disease or symptoms associated with the disease. The therapeutic agent may be administered before, during or after the onset of disease. The treatment of ongoing disease, where the treatment stabilizes or reduces the undesirable clinical symptoms of the patient, may be of particular interest. In some embodiments, treatment is performed prior to complete loss of function in the affected tissues. In some embodiments, the subject therapy will be administered during the symptomatic stage of the disease, and in some embodiments, after the symptomatic stage of the disease.

The term **"individual", "subject"** and **"patient"** used interchangeably herein and refer to any subject for whom treatment or therapy is desired. The subject may be a mammalian subject. Mammalian subjects include, e. g., humans, non-human primates, rodents, (e.g., rats, mice), lagomorphs (e.g, rabbits), ungulates (e.g., cows, sheep, pigs, horses, goats, and the like), etc. In some embodiments, the subject is a human. In some embodiments, the subject is a non-human primate, for example a cynomolgus monkey. In some embodiments, the subject is a companion animal (e.g., cats, dogs).

The terms **"obesity related co-morbidity," "obesity related condition"** and **"obesity related disorder"** may be used interchangeably and refer to a health condition depending on the obesity of the subject. In some embodiments, the obesity related co-morbidity or condition increases the mortality risk of the subject. Obesity related co-morbidities include but are not limited to: high blood pressure (hypertension), high LDL cholesterol, low HDL cholesterol, high levels of triglycerides (dyslipidemia), Type 2 diabetes, coronary heart disease, stroke, gallbladder disease, osteoarthritis, sleep apnea, breathing problems, cancer, gastroesophageal reflux disease, severe COVID-19, overall mortality, lower quality of life, mental illness such as clinical depression, anxiety, and other mental disorders, and body pain and difficulty with physical functioning.

A **"metabolic disorder"** as used herein refers to a disorder affecting dysregulation of mammalian metabolism, including but not limited to: obesity, diabetes (Type I and II), metabolic syndrome, antipsychotic drug-associated obesity, glucocorticoid-induced obesity, hypothalamic obesity associated with craniopharyngioma, and monogenetic disorder associated obesity. The monogenetic disorders associated with obesity in humans, may include but are not limited to Bardet-Biedl syndrome, and a disorder arising from a mutation in one or more of the following genes ADCY3, ALMS1, ARL6, BBS1, BBS2, BBS4, BBS5, BBS7, BBS9, BBS10, BBS12, BDNF, CCDC28B, CEP290, CREBBP, EP300, GNAS, IER3IP1, MC3R, MKKS, MKS1, MRAP2, NTRK2, PCSK1, PHF6, POMC, SH2B1, SIM1, TMEM67, TRIM32, TTC8 and VPS13B, or combinations thereof. A metabolic disorder may also be associated with a complex genetic disorder, e.g., Prader-Willi syndrome.

As used herein, **"Body Mass Index"** or **"BMI"** is calculated as weight in kilograms (kg) divided by height in meters squared (m²), rounded to one decimal place. As used herein, "obesity" in adult humans is defined as a BMI greater than or equal to 30 kg/m². **"Obesity"** in human youth is defined as a BMI greater than or equal to the age- and sex-specific 95th percentile of the 2000 CDC growth charts. The term **"overweight** " is defined as a BMI of greater than or equal to 25 and less than 30.

**"Lean mass"** is defined as total body mass of a subject minus the fat mass and minus the bone mass of the subject. Lean mass and fat mass may be measured by, for example, bioelectrical impedance analysis (BIA), magnetic resonance imaging (MRI) or dual X-ray absorptiometry (DXA).

The term **"pharmaceutical composition"** as used herein refers to a pharmaceutically active compound, such as the immunoconjugates of the present disclosure, and a pharmaceutically acceptable excipient.

In certain embodiments, the present disclosure relates to a pharmaceutical composition comprising an immune conjugate of the present disclosure.

In certain embodiments, the present disclosure relates to an immunoconjugate or a pharmaceutical composition of the present disclosure for use in medicine. In certain embodiments, said use in medicine is the use in the treatment of a metabolic disorder. In certain embodiments, said metabolic disorder is selected from the group consisting of: obesity, diabetes, metabolic syndrome, anti-psychotic drug-associated obesity, glucocorticoid-induced obesity, hypothalamic obesity associated with craniopharyngioma, Prader-Willi syndrome, and a monogenetic disorder associated with obesity. In certain embodiments, said treatment is useful for an obesity related co-morbidity, wherein the condition is selected from the group of: glucose intolerance, prediabetes, insulin resistance, high triglycerides, overweight associated physical impairment, osteoporosis, renal disease, obstructive sleep apnea, sexual hormones impairment, endocrine reproductive disorders, osteoarthritis, gastrointestinal cancers, dyslipidemia, hypertension, heart failure, coronary heart disease, stroke, and/or gallstones.

### Examples

### Example 1: Generation of antibodies

Several ActRll receptor antibodies were produced. These antibodies were conjugated to various agonistic GLP-1 peptides and tested in various assays. Depending on the experiments, the variable heavy chains and variable light chains were fused to either human or mouse constant regions.

Antibodies of the present disclosure were generated by Icosagen (Tartumaa, Estonia) and WuXi Biologics (Shanghai, China) using standard technologies. Binders were purified via HiTrap MabSelect PrismA and preparative SEC-HPLC. QC was performed using capillary electrophoresis on LabChip GXII, measurement of the endotoxin content and analytical SEC-HPLC. Endotoxin levels were below 0.02 EU/mg. Monomer content was >95 after one freeze-thaw cycle

The antibodies generated include the following (all CDRs according to Kabat):

| **Antibody *Caveman*** | | |
|---|---|---|
| **Description** | **Amino acid sequence** | **SEQ ID No.** |
| Heavy chain | QVQLVQSGAEVKKPGASVKVSCKASGYTFTSSYINWVRQAPGQGLEWMGTIN PVSGSTSYAQKFQGRVTMTRDTSISTAYMELSRLRSDDTAVYYCARGGWFDYWG QGTLVTVSSASTKGPSVFPLAPSSKSTSGGTAALGCLVKDYFPEPVTVSWNSGALT SGVHTFPAVLQSSGLYSLSSVVTVPSSSLGTQTYICNVNHKPSNTKVDKKVEPKSC DKTHTCPPCPAPEAAGGPSVFLFPPKPKDTLYITREPEVTCVVVDVSHEDPCVKFN WYVDGVEVHNAKTKPREEQYNSTYRVVSVLTVLHQDWLNGKEYKCKVSNKALA APIEKTISKAKGQPREPQVYTLPPSREEMTKNQVSLTCLVKGFYPSDIAVEWESNG QPENNYKTTPPVLDSDGSFFLYSKLTVDKSRWQQGNVFSCSVMHEALHNHYTQ KSLSLSPGK | 16 |
| Light chain | QSALTQPASVSGSPGQSITISCTGTSSDVGSYNYVNWYQQHPGKAPKLMIYGVS KRPSGVSNRFSGSKSGNTASLTISGLQAEDEADYYCGTFAGGSYYGVFGGGTKLTV LGQPKAAPSVTLFPPSSEELQANKATLVCLISDFYPGAVTVAWKADSSPVKAGVE TTTPSKQSNNKYAASSYLSLTPEQWKSHRSYSCQVTHEGSTVEKTVAPTECS | 17 |
| VH | | 18 |
| VL | | 19 |
| HCDR1 | SSYIN | 20 |
| HCDR2 | TINPVSGSTSYAQKFQG | 21 |
| HCDR3 | GGWFDY | 22 |
| LCDR1 | TGTSSDVGSYNYVN | 23 |
| LCDR2 | GVSKRPS | 24 |
| LCDR3 | GTFAGGSYYGV | 25 |

| **Antibody *Bamm-Bamm*** | | |
|---|---|---|
| **Description** | **Amino acid sequence** | **SEQ ID No.** |
| Heavy chain | QVQLVQSGAEVKKPGSSVKVSCKASGYTFTSSYINWVRQAPGQGLEWMGTINA VSGSTSYAQKFQGRVTITADTSTSTAYMELSSLRSEDTAVYYCARGGWFDYWGQ GTLVTVSSASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKKVEPKSCD KTHTCPPCPAPEAAGGPSVFLFPPKPKDTLMISRTPEVTCVVVDVSHEDPCVKFN WYVDGVEVHNAKTKPREEQYNSTYRVVSVLTVLHQDWLNGKEYKCKVSNKALA APIEKTISKAKGQPREPQVYTLPPSREEMTKNQVSLTCLVKGFYPSDIAVEWESNG QPENNYKTTPPVLDSDGSFFLYSKLTVDKSRWQQGNVFSCSVMHEALHNHYTQ KSLSLSPGK | 26 |
| Light chain | QSALTQPRSVSGSPGQSVTISCTGTSSDVGSKNYVNWYQQHPGKAPKLMIYGVS KRESGVPDRFSGSKSGNTASLTISGLQAEDEADYYCGTFAGGKYYGVFGGGTKLT VLGQPKAAPSVTLFPPSSEELQANKATLVCLISDFYPGAVTVAWKADSSPVKAGV ETTTPSKQSNNKYAASSYLSLTPEQWKSHRSYSCQVTHEGSTVEKTVAPTECS | 27 |
| VH | QVQLVQSGAEVKKPGSSVKVSCKASGYTFTSSYINWVRQAPGQGLEWMGTINA VSGSTSYAQKFQGRVTITADTSTSTAYMELSSLRSEDTAVYYCARGGWFDYWGQ GTLVTVSS | 28 |
| VL | QSALTQPRSVSGSPGQSVTISCTGTSSDVGSKNYVNWYQQHPGKAPKLMIYGVS KRESGVPDRFSGSKSGNTASLTISGLQAEDEADYYCGTFAGGKYYGVFGGGTKLT VL | 29 |
| HCDR1 | SSYIN | 20 |
| HCDR2 | TINAVSGSTSYAQKFQG | 30 |
| HCDR3 | GGWFDY | 22 |
| LCDR1 | TGTSSDVGSKNYVN | 31 |
| LCDR2 | GVSKRES | 32 |
| LCDR3 | GTFAGGKYYGV | 33 |

| **Antibody *Fred*** | | |
|---|---|---|
| **Description** | **Amino acid sequence** | **SEQ ID No.** |
| Heavy chain | QVQLVQSGAEVKKPGSSVKVSCKASGYTFTSSYINWVRQAPGQGLEWMGTINA VSGSTSYAQKFQGRVTITADTSTSTAYMELSSLRSEDTAVYYCARGGWFDYWGQ GTLVTVSSAKTTAPSVYPLAPVCGDTTGSSVTLGCLVKGYFPEPVTLTWNSGSLSS GVHTFPAVLQSDLYTLSSSVTVTSSTWPSQSITCNVAHPASSTKVDKKIEPRGPTIK PCPPCKCPAPNAAGGPSVFIFPPKIKDVLMISLSPIVTCVVVDVSEDDPCVQISWF VNNVEVHTAQTQTHREDYNSTLRVVSALPIQHQDWMSGKEFKCKVNNKDLAA PIERTISKPKGSVRAPQVYVLPPPEEEMTKKQVTLTCMVTDFMPEDIYVEWTNN GKTELNYKNTEPVLDSDGSYFMYSKLRVEKKNWVERNSYSCSVVHEGLHNHHT TKSFSRTPGK | 34 |
| Light chain | QSALTQPRSVSGSPGQSVTISCTGTSSDVGSANYVNWYQQHPGKAPKPMIYGV SRRPSGVPDRFSGSKSGNTASLTISGLQAEDEADYYCGTFAGGKYYGVFGGGTKL TVLGQPKAAPSVTLFPPSSEELQANKATLVCLISDFYPGAVTVAWKADSSPVKAG VETTTPSKQSNNKYAASSYLSLTPEQWKSHRSYSCQVTHEGSTVEKTVAPTECS | 35 |
| VH | QVQLVQSGAEVKKPGSSVKVSCKASGYTFTSSYINWVRQAPGQGLEWMGTINA VSGSTSYAQKFQGRVTITADTSTSTAYMELSSLRSEDTAVYYCARGGWFDYWGQ GTLVTVSS | 28 |
| VL | QSALTQPRSVSGSPGQSVTISCTGTSSDVGSANYVNWYQQHPGKAPKPMIYGV SRRPSGVPDRFSGSKSGNTASLTISGLQAEDEADYYCGTFAGGKYYGVFGGGTKL TVL | 36 |
| HCDR1 | SSYIN | 20 |
| HCDR2 | TINAVSGSTSYAQKFQG | 30 |
| HCDR3 | GGWFDY | 22 |
| LCDR1 | TGTSSDVGSANYVN | 37 |
| LCDR2 | GVSRRPS | 38 |
| LCDR3 | GTFAGGKYYGV | 33 |

| **Antibody *Barney*** | | |
|---|---|---|
| **Description** | **Amino acid sequence** | **SEQ ID No.** |
| Heavy chain | QVQLVQSGAEVRKPGSSVKVSCKASGYTFTSSYINWVRQAPGQGLEWMGTINP VSGSTSYAQKFQGRVTITADTSTSTAYMELSSLRSEDTAVYYCARGGWFDYWGQ GTLVTVSSAKTTAPSVYPLAPVCGDTTGSSVTLGCLVKGYFPEPVTLTWNSGSLSS GVHTFPAVLQSDLYTLSSSVTVTSSTWPSQSITCNVAHPASSTKVDKKIEPRGPTIK PCPPCKCPAPNAAGGPSVFIFPPKIKDVLMISLSPIVTCVVVDVSEDDPCVQISWF VNNVEVHTAQTQTHREDYNSTLRVVSALPIQHQDWMSGKEFKCKVNNKDLAA PIERTISKPKGSVRAPQVYVLPPPEEEMTKKQVTLTCMVTDFMPEDIYVEWTNN GKTELNYKNTEPVLDSDGSYFMYSKLRVEKKNWVERNSYSCSVVHEGLHNHHT TKSFSRTPGK | 39 |
| Light chain | QSALTQPRSVSGSPGQSVTISCTGTSSDVGSYNRVNWYQQHPGKAPKLMIYGVS KRESGVPDRFSGSKSGNTASLTISGLQAEDEADYYCGTFAGGRYYGVFGGGTKLT VLGQPKAAPSVTLFPPSSEELQANKATLVCLISDFYPGAVTVAWKADSSPVKAGV ETTTPSKQSNNKYAASSYLSLTPEQWKSHRSYSCQVTHEGSTVEKTVAPTECS | 40 |
| VH | QVQLVQSGAEVRKPGSSVKVSCKASGYTFTSSYINWVRQAPGQGLEWMGTINP VSGSTSYAQKFQGRVTITADTSTSTAYMELSSLRSEDTAVYYCARGGWFDYWGQ GTLVTVSS | 41 |
| VL | QSALTQPRSVSGSPGQSVTISCTGTSSDVGSYNRVNWYQQHPGKAPKLMIYGVS KRESGVPDRFSGSKSGNTASLTISGLQAEDEADYYCGTFAGGRYYGVFGGGTKLT VL | 42 |
| HCDR1 | SSYIN | 20 |
| HCDR2 | TINPVSGSTSYAQKFQG | 21 |
| HCDR3 | GGWFDY | 22 |
| LCDR1 | TGTSSDVGSYNRVN | 43 |
| LCDR2 | GVSKRES | 32 |
| LCDR3 | GTFAGGRYYGV | 44 |

| **Antibody *Wilma*** | | |
|---|---|---|
| **Description** | **Amino acid sequence** | **SEQ ID No.** |
| Heavy chain | QVQLVQSGAEVKKPGSSVKVSCKASGYTFTSSYINWVRQAPGQGLEWMGTINA VSGSTSYAQKFQGRVTITADTSTSTAYMELSSLRSEDTAVYYCARGGWFDYWGQ GTLVTVSSAKTTAPSVYPLAPVCGDTTGSSVTLGCLVKGYFPEPVTLTWNSGSLSS GVHTFPAVLQSDLYTLSSSVTVTSSTWPSQSITCNVAHPASSTKVDKKIEPRGPTIK | 60 |
| | PCPPCKCPAPNAAGGPSVFIFPPKIKDVLMISLSPIVTCVVVDVSEDDPCVQISWF VNNVEVHTAQTQTHREDYNSTLRVVSALPIQHQDWMSGKEFKCKVNNKDLAA PIERTISKPKGSVRAPQVYVLPPPEEEMTKKQVTLTCMVTDFMPEDIYVEWTNN GKTELNYKNTEPVLDSDGSYFMYSKLRVEKKNWVERNSYSCSVVHEGLHNHHT TKSFSRTPGK | |
| Light chain | QSALTQPRSVSGSPGQSVTISCTGTSSDVGSKNYVNWYQQHPGKAPKLMIYGVS KRESGVPDRFSGSKSGNTASLTISGLQAEDEADYYCGTFAGGKYYGVFGGGTKLT VLGQPKSSPSVTLFPPSSEELETNKATLVCTITDFYPGVVTVDWKVDGTPVTQGM ETTQPSKQSNNKYMASSYLTLTARAWERHSSYSCQVTHEGHTVEKSLSRADCS | 45 |
| VH | QVQLVQSGAEVKKPGSSVKVSCKASGYTFTSSYINWVRQAPGQGLEWMGTINA VSGSTSYAQKFQGRVTITADTSTSTAYMELSSLRSEDTAVYYCARGGWFDYWGQ GTLVTVSS | 28 |
| VL | QSALTQPRSVSGSPGQSVTISCTGTSSDVGSKNYVNWYQQHPGKAPKLMIYGVS KRESGVPDRFSGSKSGNTASLTISGLQAEDEADYYCGTFAGGKYYGVFGGGTKLT VL | 29 |
| HCDR1 | SSYIN | 20 |
| HCDR2 | TINAVSGSTSYAQKFQG | 30 |
| HCDR3 | GGWFDY | 22 |
| LCDR1 | TGTSSDVGSKNYVN | 31 |
| LCDR2 | GVSKRES | 32 |
| LCDR3 | GTFAGGKYYGV | 33 |

| **Antibody *Betty*** | | |
|---|---|---|
| **Description** | **Amino acid sequence** | **SEQ ID No.** |
| Heavy chain | QVQLVQSGAEVKKPGSSVKVSCKASGYTFTSSYINWVRQAPGQGLEWMGTINA VSGSTSYAQKFQGRVTITADTSTSTAYMELSSLRSEDTAVYYCARGGWFDYWGQ GTLVTVSSAKTTAPSVYPLAPVCGDTTGSSVTLGCLVKGYFPEPVTLTWNSGSLSS GVHTFPAVLQSDLYTLSSSVTVTSSTWPSQSITCNVAHPASSTKVDKKIEPRGPTIK PCPPCKCPAPNAAGGPSVFIFPPKIKDVLMISLSPIVTCVVVDVSEDDPCVQISWF VNNVEVHTAQTQTHREDYNSTLRVVSALPIQHQDWMSGKEFKCKVNNKDLAA PIERTISKPKGSVRAPQVYVLPPPEEEMTKKQVTLTCMVTDFMPEDIYVEWTNN GKTELNYKNTEPVLDSDGSYFMYSKLRVEKKNWVERNSYSCSVVHEGLHNHHT TKSFSRTPGK | 34 |
| Light chain | QSALTQPRSVSGSPGQSVTISCTGTSSDVGSANYVNWYQQHPGKAPKPMIYGV SRRPSGVPDRFSGSKSGNTASLTISGLQAEDEADYYCGTFAGGKYYGVFGGGTKL TVLGQPKSSPSVTLFPPSSEELETNKATLVCTITDFYPGVVTVDWKVDGTPVTQG METTQPSKQSNNKYMASSYLTLTARAWERHSSYSCQVTHEGHTVEKSLSRADCS | 46 |
| VH | QVQLVQSGAEVKKPGSSVKVSCKASGYTFTSSYINWVRQAPGQGLEWMGTINA VSGSTSYAQKFQGRVTITADTSTSTAYMELSSLRSEDTAVYYCARGGWFDYWGQ GTLVTVSS | 28 |
| VL | QSALTQPRSVSGSPGQSVTISCTGTSSDVGSANYVNWYQQHPGKAPKPMIYGV SRRPSGVPDRFSGSKSGNTASLTISGLQAEDEADYYCGTFAGGKYYGVFGGGTKL TVL | 36 |
| HCDR1 | SSYIN | 20 |
| HCDR2 | TINAVSGSTSYAQKFQG | 30 |
| HCDR3 | GGWFDY | 22 |
| LCDR1 | TGTSSDVGSANYVN | 37 |
| LCDR2 | GVSRRPS | 38 |
| LCDR3 | GTFAGGKYYGV | 33 |

| **Antibody *Dino*** | | |
|---|---|---|
| **Description** | **Amino acid sequence** | **SEQ ID No.** |
| Heavy chain | QVQLVQSGAEVRKPGSSVKVSCKASGYTFTSSYINWVRQAPGQGLEWMGTINP VSGSTSYAQKFQGRVTITADTSTSTAYMELSSLRSEDTAVYYCARGGWFDYWGQ GTLVTVSSAKTTAPSVYPLAPVCGDTTGSSVTLGCLVKGYFPEPVTLTWNSGSLSS GVHTFPAVLQSDLYTLSSSVTVTSSTWPSQSITCNVAHPASSTKVDKKIEPRGPTIK PCPPCKCPAPNAAGGPSVFIFPPKIKDVLMISLSPIVTCVVVDVSEDDPCVQISWF VNNVEVHTAQTQTHREDYNSTLRVVSALPIQHQDWMSGKEFKCKVNNKDLAA PIERTISKPKGSVRAPQVYVLPPPEEEMTKKQVTLTCMVTDFMPEDIYVEWTNN GKTELNYKNTEPVLDSDGSYFMYSKLRVEKKNWVERNSYSCSVVHEGLHNHHT TKSFSRTPGK | 39 |
| Light chain | QSALTQPRSVSGSPGQSVTISCTGTSSDVGSYNRVNWYQQHPGKAPKLMIYGVS KRESGVPDRFSGSKSGNTASLTISGLQAEDEADYYCGTFAGGRYYGVFGGGTKLT VLGQPKSSPSVTLFPPSSEELETNKATLVCTITDFYPGVVTVDWKVDGTPVTQGM ETTQPSKQSNNKYMASSYLTLTARAWERHSSYSCQVTHEGHTVEKSLSRADCS | 47 |
| VH | QVQLVQSGAEVRKPGSSVKVSCKASGYTFTSSYINWVRQAPGQGLEWMGTINP VSGSTSYAQKFQGRVTITADTSTSTAYMELSSLRSEDTAVYYCARGGWFDYWGQ GTLVTVSS | 41 |
| VL | QSALTQPRSVSGSPGQSVTISCTGTSSDVGSYNRVNWYQQHPGKAPKLMIYGVS KRESGVPDRFSGSKSGNTASLTISGLQAEDEADYYCGTFAGGRYYGVFGGGTKLT VL | 42 |
| HCDR1 | SSYIN | 20 |
| HCDR2 | TINPVSGSTSYAQKFQG | 21 |
| HCDR3 | GGWFDY | 22 |
| LCDR1 | TGTSSDVGSYNRVN | 43 |
| LCDR2 | GVSKRES | 32 |
| LCDR3 | GTFAGG RYYGV | 44 |

| **Antibody *State*** - control antibody with specificity for hen egg lysozyme | | |
|---|---|---|
| **Description** | **Amino acid sequence** | **SEQ ID No.** |
| Heavy chain | QVQLQQSGPGLVKPSQTLSLTCAISGDSVSSNSAAWSWIRQSPGRGLEWLGRIY YRSKWYNDYAVSVKSRITINPDTSKNQFSLQLNSVTPEDTAVYYCARLDHRYHED TVYPGMDVWGQGTLVTVSSAKTTAPSVYPLAPVCGDTTGSSVTLGCLVKGYFPE PVTLTWNSGSLSSGVHTFPAVLQSDLYTLSSSVTVTSSTWPSQSITCNVAHPASST KVDKKIEPRGPTIKPCPPCKCPAPNAAGGPSVFIFPPKIKDVLMISLSPIVTCVVVD VSEDDPCVQISWFVNNVEVHTAQTQTHREDYNSTLRVVSALPIQHQDWMSGK EFKCKVNNKDLAAPIERTISKPKGSVRAPQVYVLPPPEEEMTKKQVTLTCMVTDF MPEDIYVEWTNNGKTELNYKNTEPVLDSDGSYFMYSKLRVEKKNWVERNSYSC SVVHEGLHNHHTTKSFSRTPGK | 48 |
| Light chain | DIELTQPPSVSVAPGQTARISCSGDNLPAYTVTWYQQKPGQAPVLVIYDDSDRPS GIPERFSGSNSGNTATLTISGTQAEDEADYYCASWDPSSGVVFGGGTKLTVLGQP KSSPSVTLFPPSSEELETNKATLVCTITDFYPGVVTVDWKVDGTPVTQGMETTQP SKQSNNKYMASSYLTLTARAWERHSSYSCQVTHEGHTVEKSLSRADCS | 49 |
| VH | QVQLQQSGPGLVKPSQTLSLTCAISGDSVSSNSAAWSWIRQSPGRGLEWLGRIY YRSKWYNDYAVSVKSRITINPDTSKNQFSLQLNSVTPEDTAVYYCARLDHRYHED TVYPGMDVWGQGTLVTVSS | 50 |
| VL | DIELTQPPSVSVAPGQTARISCSGDNLPAYTVTWYQQKPGQAPVLVIYDDSDRPS GIPERFSGSNSGNTATLTISGTQAEDEADYYCASWDPSSGVVFGGGTKLTVL | 51 |
| HCDR1 | SNSAAWS | 52 |
| HCDR2 | RIYYRSKWYNDYAVSVKS | 53 |
| HCDR3 | LDHRYHEDTVYPGMDV | 54 |
| LCDR1 | SGDNLPAYTVT | 55 |
| LCDR2 | DDSDRPS | 56 |
| LCDR3 | ASWDPSSGVV | 57 |

### Example 2: Generation of compounds containing agonistic GLP-1 peptides

Various compounds containing agonistic GLP-1 peptides were synthesized. All compounds were generated using Fmoc-strategy and were synthesized and purified on state-of-the-art instrumentation using Rink resin as a solid support as exemplified below for Compound 9. Compounds contain the agonistic GLP-1 peptides, as well as linkers and reactive groups for conjugation to the antibody.

### SPPS synthesis and purification of Compound 9:

Structure see Figure 3 (bottom)
HGEGTFTSDVSSYLEEQAAKEFIAWLVKGGGK(MiniPEGAcBr) (SEQ ID No. 9)
1) Resin preparation: The DMF (20 mL) was added to the Rink amide MBHA Resin (0.50 mmol, 1.00 eq, Sub: 0.5 mmol/g) and agitated with N₂ at 25 °C for 0.5 hr. Then the mixture was filtered to get the resin. The resin was washed with DMF (40.0 mL * 5).
2) De-protection Fmoc group: 20% piperidine in DMF (40.0 mL) was added and agitated the resin with N₂ at 25 °C for 15 min. The resin was washed with DMF (40.0 mL * 5) and filtered to get the resin.
3) Coupling: A solution of Fmoc-Lys(Dde)-OH (3.0 eq) and DIEA (6.0 eq) in DMF (20.0 mL) was added to the resin, then the HBTU (2.85 eq) was added, the mixture was agitated with N₂ at 25 °C for 30 min. The resin was washed with DMF (40.0 mL * 5). The coupling reaction was monitored by ninhydrin color reaction.
4) Repeat above step 2 to 3 for the coupling of following amino acids: (2-30). Note:

| **#** | **Materials** | **Coupling reagents** |
|---|---|---|
| 1 | Fmoc-Lys(Dde)-OH (3.0 eq) | HBTU (2.85 eq) and DIEA (6.00 eq) |
| 2 | Fmoc-Gly-Gly-OH (3.0 eq) | HBTU (2.85 eq) and DIEA (6.00 eq) |
| 3 | Fmoc-Lys(Boc)-OH (3.0 eq) | HBTU (2.85 eq) and DIEA (6.00 eq) |
| 4 | Fmoc-Val-OH (3.0 eq) | HBTU (2.85 eq) and DIEA (6.00 eq) |
| 5 | Fmoc-Leu-OH (3.0 eq) | HBTU (2.85 eq) and DIEA (6.00 eq) |
| 6 | Fmoc-Trp(Boc)-OH (3.0 eq) | HBTU (2.85 eq) and DIEA (6.00 eq) |
| 7 | Fmoc-Ala-OH (3.0 eq) | HBTU (2.85 eq) and DIEA (6.00 eq) |
| 8 | Fmoc-Ile-OH (3.0 eq) | HBTU (2.85 eq) and DIEA (6.00 eq) |
| 9 | Fmoc-Phe-OH (3.0 eq) | HBTU (2.85 eq) and DIEA (6.00 eq) |
| 10 | Fmoc-Glu(OtBu)-OH (3.0 eq) | HBTU (2.85 eq) and DIEA (6.00 eq) |
| 11 | Fmoc-Lys(Boc)-OH (3.0 eq) | HBTU (2.85 eq) and DIEA (6.00 eq) |
| 12 | Fmoc-Ala-OH (3.0 eq) | HBTU (2.85 eq) and DIEA (6.00 eq) |
| 13 | Fmoc-Ala-OH (3.0 eq) | HBTU (2.85 eq) and DIEA (6.00 eq) |
| 14 | Fmoc-Gln(Trt)-OH (3.0 eq) | HBTU (2.85 eq) and DIEA (6.00 eq) |
| 15 | Fmoc-Glu(OtBu)-OH (3.0 eq) | HBTU (2.85 eq) and DIEA (6.00 eq) |
| 16 | Fmoc-Glu(OtBu)-OH (3.0 eq) | HBTU (2.85 eq) and DIEA (6.00 eq) |
| 17 | Fmoc-Leu-OH (3.0 eq) | HBTU (2.85 eq) and DIEA (6.00 eq) |
| 18 | Fmoc-Tyr(tBu)-OH (3.0 eq) | HBTU (2.85 eq) and DIEA (6.00 eq) |
| 19 | Fmoc-Ser(tBu)-OH (3.0 eq) | HBTU (2.85 eq) and DIEA (6.00 eq) |
| 20 | Fmoc-Ser(tBu)-OH (3.0 eq) | HBTU (2.85 eq) and DIEA (6.00 eq) |
| 21 | Fmoc-Val-OH (3.0 eq) | HBTU (2.85 eq) and DIEA (6.00 eq) |
| 22 | Fmoc-Asp(OtBu)-OH (3.0 eq) | HBTU (2.85 eq) and DIEA (6.00 eq) |
| 23 | Fmoc-Ser(tBu)-OH (3.0 eq) | HBTU (2.85 eq) and DIEA (6.00 eq) |
| 24 | Fmoc-Thr(tBu)-OH (3.0 eq) | HBTU (2.85 eq) and DIEA (6.00 eq) |
| 25 | Fmoc-Phe-OH (3.0 eq) | HBTU (2.85 eq) and DIEA (6.00 eq) |
| 26 | Fmoc-Thr(tBu)-OH (3.0 eq) | HBTU (2.85 eq) and DIEA (6.00 eq) |
| 27 | Fmoc-Gly-OH (3.0 eq) | HBTU (2.85 eq) and DIEA (6.00 eq) |
| 28 | Fmoc-Glu(OtBu)-OH (3.0 eq) | HBTU (2.85 eq) and DIEA (6.00 eq) |
| 29 | Fmoc-Gly-OH (3.0 eq) | HBTU (2.85 eq) and DIEA (6.00 eq) |
| 30 | Boc-His(Trt)-OH (3.0 eq) | HBTU (2.85 eq) and DIEA (6.00 eq) |

5) De-protection Dde group: 3% hydrazine hydrate in DMF (40.0 mL) was added and agitated the resin with N₂ at 25 °C for 30 min. The resin was washed with DMF (40.0 mL * 5) and filtered to get the resin.
6) Coupling: A solution of Fmoc-PEG4-OH (3.0 eq) (3.0 eq) and DIEA (6.00 eq) in DMF (20.0 mL) was added to the resin, then the HBTU (2.85 eq) was added, the mixture was agitated with N₂ at 25 °C for 30 min. The resin was washed with DMF (40.0 mL * 5). The coupling reaction was monitored by ninhydrin color reaction.
7) Repeat above step 2 to 3 for the coupling of following amino acids: (32). Note:

| | | |
|---|---|---|
| 31 | Fmoc-PEG4-OH (3.00 eq) | HBTU (2.85 eq) and DIEA (6.00 eq) |
| 32 | Fmoc-PEG4-OH (3.00 eq) | HBTU (2.85 eq) and DIEA (6.00 eq) |

8) De-protection Fmoc group: 20% piperidine in DMF (40.0 mL) was added and agitated the resin with N₂ at 25 °C for 15 min. The resin was washed with DMF (40.0 mL * 5) and filtered to get the resin.
9) Coupling AcBr: A solution of Br-Ac-OH (6.0 eq) and DIC (6.00 eq) in DMF (20.0 mL) was added to the resin and agitated with N₂ at 25 C for 30 min. The resin was washed with DMF (40.0 mL * 5). The coupling reaction was monitored by ninhydrin color reaction.

### Peptide Cleavage and Purification:

1) After SPPS completion, the resin was washed with DMF (50 mL) twice, washed with MeOH (50 mL) for 3 times and dried under vacuum to get the peptide resin 5.0 g.
2) 50 mL cleavage cocktail (2.5%H2O/2.5%Tis/2.5%MPA/92.5%TFA) was added to the flask containing the side chain protected peptide resin at 25 °C and the mixture was stirred for 120 min.
3) The cleavage cocktail was collected after filtration, the crude peptide was precipitated with 500 mL cold isopropyl ether and centrifuged (2 min at 3000 rpm) and washed two times with 500 mL isopropyl ether. Dry the crude peptide under vacuum 1 hr.
4) The crude peptide (2.0 g) was purified by prep-HPLC (TFA condition: A: 0.075 % TFA in H₂O, B: ACN). 217.8 mg of final product were obtained as a white solid (HPLC purity = 89.8%).

### Purification conditions:

| Separation condition | |
|---|---|
| Dissolution condition | Dissolve in 80%TFA-H2O |
| Instrument | Gilson GX-281 |
| Mobile Phase | A: H₂O (0.075% TFA in H₂O) |
| | B: CH₃CN |
| Gradient | 23-48-50 min, Retention time: 23.5 min |
| Column | luna ,10um, c18,100A, 25mm+Gemin,5um,c18,110A |
| Flow Rate | 20 mL/Min |
| Wavelength | 220/254 nm |
| Oven Tem. | 30°C |

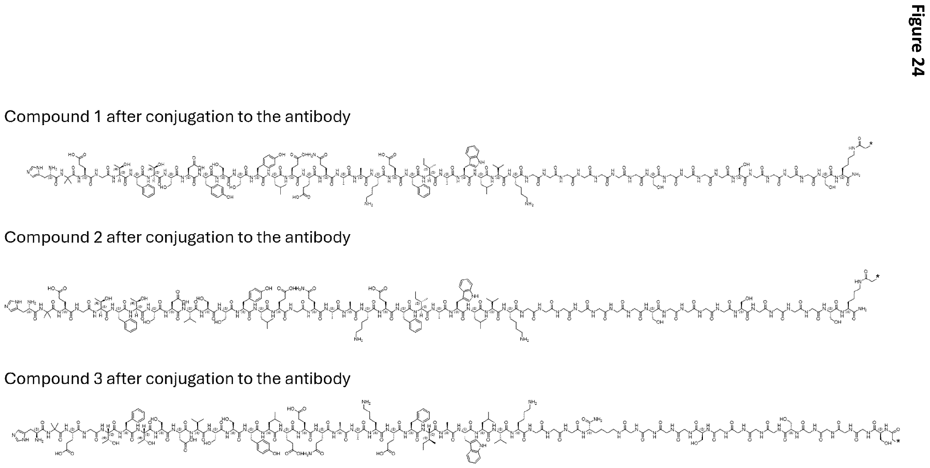

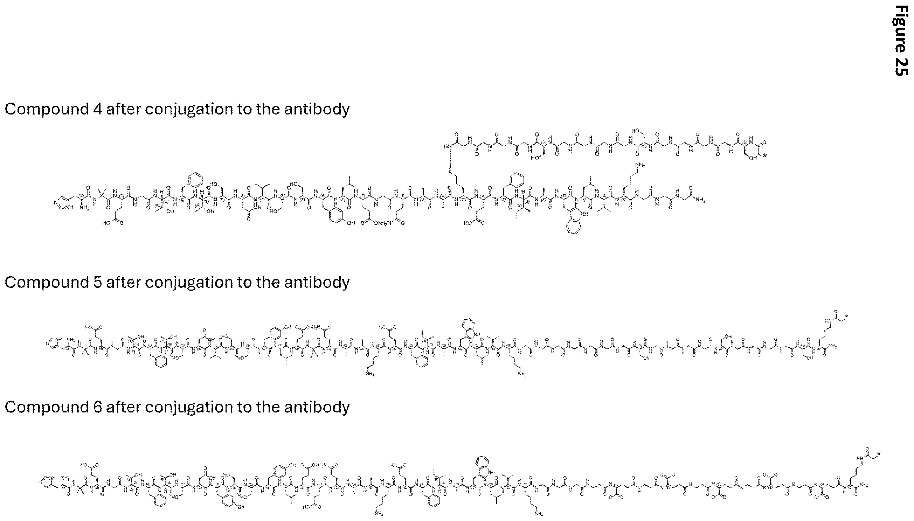

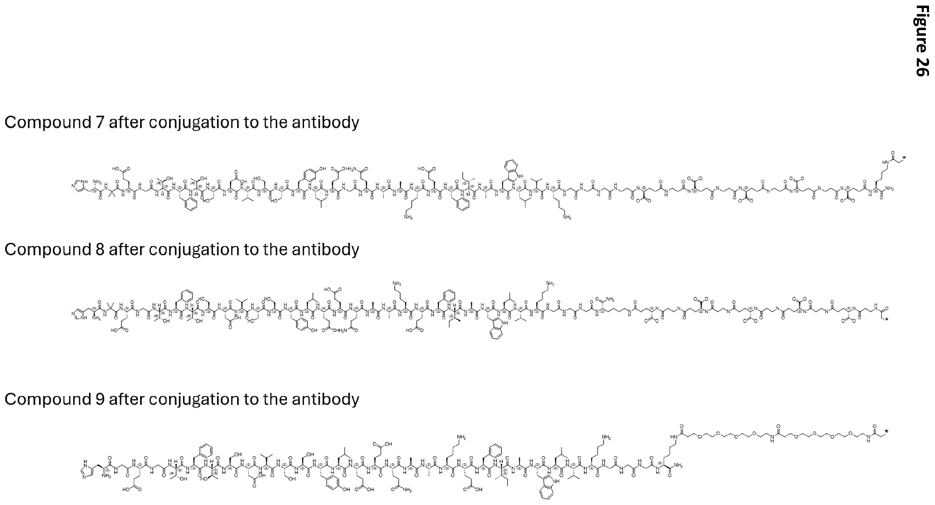

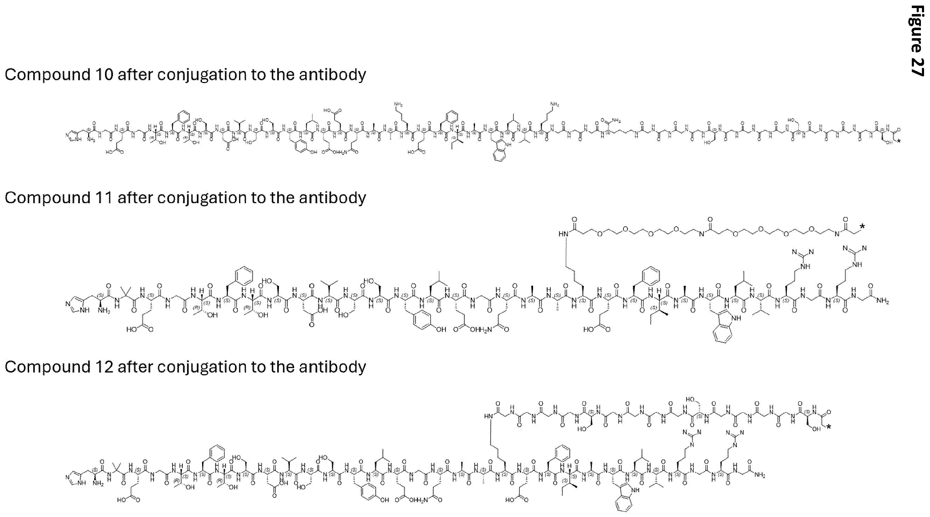

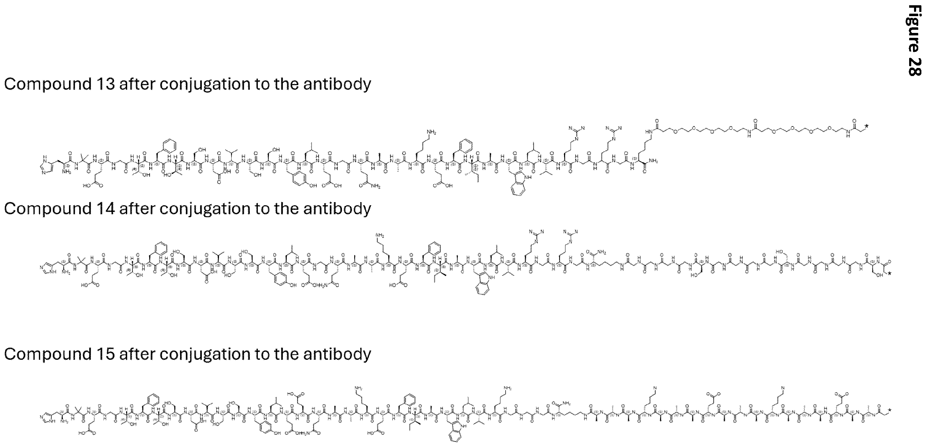

The following compounds were generated accordingly:
Compound 1:
   Structure of Compound 1:
      See Figure 1 (top)
      HAibEGTFTSDYSSYLEEQAAKEFIAWLVKGGGGGGGSGGGGSGGGGSK(AcBr) (SEQ ID No. 1) ESMS: MS cal.: 4600.7; MS observed: [M+3H]³⁺ = 1537.4
   **Structure** of Compound 1 after conjugation to the antibody:
      See Figure 24 (top)
      HAibEGTFTSDYSSYLEEQAAKEFIAWLVKGGGGGGGSGGGGSGGGGSK(Ac*) (SEQ ID No. 62)
   Amino acid sequence of the GLP-1 agonist contained in Compound 1:
      HAibEGTFTSDYSSYLEEQAAKEFIAWLVKGGG (SEQ ID No.77)
Compound 2:
   Structure of Compound 2:
      See Figure 1 (middle)
      HAibEGTFTSDVSSYLEGQAAKEFIAWLVKGGGGGGGSGGGGSGGGGSK(AcBr) (SEQ ID No. 2) ESMS: MS cal.: 4464.6; MS observed: [M+3H]³⁺ = 1489.0
   Structure of Compound 2 after conjugation to the antibody:
      See Figure 24 (middle)
      HAibEGTFTSDVSSYLEGQAAKEFIAWLVKGGGGGGGSGGGGSGGGGSK(Ac*) (SEQ ID No. 63)
   Amino acid sequence of the GLP-1 agonist contained in Compound 2:
      HAibEGTFTSDVSSYLEGQAAKEFIAWLVKGGG (SEQ ID No.78)
Compound 3:
   Structure of Compound 3:
      See Figure 1 (bottom)
      HAibEGTFTSDVSSYLEEQAAKEFIAWLVKGGGK(GGGGSGGGGSGGGGSAcBr) (SEQ ID No. 3) ESMS: MS cal.: 4536.6; MS observed: [M+3H]³⁺ = 1513.0
   Structure of Compound 3 after conjugation to the antibody:
      See Figure 24 (bottom)
      HAibEGTFTSDVSSYLEEQAAKEFIAWLVKGGGK(GGGGSGGGGSGGGGSAc*) (SEQ ID No. 64)
   **Amino acid** sequence of the GLP-1 agonist contained in Compound 3:
      HAibEGTFTSDVSSYLEEQAAKEFIAWLVKGGG (SEQ ID No.79)
Compound 4:
   Structure of Compound 4:
      See Figure 2 (top)
      HAibEGTFTSDVSSYLEGQAAK(GGGGSGGGGSGGGGSAcBr)EFIAWLVKGGG (SEQ ID No. 4) ESMS: MS cal.: 4336.4; MS observed: [M+3H]³⁺ = 1446.3
   Structure of Compound 4 after conjugation to the antibody:
      See Figure 25 (top)
      HAibEGTFTSDVSSYLEGQAAK(GGGGSGGGGSGGGGSAc*)EFIAWLVKGGG (SEQ ID No. 65)
   Amino acid sequence of the GLP-1 agonist contained in Compound 4:
      HAibEGTFTSDVSSYLEGQAAKEFIAWLVKGGG (SEQ ID No.78)
Compound 5:
   Structure of Compound 5:
      See Figure 2 (middle)
      HAibEGTFTSDVSSylEAibQAAKEFIAWLVKGGGGGGGSGGGGSGGGGSK(AcBr) (SEQ ID No. 5) ESMS: MS cal.: 4492.6; MS observed: [M+3H]³⁺ = 1498.4
   Structure of Compound 5 after conjugation to the antibody:
      See Figure 25 (middle)
      HAibEGTFTSDVSSylEAibQAAKEFIAWLVKGGGGGGGSGGGGSGGGGSK(Ac*) (SEQ ID No. 66)
   Amino acid sequence of the GLP-1 agonist contained in Compound 5:
      HAibEGTFTSDVSSylEAibQAAKEFIAWLVKGGG (SEQ ID No.80)
Compound 6:
   Structure of Compound 6:
      See Figure 2 (bottom)
      HAibEGTFTSDYSSYLEEQAAKEFIAWLVKGGGβAγEβAγEβAγEβAγEβAγEK(AcBr) (SEQ ID No. 6) ESMS: MS cal.: 4655.8; MS observed: [M+3H]³⁺ = 1552.7
   Structure of Compound 6 after conjugation to the antibody:
      See Figure 25 (bottom)
      HAibEGTFTSDYSSYLEEQAAKEFIAWLVKGGGβAγEβAγEβAγEβAγEβAγEK(Ac*) (SEQ ID No. 67)
   Amino acid sequence of the GLP-1 agonist contained in Compound 6:
      HAibEGTFTSDYSSYLEEQAAKEFIAWLVKGGG (SEQ ID No.77)
Compound 7:
   Structure of Compound 7:
      See Figure 3 (top)
      HAibEGTFTSDVSSYLEGQAAKEFIAWLVKGGGβAγEβAγEβAγEβAγEβAγEK(AcBr) (SEQ ID No. 7) ESMS: MS cal.: 4519.7; MS observed: [M+3H]³⁺ = 1507.4
   Structure of Compound 7 after conjugation to the antibody:
      See Figure 26 (top)
      HAibEGTFTSDVSSYLEGQAAKEFIAWLVKGGGβAγEβAγEβAγEβAγEβAγEK(Ac*) (SEQ ID No. 68)
   Amino acid sequence of the GLP-1 agonist contained in Compound 7:
      HAibEGTFTSDVSSYLEGQAAKEFIAWLVKGGG (SEQ ID No.78)
Compound 8:
   Structure of Compound 8:
      See Figure 3 (middle)
      HAibEGTFTSDVSSYLEEQAAKEFIAWLVKGGGK(yEβAγEβAγEβAγEβAγEβAAcBr) (SEQ ID No. 8) ESMS: MS cal.: 4591.8; MS observed: [M+3H]³⁺ = 1531.4
   Structure of Compound 8 after conjugation to the antibody:
      See Figure 26 (middle)
      HAibEGTFTSDVSSYLEEQAAKEFIAWLVKGGGK(γEβAγEβAγEβAγEβAγEβAAc*) (SEQ ID No. 69)
   Amino acid sequence of the GLP-1 agonist contained in Compound 8:
      HAibEGTFTSDVSSYLEEQAAKEFIAWLVKGGG (SEQ ID No.79)
Compound 9:
   Structure of Compound 9:
      See Figure 3 (bottom)
      HGEGTFTSDVSSYLEEQAAKEFIAWLVKGGGK(MiniPEGAcBr) (SEQ ID No. 9) ESMS: MS cal.: 4057.32; MS observed: [M+3H]³⁺ = 1353.31
   Structure of Compound 9 after conjugation to the antibody:
      See Figure 26 (bottom)
      HGEGTFTSDVSSYLEEQAAKEFIAWLVKGGGK(MiniPEGAc*) (SEQ ID No. 70) Amino acid sequence of the GLP-1 agonist contained in Compound 9: HGEGTFTSDVSSYLEEQAAKEFIAWLVKGGG (SEQ ID No.81)
Compound 10:
   Structure of Compound 10:
      See Figure 4 (top)
      HGEGTFTSDVSSYLEEQAAKEFIAWLVKGGGK(GGGGSGGGGSGGGGSAcBr) (SEQ ID No. 10) ESMS: MS cal.: 4508.5; MS observed: [M+3H]³⁺ = 1503.0
   Structure of Compound 10 after conjugation to the antibody:
      See Figure 27 (top)
      HGEGTFTSDVSSYLEEQAAKEFIAWLVKGGGK(GGGGSGGGGSGGGGSAc*) (SEQ ID No. 71)
   Amino acid sequence of the GLP-1 agonist contained in Compound 10:
      HGEGTFTSDVSSYLEEQAAKEFIAWLVKGGG (SEQ ID No.81)
Compound 11:
   Structure of Compound 11:
      See Figure 4 (middle)
      HAibEGTFTSDVSSYLEGQAAK(MiniPEGAcBr)EFIAWLVRGRG (SEQ ID No. 11) ESMS: MS cal.: 4012.2; MS observed: [M+3H]³⁺ = 1338.3
   Structure of Compound 11 after conjugation to the antibody:
      See Figure 27 (middle)
      HAibEGTFTSDVSSYLEGQAAK(MiniPEGAc*)EFIAWLVRGRG (SEQ ID No. 72)
   Amino acid sequence of the GLP-1 agonist contained in Compound 11:
      HAibEGTFTSDVSSYLEGQAAKEFIAWLVRGRG (SEQ ID No.82)
Compound 12:
   Structure of Compound 12:
      See Figure 4 (bottom)
      HAibEGTFTSDVSSYLEGQAAK(GGGGSGGGGSGGGGSAcBr)EFIAWLVRGRG (SEQ ID No. 12) ESMS: MS cal.: 4463.5; MS observed: [M+3H]³⁺ = 1488. 6
   Structure of Compound 12 after conjugation to the antibody:
      See Figure 27 (bottom) HAibEGTFTSDVSSYLEGQAAK(GGGGSGGGGSGGGGSAc*)EFIAWLVRGRG (SEQ ID No. 73)
   Amino acid sequence of the GLP-1 agonist contained in Compound 12:
      HAibEGTFTSDVSSYLEGQAAKEFIAWLVRGRG (SEQ ID No.82)
Compound 13:
   Structure of Compound 13:
      See Figure 5 (top)
      HAibEGTFTSDVSSYLEGQAAKEFIAWLVRGRGK(MiniPEGAcBr) (SEQ ID No. 13) ESMS: MS cal.: 4041.7; MS observed: [M+2H]²⁺ = 2071.0
   Structure of Compound 13 after conjugation to the antibody:
      See Figure 28 (top)
      HAibEGTFTSDVSSYLEGQAAKEFIAWLVRGRGK(MiniPEGAc*) (SEQ ID No. 74)
   Amino acid sequence of the GLP-1 agonist contained in Compound 13:
      HAibEGTFTSDVSSYLEGQAAKEFIAWLVRGRG (SEQ ID No.82)
Compound 14:
   Structure of Compound 14:
      See Figure 5 (middle)
      HAibEGTFTSDVSSYLEGQAAKEFIAWLVRGRGK(GGGGSGGGGSGGGGSACcBr) (SEQ ID No. 14)
      ESMS: MS cal.: 4591.7; MS observed: [M+2H]³⁺ = 1148.8
   Structure of Compound 14 after conjugation to the antibody:
      See Figure 28 (middle)
      HAibEGTFTSDVSSYLEGQAAKEFIAWLVRGRGK(GGGGSGGGGSGGGGSAc*) (SEQ ID No. 75)
   Amino acid sequence of the GLP-1 agonist contained in Compound 14:
      HAibEGTFTSDVSSYLEGQAAKEFIAWLVRGRG (SEQ ID No.82)
Compound 15:
   Structure of Compound 15:
      See Figure 5 (bottom)
      HAibEGTFTSDVSSYLEEQAAKEFIAWLVKGGGK(AAAKAAAEAAAKAAAEAAAcBr) (SEQ ID No. 15) ESMS: MS cal.: 5100.5; MS observed: [M+3H]³⁺ = 1701.2
   Structure of Compound 15 after conjugation to the antibody:
      See Figure 28 (bottom)
      HAibEGTFTSDVSSYLEEQAAKEFIAWLVKGGGK(AAAKAAAEAAAKAAAEAAAc*) (SEQ ID No. 76)
   Amino acid sequence of the GLP-1 agonist contained in Compound 15:
      HAibEGTFTSDVSSYLEEQAAKEFIAWLVKGGG (SEQ ID No.79)

Abbreviations:
Aib: Aminoisobutyric acid
BrAc: Bromoacetic acid
βA: β-Alanine
γE: γ-Glutamic acid
MiniPEG: 1-Amino-3,6,9,12-tetraoxapentadecan-15-oic acid - CAS 581065-95-4
MBHA Resin: 4-Methylbenzhydrylaminhydrochloride
DDE: 2-Acetyldimedone
MPA: 3-Mercaptopropionic acid
DIC: N,N'-Diisopropylcarbodiimid
TIS: Triisopropylsilane
HBTU: (2-(1H-Benzotriazol-1-yl)-1,1,3,3-tetramethyluronium-hexafluorophosphate

### Example 3: Conjugation of peptides to cysteine engineered antibodies

Compounds were coupled to the cysteine engineered antibodies via halogen-substitution of accordingly modified peptides, i.e. Bromo acetylated peptides. 5mg of the antibody dissolved in Histidine/NaCl buffer (conc 5mg/ml) was buffer exchanged to the same volume of reduction buffer (50mM Na2SO4/2.0mM EDTA, pH7.5) via 3 cycles on Amicon Ultra-4, Centrifual Filters, Ultracel - 30K (3x 10min, 10000rpm). To the final volume 100eq of TCEP solution in water (0.5M conc) was added and the reaction vessel was shaken at 20° C for 22h. After the mix was desalted into new reaction buffer using Zeba desalt columns (MWCO 40000, prepared for use according to the buffer exchange procedure (Thermo Scientific)), using conjugation buffer (centrifuge to remove storage buffer 2 min, add 2 ml reaction buffer, centrifuge 2min, repeat 2x, centrifuge 6 min after 3rd buffer addition - centrifuge speed 700 g). Sample was then added to the desalt column and centrifuged for 4 minutes to collect the desalted sample. The eluent was then transferred into a 2ml Eppendorf tube and 20eq DHAA solution in PBS (10mM conc) was added. The solution was slightly shaken at RT. After another 22h 10eq BrAc modified peptide solution (10mM in Water) was added and the mixture was shaken at RT over night. Thereafter the sample was filter-purified and buffer exchanged to PBS buffer via 3 cycles on Amicon Ultra-4, Centrifual Filters, Ultracel - 10K (3x 10min, 10000rpm). The obtained conjugate in PBS (conc ca 5mg/ml) was then purified by prep SEC (Superdex 200 Increase, 10/300 GL column, PBS as eluent) and after pooling the pure fractions and the final analysis of the product was done by HIC-chromatography and LCMS. Final concentration was determined by Nanodrop UV. All immunoconjugates were characterized and quality assessed (MS, SDS PAGE, SEC, HIC, Heparin binding columns).

The following table shows the nomenclature of the conjugates used in subsequent experiments (Compound = Compound as defined in Example 2):

| **Conjugate ID** | **Antibody** | **Compound** |
|---|---|---|
| Conjugate 1 | Wilma | 1 |
| Conjugate 2 | Wilma | 2 |
| Conjugate 3 | Wilma | 3 |
| Conjugate 4 | Wilma | 4 |
| Conjugate 5 | Wilma | 5 |
| Conjugate 6 | Slate | 1 |
| Conjugate 7 | Bamm-Bamm | 1 |
| Conjugate 8 | Bamm-Bamm | 2 |
| Conjugate 9 | Bamm-Bamm | 3 |
| Conjugate 10 | Bamm-Bamm | 6 |
| Conjugate 11 | Bamm-Bamm | 8 |
| Conjugate 12 | Betty | 3 |
| Conjugate 13 | Dino | 3 |
| Conjugate 14 | Slate | 3 |
| Conjugate 15 | Wilma | 6 |
| Conjugate 16 | Wilma | 7 |
| Conjugate 17 | Wilma | 8 |
| Conjugate 18 | Bamm-Bamm | 9 |
| Conjugate 19 | Bamm-Bamm | 10 |
| Conjugate 20 | Bamm-Bamm | 11 |
| Conjugate 21 | Bamm-Bamm | 12 |
| Conjugate 22 | Bamm-Bamm | 13 |
| Conjugate 23 | Bamm-Bamm | 15 |
| Conjugate 24 | Bamm-Bamm | 4 |
| Conjugate 25 | Bamm-Bamm | 5 |
| Conjugate 26 | Bamm-Bamm | 2* |
| Conjugate 27 | Bamm-Bamm | 3* |
| Conjugate 28 | Bamm-Bamm | 4* |
| Conjugate 29 | Bamm-Bamm | 5* |
| Conjugate 30 | Caveman | 9 |
| Conjugate 31 | Caveman | 10 |
| Conjugate 32 | Caveman | 11 |
| Conjugate 33 | Caveman | 12 |
| Conjugate 34 | Caveman | 13 |
| Conjugate 35 | Caveman | 14 |
| Conjugate 36 | Fred | 9 |
| Conjugate 37 | Fred | 10 |
| Conjugate 38 | Fred | 11 |
| Conjugate 39 | Fred | 12 |
| Conjugate 40 | Fred | 13 |
| Conjugate 41 | Fred | 14 |
| Conjugate 42 | Barney | 9 |
| Conjugate 43 | Barney | 10 |
| Conjugate 44 | Barney | 11 |
| Conjugate 45 | Barney | 12 |
| Conjugate 46 | Barney | 13 |
| Conjugate 47 | Barney | 14 |

| | | |
|---|---|---|
| * : these conjugates only carry one agonistic GLP-1 peptide on the engineered cysteine residues of the constant region | | |

### Example 4: ActR2A/B binding & functional activity

### Example 4.1: Affinity of antibody & conjugates to ActRIIA and ActRIIB

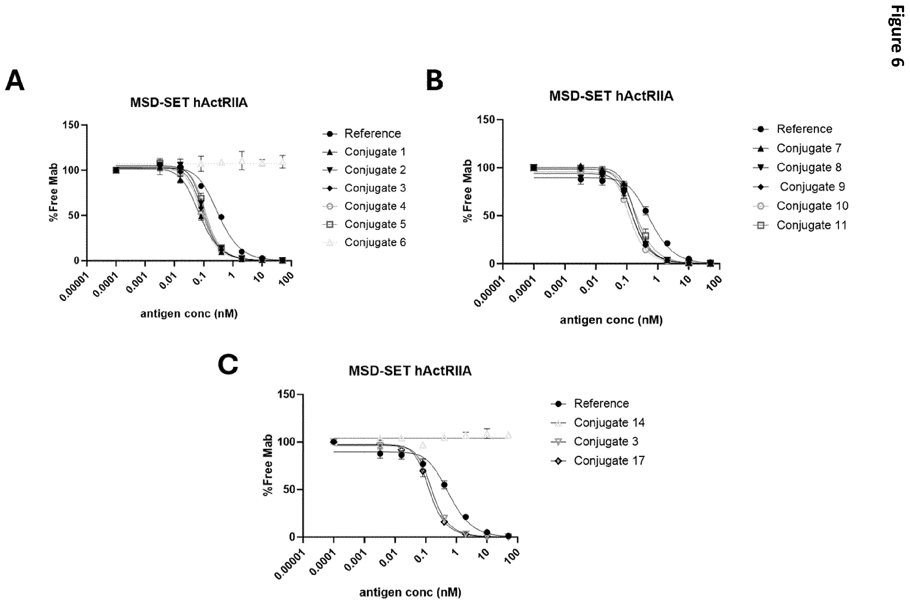

The affinity interaction profile of the immunoglobulins was determined using an immuno-based-assay called Meso Scale Discovery (MSD) Solution Equilibrium Titration (MSD-SET). In this assay, the antigen (either ActRIIA or ActRIIB) was coated onto an MSD 96-well detection plate. The SET plate was prepared by mixing the immunoglobulins at a constant concentration with titrated antigen and incubated overnight at 4°C to reach equilibrium. After blocking of the MSD plate, 50 µl of the sample solutions containing the Immunoglobulin-Antigen mix (SET plate) were transferred onto the antigen-coated detection MSD plate. These samples were then incubated for 20 minutes to allow for binding of any free antibodies without significantly shifting the equilibrium. To detect the bound antibodies on the plate, a detection antibody Anti Human Antibody (Goat) Sulfo-TAG Labelled (PN: R32AJ-5) was added. The electrochemiluminescence (ECL) readout was then detected by adding a Read Buffer and measuring ECL using an MSD instrument (Sector S 600MM). Data analysis was performed on GraphPad Prism using equation for IgG from Journal of Biomolecular Screening 2015, Vol. 20(10) 1256-1267. Tested in this experiment were various antibodies, containing an engineered cysteine at position 272 (E272C). Antibodies were conjugated to peptides, and binding to receptors ActRIIA and ActRIIB was compared to the unconjugated cysteine-engineered antibody. Results of the binding affinities of the conjugates tested to ActR2A and ActR2B receptors are shown in the following table (nb= non-binding) and in Figures 6 (ActRIIA) and 7 (ActRIIB):

| **Conjugate ID** | **Affinity ActR2A [pM]** | **Affinity ActR2B [pM]** |
|---|---|---|
| Conjugate 1 | 26 | 3 |
| Conjugate 2 | 23 | 3 |
| Conjugate 3 | 23 | 4 |
| Conjugate 4 | 20 | 2 |
| Conjugate 5 | 26 | 4 |
| Conjugate 6 | nb | nb |
| Conjugate 7 | 40 | 8 |
| Conjugate 8 | 42 | 8 |
| Conjugate 9 | 37 | 9 |
| Conjugate 10 | 30 | 10 |
| Conjugate 11 | 60 | 12 |
| Conjugate 14 | nb | nb |
| Conjugate 17 | 31 | 7 |
| Conjugate 24 | 22 | 3 |
| Conjugate 25 | 23 | 3 |
| Conjugate 40 | 40 | 33 |
| Conjugate 41 | 59 | 38 |

It was observed that all conjugates had a similar affinity to ActR2A and ActR2B. Only the control antibodies (Conjugate 6 and Conjugate 14) did not bind to ActR2A and ActR2B. Conjugation of the agonistic GLP-1 peptides hence did not affect the affinity of the antibody.

### Example 4.2: Antagonistic activity in ActRIIA/B reporter cell assays

Furthermore, antagonistic activity was assessed in an ActRIIA/B reporter cell assay. TGFB/SMAD Signaling Pathway SBE Reporter HEK293 cells (BPS BioScience) were washed with PBS, detached using 0.05% Trypsin and seeded at 35000 cells/well in a 96-well white flat-bottom plate in 100 µL Medium (MEM media, 10% FBS, 1% NEAA, 1 mM Na pyruvate, 1% Pen/Strep). The next day, medium was exchanged with 90 µL assay medium (MEM media, 0.5% FBS, 1% NEAA, 1 mM Na pyruvate, 1% Pen/Strep) containing diluted Antibodies of interest (10-0.00015 ug/mL) or Control. Cells were incubated at 37°C, 5% CO₂ for 4h. Subsequently, 10 µL of 10ng/ml Activin (Cat#120-14E, Peprotech) or 500 ng/mL Myostatin (Cat#120-00, Peprotech) were added to the cells and cells incubated overnight. The next day, 100 µL of ONE-Step Luciferase Detection reagent (BPS Bioscience) were added to the cell solution (both equilibrated to room temperature) and incubated for 15 min on a plate shaker, before measuring luminescence using the Envision Plate Reader.

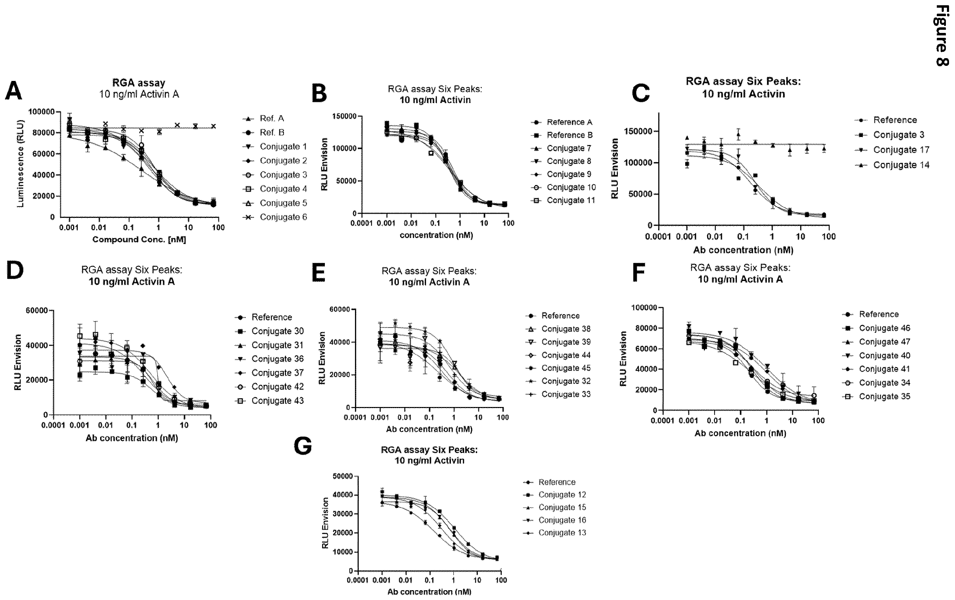

Tested in this experiment were various antibodies, containing an engineered cysteine at position 272. Antibodies were conjugated to various peptides as indicated below, and activity in ActRIIA/B reporter cell assays was compared to the unconjugated cysteine-engineered antibody. Functional ActR2A/B activity as measured by SMAD phosphorylation are shown in the following table (nd = not determined) and in Figures 8 (Activin A) and 9 (Myostatin):

| **Conjugate ID** | **IC50 ActR2A/B [pM] - Activin A** | **IC50 ActR2A/B [pM] - Myostatin** |
|---|---|---|
| Conjugate 1 | 687 | 621 |
| Conjugate 2 | 490 | 567 |
| Conjugate 3 | 721 | 605 |
| Conjugate 4 | 784 | 870 |
| Conjugate 5 | 596 | 442 |
| Conjugate 6 | inactive | inactive |
| Conjugate 7 | 307 | nd |
| Conjugate 8 | 330 | nd |
| Conjugate 9 | 465 | nd |
| Conjugate 10 | 442 | nd |
| Conjugate 11 | 359 | nd |
| Conjugate 12 | 1101 | nd |
| Conjugate 13 | 767 | nd |
| Conjugate 14 | inactive | nd |
| Conjugate 15 | 675 | nd |
| Conjugate 16 | 308 | nd |
| Conjugate 17 | 261 | nd |
| Conjugate 30 | 584 | nd |
| Conjugate 31 | 615 | nd |
| Conjugate 32 | 312 | 564 |
| Conjugate 33 | 543 | 789 |
| Conjugate 34 | 279 | 750 |
| Conjugate 35 | 243 | 787 |
| Conjugate 36 | 986 | nd |
| Conjugate 37 | 2129 | nd |
| Conjugate 38 | 1514 | nd |
| Conjugate 39 | 1631 | nd |
| Conjugate 40 | 1118 | 1946 |
| Conjugate 41 | 1035 | 1611 |
| Conjugate 42 | 645 | nd |
| Conjugate 43 | 494 | nd |
| Conjugate 44 | 516 | nd |
| Conjugate 45 | 221 | nd |
| Conjugate 46 | 269 | nd |
| Conjugate 47 | 312 | nd |

It can be seen that the conjugates also remain fully functional with respect to ActR2 receptor pharmacology.

### Example 4.3: Affinity of the antibody conjugates to GLPR

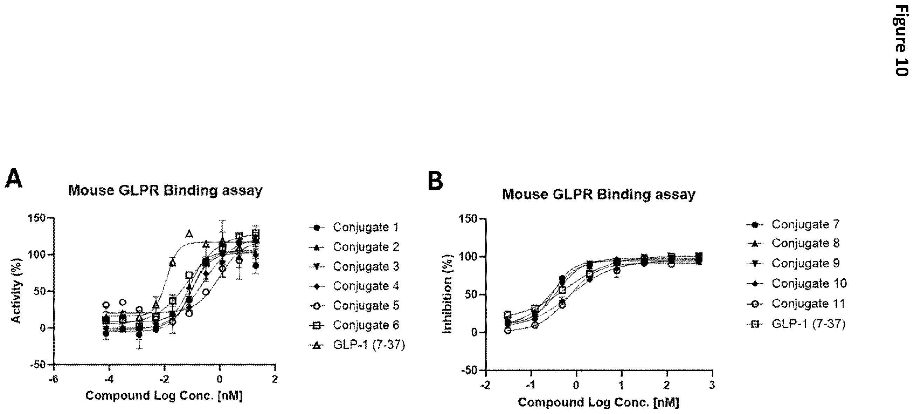

The binding profile of the conjugates was determined using a radioligand competition binding assay. In this assay, the GLP-1 receptor (membrane preparation from human [h], cynomolgus [c] or mouse [m] GLP-1R expressing HEK293 cells) at a fixed concentration of 6, 10 or 2 µg/well respectively was incubated with 0.15 nM [125I]-GLP-1 (7-36) radioligand (PerkinElmer) and increasing concentrations (4-fold dilution, ranging from 0.03 to 500 nM) of unlabeled GLP-1 (7-36) as reference or IgG conjugates. After 1 hour incubation at room temperature, the reaction mixture was filtered through pre-soaked (30 min with 50 µL of 50mM HEPES/0.5% BSA) UniFilter-96 GF/C filter plates using Perkin Elmer FilterMate Harvester. After 8 washes with cold wash buffer, the filter plates were dried for 1 hour at 50°C and the bottom sealed using Unifilter-96 backing seal tape (PerkinElmer). Then, after addition of 45 µL of MicroScint-O cocktail (PerkinElmer), the top of the filter plates was sealed with TopSeal-A sealing film (PerkinElmer). Radioactivity trapped on the filter was measured using a Perkin Elmer MicroBeta2 Microplate scintillation counter. Binding data were normalised to % Inhibition and curves were plotted using the non-linear regression 4PL analysis in the Prism software (GraphPad Software, San Diego, CA). Results are shown in the follow table and in Figures 10 (mouse), 11 (cyno) and 12 (human).

| **Conjugate ID** | **GLPR binding (pM) m/c/h** | **% inhibition m/c/h** |
|---|---|---|
| Conjugate 1 | 579/374/426 | 99/99/97 |
| Conjugate 2 | 431/388/612 | 98/90/97 |
| Conjugate 3 | 549/364/619 | 99/106/97 |
| Conjugate 4 | 3627/1441/1831 | 87/90/92 |
| Conjugate 5 | 316700/90540/330500 | 49/55/59 |
| Conjugate 6 | 580/449/1972 | 98/96/94 |
| Conjugate 7 | 310/560/436 | 94/96/96 |
| Conjugate 8 | 292/443/449 | 99/93/97 |
| Conjugate 9 | 379/548/398 | 100/95/95 |
| Conjugate 10 | 739/663/667 | 95/78/90 |
| Conjugate 11 | 657/435/511 | 95/104/90 |

| | | |
|---|---|---|
| m=mouse; c=cynomolgus monkey; h=human | | |

It can be seen that all conjugates (except Conjugates 4 & 5) remain strongly binding to the GLPR.

### Example 4.4: Functional GLPR activity in a cAMP assay

Before assay set up, assay buffer was prepared as 1x stimulation buffer (Cisbio #62AM4PEJ) containing 500uM IBMX (Sigma, #I5879). Compounds were 4-fold serially diluted 10-points in assay buffer using the Bravo V11 Machine (Agilent) and 5µL of compounds were transferred to the OptiPlate-384 (PerkinElmer, #6007290) to reach as highest concentration 20 nM in the assay plate. The plate was centrifuged for 5 sec at 1000rpm.

HEK293 cells expressing GLP-1R (constructed by WuXi Apptech) were detached using 0.25% Trypsin at 37°C for a couple of minutes. When cells detached, cell suspension was diluted in 10mL of HBSS (Invitrogen, #14025) and transferred into a 15mL tube. Cells were centrifuged at 1000rpm for 5minutes at room temperature. Supernatant was removed and cell pellet resuspended in 10mL HBSS. Cells were re-suspended in assay buffer to a final concentration of 0.2Mio cells/mL. 10µL of this cell solution was transferred to the Optiplate-384.

The plate was incubated for 30min at 23°C before adding 10µL cAMP working detection solution (38 parts of cAMP Lysis Buffer, 1 part of cAMP-D2, 1 part anti-cAMP cryptate reagent) as described by the provider (CisBio, #62AM4PEJ). The plate was covered with TopSeal-A film and incubated for 60min at room temperature. TopSeal-A film was removed, and signal analyzed using the EnVision2015 (PerkinElmer).

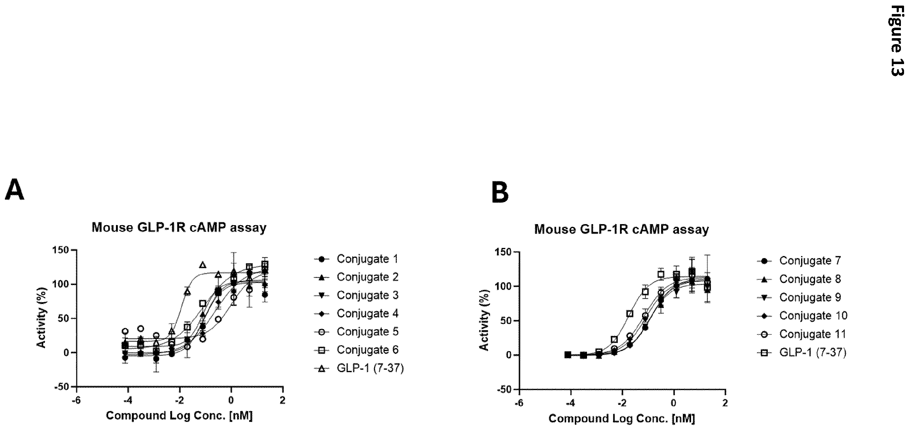

Results are shown in the table of Example 4.5 and in Figures 13 (mouse GLP1-R), 14 (rat GLP1-R), 15 (cynomolgus GLP1-R) and 16 (human GLP1-R).

### Example 4.5: Functional GLPR activity in a Reporter Gene Assay

HEK293 cells expressing GLP-1R (BPS Bioscience, #78176) were washed with 14mL PBS and detached using 0.05% Trypsin at RT for a couple of minutes. When cells detached, cell suspension was diluted to 10mL of Growth medium (MEM (Gibco, #11095-080), 10% FBS, 1% NEAA, 1% sodium pyruvate, 100U/mL P/S, 400ug/mL Geneticin, 100ug/mL Hygromycin) and transferred into a 50mL tube. Cells were centrifuged at 300rcf for 5minutes at room temperature. Supernatant was removed and cell pellet resuspended in Thaw medium (MEM (Gibco, #11095-080), 10% FBS, 1% NEAA, 1% sodium pyruvate, 100U/mL P/S) to a final concentration of 0.3Mio cells/mL. 100µL of this cell solution was transferred to the microtiter plate (Corning, #3610). The plate was incubated overnight at 37°C, 5% CO₂.

The next day, compounds were 5-fold serially diluted 10-points in assay buffer (Opti-MEM (Gibco, #31985-070), 0.1% BSA) in a separate polypropylene plate from the highest concentration of 50nM. Media was removed from the cells by aspiration and 100µL of compounds were transferred to the assay plate. The plate was incubated for 6hours at 37°C, 5%CO₂.

Reading solution was prepared by mixing 10mL of Component A and 100uL of Component B (BPS Bioscience, #60690) per assay plate. 100µL of working solution was added to the assay plate and incubated for 15min at RT. The bottom of the plate was covered with BackSeal (Revvity, #6005199), and signal analyzed using the EnVision2015 (PerkinElmer).

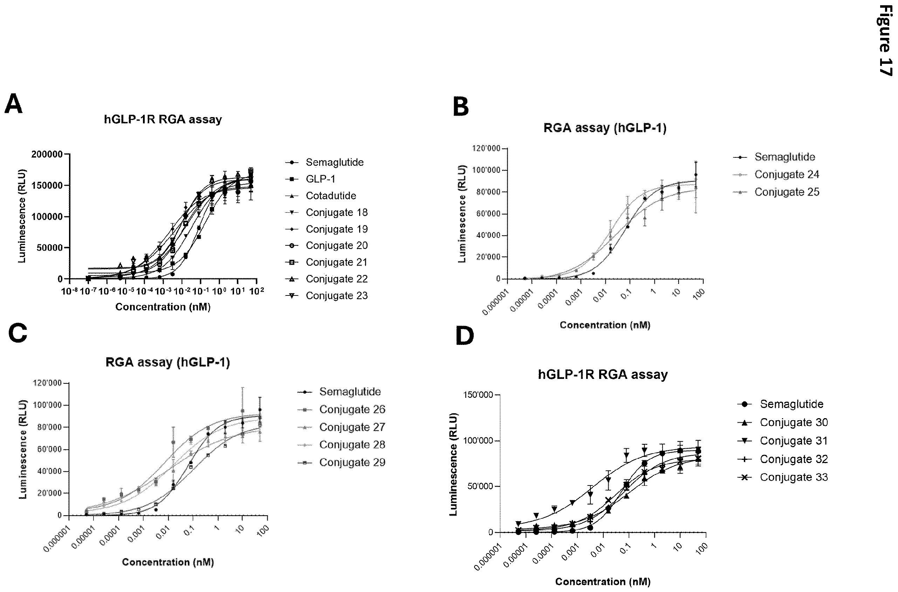

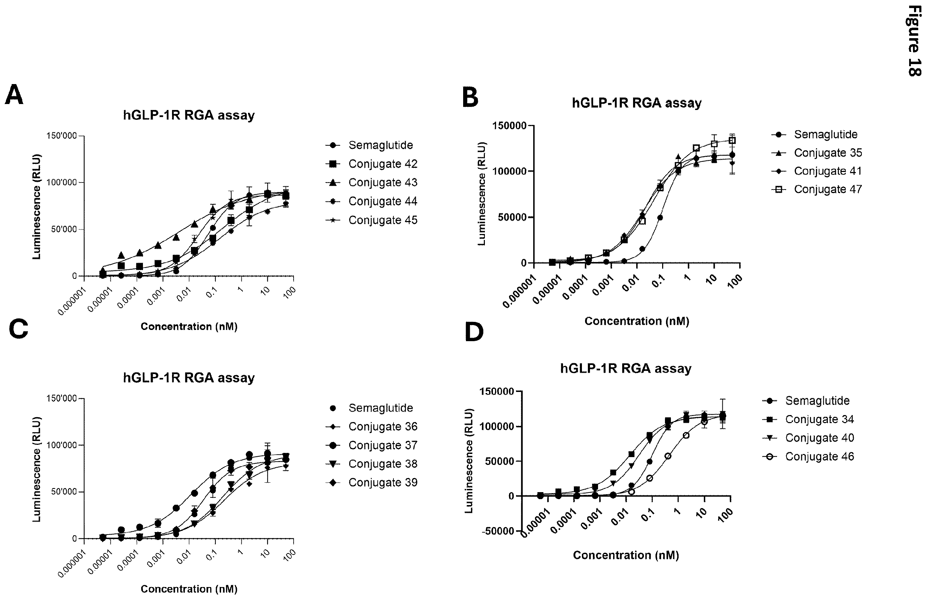

Results of the cAMP assay (Example 4.4) and the reporter gene assay are shown in the following table and Figures 17 and 18:

| **Conjugate ID** | **EC50 GLP-1 [pM] m/r/c/h** | **% efficacy m/r/c/h** | **Assay type** |
|---|---|---|---|
| Conjugate 1 | 88/70/20/46 | 85/93/100/93 | cAMP |
| Conjugate 2 | 80/80/26/50 | 102/88/103/101 | cAMP |
| Conjugate 3 | 96/92/21/51 | 97/98/103/99 | cAMP |
| Conjugate 4 | 237/427/25/51 | 118/95/112/98 | cAMP |
| Conjugate 5 | 977/4091/434/155 | 122/92/73/79 | cAMP |
| Conjugate 6 | 83/116/28/77 | 129/87/112/98 | cAMP |
| Conjugate 7 | 133/161/30/86 | 111/112/87/94 | cAMP |
| Conjugate 8 | 114/127/28/110 | 95/115/100/97 | cAMP |
| Conjugate 9 | 122/88/31/125 | 99/101/90/101 | cAMP |
| Conjugate 10 | 81/65/37/123 | 102/98/113/104 | cAMP |
| Conjugate 11 | 70/68/18/85 | 106/112/118/100 | cAMP |
| Conjugate 12 | nd/nd/nd/162 | nd/nd/nd/91 | cAMP |
| Conjugate 13 | nd/nd/nd/115 | nd/nd/nd/107 | cAMP |
| Conjugate 14 | nd/nd/nd/? | nd/nd/nd/? | cAMP |
| Conjugate 15 | nd/nd/nd/79 | nd/nd/nd/110 | cAMP |
| Conjugate 16 | nd/nd/nd/64 | nd/nd/nd/98 | cAMP |
| Conjugate 17 | nd/nd/nd/221 | nd/nd/nd/99 | cAMP |
| Conjugate 18 | nd/nd/nd/31 | nd/nd/nd/101 | RGA |
| Conjugate 18 | nd/nd/nd/2 | nd/nd/nd/91 | RGA |
| Conjugate 20 | nd/nd/nd/10 | nd/nd/nd/92 | RGA |
| Conjugate 21 | nd/nd/nd/15 | nd/nd/nd/102 | RGA |
| Conjugate 22 | nd/nd/nd/162 | nd/nd/nd/101 | RGA |
| Conjugate 23 | nd/nd/nd/10 | nd/nd/nd/101 | RGA |
| Conjugate 24 | nd/nd/nd/16 | nd/nd/nd/96 | RGA |
| Conjugate 25 | nd/nd/nd/27 | nd/nd/nd/93 | RGA |
| Conjugate 26 | nd/nd/nd/11 | nd/nd/nd/102 | RGA |
| Conjugate 27 | nd/nd/nd/10 | nd/nd/nd/89 | RGA |
| Conjugate 28 | nd/nd/nd/15 | nd/nd/nd/98 | RGA |
| Conjugate 29 | nd/nd/nd/100 | nd/nd/nd/92 | RGA |
| Conjugate 30 | nd/nd/nd/103 | nd/nd/nd/93 | RGA |
| Conjugate 31 | nd/nd/nd/4 | nd/nd/nd/105 | RGA |
| Conjugate 32 | nd/nd/nd/65 | nd/nd/nd/97 | RGA |
| Conjugate 33 | nd/nd/nd/32 | nd/nd/nd/87 | RGA |
| Conjugate 34 | nd/nd/nd/14 | nd/nd/nd/97 | RGA |
| Conjugate 35 | nd/nd/nd/21 | nd/nd/nd/100 | RGA |
| Conjugate 36 | nd/nd/nd/214 | nd/nd/nd/91 | RGA |
| Conjugate 37 | nd/nd/nd/11 | nd/nd/nd/102 | RGA |
| Conjugate 38 | nd/nd/nd/201 | nd/nd/nd/101 | RGA |
| Conjugate 39 | nd/nd/nd/36 | nd/nd/nd/92 | RGA |
| Conjugate 40 | nd/nd/nd/30 | nd/nd/nd/97 | RGA |
| Conjugate 41 | nd/nd/nd/182 | nd/nd/nd/97 | RGA |
| Conjugate 42 | nd/nd/nd/182 | nd/nd/nd/109 | RGA |
| Conjugate 43 | nd/nd/nd/4 | nd/nd/nd/104 | RGA |
| Conjugate 44 | nd/nd/nd/148 | nd/nd/nd/91 | RGA |
| Conjugate 45 | nd/nd/nd/22 | nd/nd/nd/97 | RGA |
| Conjugate 46 | nd/nd/nd/452 | nd/nd/nd/100 | RGA |
| Conjugate 47 | nd/nd/nd/45 | nd/nd/nd/115 | RGA |

| | | | |
|---|---|---|---|
| m=mouse; r=rat; c=cynomolgus monkey; h=human; nd=not determined | | | |

It can be seen that all conjugates remain fully functional with respect to GLP1R activation.

### Example 4.6: Conclusion from in vitro studies

It was found that the conjugation of agonistic GLP-1 peptides to ActRll antibodies does not effect the activity of either part of the molecule. Specifically, the conjugated agonistic GLP-1 peptide does not affect binding and activity of the ActRll antibody and fully translates into functional ActRII activity. Likewise, the activity of the agonistic GLP-1 peptide when conjugated to the ActRll antibody is equivalent to the isolated (unconjugated) agonistic GLP-1 peptide. Both parts of the molecules, i.e. the agonistic GLP-1 peptide and the ActRll antibody, remain fully active despite the conjugation of the respective other part of the molecule.

This finding provides great advantages for the development of a respective therapeutic agent. Compared to a combination therapy consisting of the two individual moieties, a conjugation product only requires a single formulation, a single route of administration, and avoids the necessity to identify a compatible dosage regimen for two individual molecules.

### Example 5: In vivo efficacy studies

The in vitro studies shown herein were experimentally verified by in vivo studies. In fact, the in vivo studies demonstrated that certain conjugates have specific advantageous properties. For these studies, diet-induced obesity (DIO) mice were used as a model to measure weight loss and body composition improvements after treatment with anti-ActRIIA/B-GLP-1 conjugates.

C57BL/6JRj male mice obtained from Janvier Labs were subjected to a 60% high fat diet (HFD) from the age of 5 weeks onwards for a period of 21 weeks before study enrolment. Animals were randomized before dosing into treatment groups based on bodyweight and body composition measurements.

### Example 5.1: First in vivo study

In this in vivo study, antibody conjugates (or their respective vehicle, 0.9% NaCl) were administered twice-weekly via intraperitoneal (IP) injection for 2 weeks, whilst semaglutide (or its respective vehicle, phosphate buffered saline containing 0.1 % bovine serum albumin), was administered via daily subcutaneous (SC) injections of 10 nmol/kg for 17 days.

Treatment groups:
Conjugate 1 dosed at 2.5mg/kg
Conjugate 2 dosed at 2.5mg/kg
Conjugate 3 dosed at 2.5mg/kg
Conjugate 4 dosed at 2.5mg/kg
Conjugate 5 dosed at 2.5mg/kg
Conjugate 3 dosed at 7.5mg/kg
Conjugate 5 dosed at 7.5mg/kg

Control groups:
Vehicle (0.9% NaCl)
Semaglutide dosed at 10 nmol/kg
Reference antibody (unconjugated) ActR2A/B binding mAb dosed at 7.5mg/kg
Conjugate 6 (antibody specific for hen egg lysozyme conjugated to peptide 1) dosed at 2.5mg/kg)

HFD was continued during treatment. The bodyweight of the animals was recorded daily. Food intake was measured daily during the first treatment week and biweekly thereafter. The body composition (fat and lean mass) was analyzed weekly by magnetic resonance imaging (MRI).

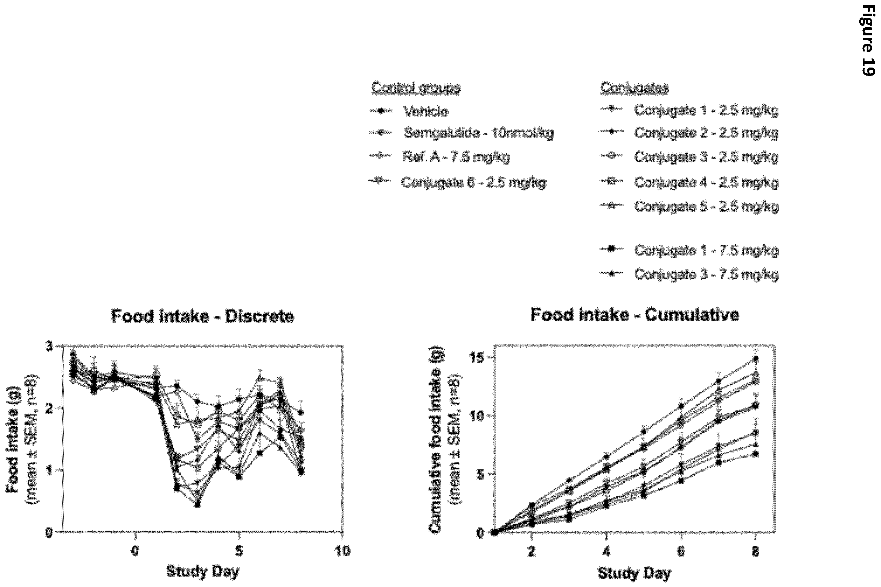

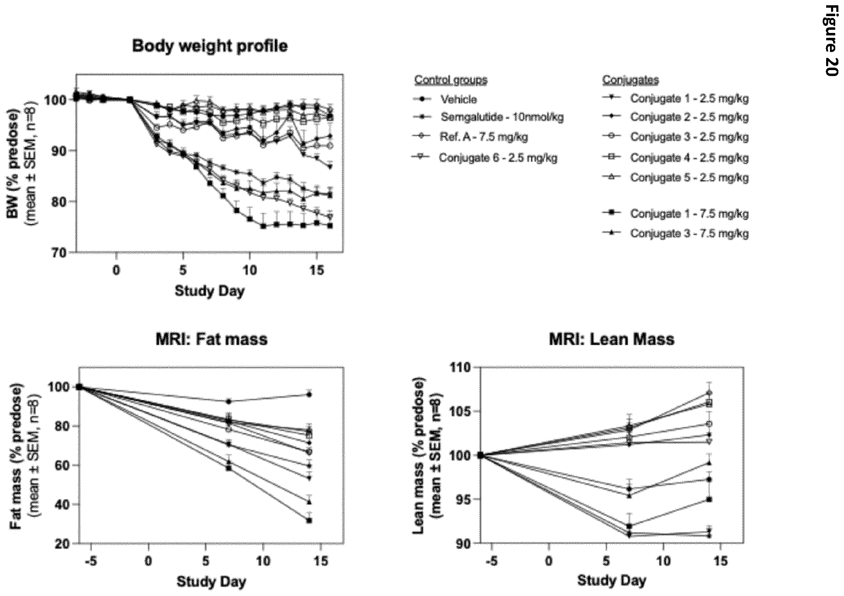

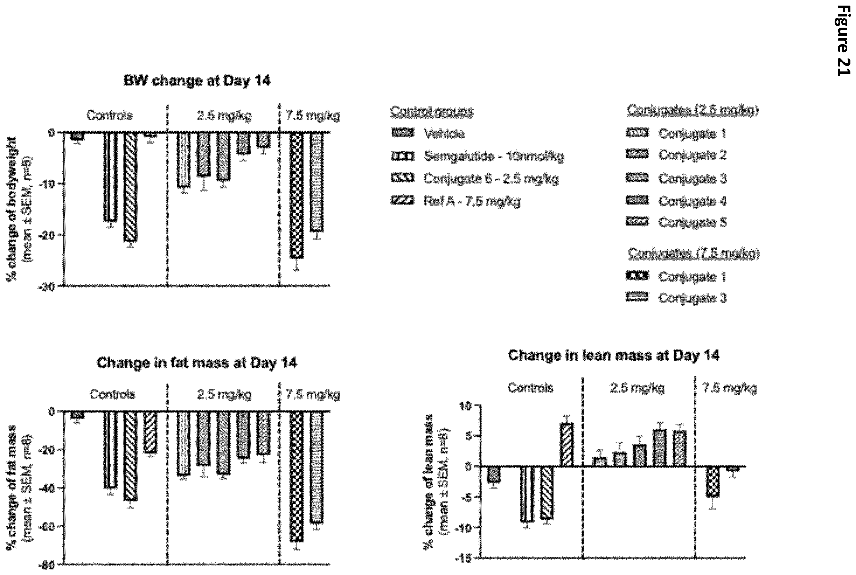

This in vivo study demonstrates the different impact of the tested conjugates on bodyweight lowering and body composition. Strong fat and lean mass reduction was observed for the semaglutide control group and the non-binding IgG conjugate group (Conjugate 6). A neutral bodyweight effect, characterized by a moderate fat mass loss (compared to e.g. semaglutide) which is compensated by a lean mass increase, was observed for the unconjugated ActR2A/B-binding antibody. A similar effect on fat mass loss was observed for Conjugates 1-5 dosed at 2.5mg/kg, while a substantially higher fat mass loss is observed for the 7.5mg/kg groups of Conjugates 1 & 3. Conjugate 3 at 7.5 mg/kg demonstrates the most positive effect on body composition with 60% fat mass loss and a neutral effect on lean (muscle) mass. Results are visualized in Figures 19-21.

### Example 5.2: Second in vivo study

C57BL/6JRj male mice obtained from Janvier Labs were subjected to a 60% high fat diet (HFD) from the age of 5 weeks onwards for a period of 21 weeks before study enrolment. Animals were randomized before dosing into treatment groups based on bodyweight and body composition measurements. Conjugates (or their respective vehicle, 0.9% NaCl) were administered weekly (10 & 25mg/kg) via intraperitoneal (IP) injection for 2 weeks.

Treatment groups:
Conjugate 3 dosed at 10mg/kg
Conjugate 3 dosed at 25mg/kg
Conjugate 17 dosed at 10mg/kg
Conjugate 17 dosed at 25mg/kg

Control groups:
Vehicle (0.9% NaCl)
Reference antibody (unconjugated) ActR2A/B binding mAb dosed at 25 mg/kg
Conjugate 14 (antibody specific for hen egg lysozyme conjugated to peptide 1) dosed at 10mg/kg
Conjugate 14 (antibody specific for hen egg lysozyme conjugated to peptide 1) dosed at 25mg/kg

HFD was continued during treatment. The bodyweight of the animals was recorded daily. Food intake was measured daily during the first treatment week and biweekly thereafter. The body composition (fat and lean mass) was analysed weekly by magnetic resonance imaging (MRI).

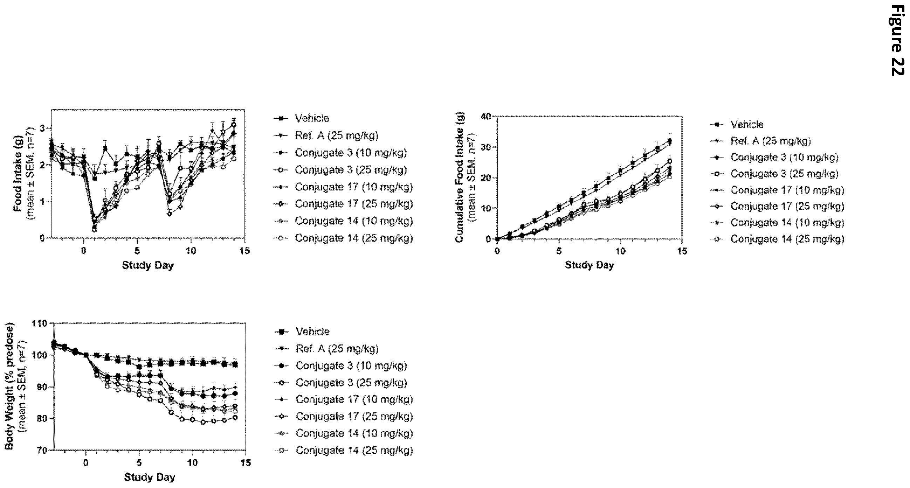

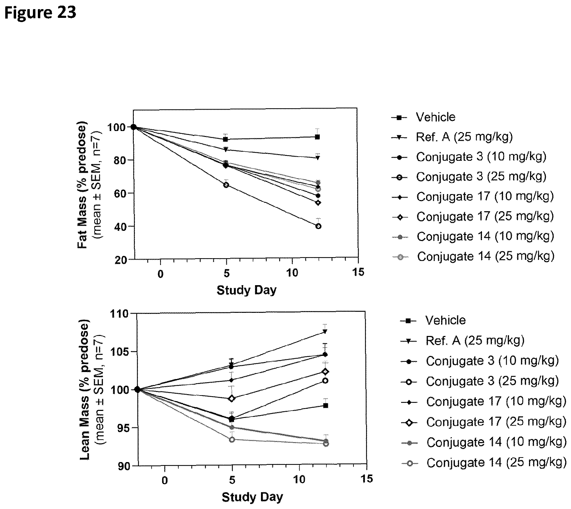

The therapeutic activity of the conjugates of the present disclosure could be confirmed in vivo. Of importance, the conjugates of the present disclosure led to pronounced decrease of fat body mass without loss of lean (muscle) mass, whereas the loss of body weight of semaglutide and non-binding IgG conjugates (Conjugate 6 & 14) is attributable to the loss of fat mass and a substantial amount of lean (muscle) body mass. Upon treatment with the ActR2A/B binding IgG conjugates lean body mass can be fully preserved or even increased. Results are visualized in Figures 22 and 23.

## Claims

1. An immunoconjugate comprising an ActRll receptor antibody and an agonistic GLP-1 peptide,
wherein said ActRll receptor antibody comprises a variable heavy chain comprising the CDR amino acid sequences of SEQ ID NO: 20 (CDRH1), SEQ ID NO: 30 (CDRH2), and SEQ ID NO: 22 (CDRH3) and a variable light chain comprising the CDR amino acid sequences of SEQ ID NO: 31 (CDRL1), SEQ ID NO: 32 (CDRL2), and SEQ ID NO: 33 (CDRL3), and
wherein said agonistic GLP-1 peptide comprises the amino acid sequence of SEQ ID No. 79.

2. The immunoconjugate according to claim 1, wherein said agonistic GLP-1 peptide is comprised in a compound comprising the amino acid sequence of SEQ ID No. 64 or 69.

3. The immunoconjugate according to claim 1 or 2, wherein said ActRll receptor antibody comprises a variable heavy chain comprising the amino acid sequences of SEQ ID NO: 28 and a variable light chain comprising the amino acid sequences of SEQ ID NO: 29.

4. The immunoconjugate according to any one of claims 1-3, wherein said ActRll receptor antibody specifically binds to a polypeptide of SEQ ID No. 58 and a polypeptide of SEQ ID No. 59.

5. The immunoconjugate according to any one of the preceding claims, wherein said agonistic GLP-1 peptide or said compounds is conjugated to said ActRll receptor antibody via an engineered cysteine in the Fc region of said ActRll receptor antibody.

6. The immunoconjugate according to claim 5, wherein said engineered cysteine is at position 272 of the constant region of the heavy chain (EU numbering).

7. The immunoconjugate according to any one of the preceding claims, wherein said ActRll receptor antibody comprises the mutations L234A, L235A and P329A in the Fc region (EU numbering).

8. The immunoconjugate according to any one of the preceding claims, wherein said ActRll receptor antibody comprises a heavy chain comprising the amino acid sequences of SEQ ID NO: 26 and a variable light chain comprising the amino acid sequences of SEQ ID NO: 27.

9. A pharmaceutical composition comprising an immune conjugate according to any one of the preceding claims.

10. An immunoconjugate according to any one of claims 1-8 or a pharmaceutical composition according to claim 9 for use in medicine.

11. An immunoconjugate or a pharmaceutical composition for use according to claim 10, wherein said use in medicine is the use in the treatment of a metabolic disorder.

12. An immunoconjugate or a pharmaceutical composition for use according to claim 11, wherein said metabolic disorder is selected from the group consisting of: obesity, diabetes, metabolic syndrome, anti-psychotic drug-associated obesity, glucocorticoid-induced obesity, hypothalamic obesity associated with craniopharyngioma, Prader-Willi syndrome, and a monogenetic disorder associated with obesity.

13. An immunoconjugate or a pharmaceutical composition for use according to claim 12, wherein said treatment is useful for an obesity related co-morbidity, wherein the condition is selected from the group of: glucose intolerance, prediabetes, insulin resistance, high triglycerides, overweight associated physical impairment, osteoporosis, renal disease, obstructive sleep apnea, sexual hormones impairment, endocrine reproductive disorders, osteoarthritis, gastrointestinal cancers, dyslipidemia, hypertension, heart failure, coronary heart disease, stroke, and/or gallstones.
